# EUROPEAN PATENT APPLICATION

(11) **EP 1 346 999 A2**
(43) Date of publication of application: **24.09.2003**
(21) Application number: 03012177.6
(22) Date of filing: 15.07.1998
(51) Int. Cl.: C07K 14/47, A61K 38/00, C12N 15/12

(54) **Human secreted protein**

(30) Priority: 16.07.1997 US 52661 P; 16.07.1997 US 52872 P; 16.07.1997 US 52871 P; 16.07.1997 US 52874; 16.07.1997 US 52873 P; 16.07.1997 US 52870 P; 16.07.1997 US 52875 P; 22.07.1997 US 53440 P; 22.07.1997 US 53441 P; 22.07.1997 US 53442 P; 18.08.1997 US 55726 P; 18.08.1997 US 55724 P; 18.08.1997 US 56359 P; 18.08.1997 US 55725 P; 18.08.1997 US 55985 P; 18.08.1997 US 55952 P; 18.08.1997 US 55946 P; 18.08.1997 US 55683 P; 18.08.1997 US 55989 P; 18.08.1997 US 56361 P
(62) Divisional of application: 98934551.7
(71) Applicant: Human Genome Sciences, Rockville, Maryland 20850 (US)
(72) Inventor: Ruben, Steven M., Olney, MD 20832 (US); Rosen, Craig A., Laytonsville, MD 20882 (US); Young, Paul E., Gaithersburg, MD 20878 (US); Greene, John M., Gaithersburg, MD 20878 (US); Ni, Jian, Germantown, MD 20874 (US); Feng, Ping, Gaithersburg, MD 20874 (US); Florence, Kimberly A., Rockville, MD 20851 (US); Hu, Jing-Shan, Sunnyvale, CA 94086 (US); Ferrie, Ann M., Germantown, MD 20874 (US); Yu, Guo-Liang, San Mateo, CA 94403 (US); Duan, Roxanne, Bethesda, MD 20817 (US); Fouad, Janet, Westerly, RI 02891 (US)
(74) Representative: VOSSIUS & PARTNER

(57) **Abstract**

The present invention relates to one human secreted protein and isolated nucleic acids encoding said protein. Also provided are vectors, host cells, antibodies, and recombinant methods for producing said human secreted protein. The invention further relates to diagnostic and therapeutic methods useful for diagnosing and treating disorders related to this human secreted protein.

<110> Human Genome Sciences, Inc.

<120> 64 Human Secreted Proteins

<130> PZ011PCT

<140> Unassigned
<141> 1998-07-15

<150> 60/052,870
<151> 1997-07-16

<150> 60/052,871
<151> 1997-07-16

<150> 60/052,872
<151> 1997-07-16

<150> 60/052,661
<151> 1997-07-16

<150> 60/052,874
<151> 1997-07-16

<150> 60/052,873
<151> 1997-07-16

<150> 60/052,875
<151> 1997-07-16

<150> 60/053,440
<151> 1997-07-22

<150> 60/053,441
<151> 1997-07-22

<150> 60/053,442
<151> 1997-07-22

<150> 60/055,952
<151> 1997-08-18

<150> 60/055,725
<151> 1997-08-18

<150> 60/056,359
<151> 1997-08-18

<150> 60/055,985
<151> 1997-08-18

<150> 60/055,724
<151> 1997-08-18

<150> 60/055,726
<151> 1997-08-18

<150> 60/056,361
<151> 1997-08-18

<150> 60/055,989
<151> 1997-08-18

<150> 60/055,946
<151> 1997-08-18

<150> 60/055,683
<151> 1997-08-18

<160> 178

<170> PatentIn Ver. 2.0

<210> 1
<211> 733
<212> DNA
<213> Homo sapiens

<400> 1
gggatccgga gcccaaatct tctgacaaaa ctcacacatg cccaccgtgc ccagcacctg 60
aattcgaggg tgcaccgtca gtcttcctct tccccccaaa acccaaggac accctcatga 120
tctcccggac tcctgaggtc acatgcgtgg tggtggacgt aagccacgaa gaccctgagg 180
tcaagttcaa ctggtacgtg gacggcgtgg aggtgcataa tgccaagaca aagccgcggg 240
aggagcagta caacagcacg taccgtgtgg tcagcgtcct caccgtcctg caccaggact 300
ggctgaatgg caaggagtac aagtgcaagg tctccaacaa agccctccca acccccatcg 360
agaaaaccat ctccaaagcc aaagggcagc cccgagaacc acaggtgtac accctgcccc 420
catcccggga tgagctgacc aagaaccagg tcagcctgac ctgcctggtc aaaggcttct 480
atccaagcga catcgccgtg gagtgggaga gcaatgggca gccggagaac aactacaaga 540
ccacgcctcc cgtgctggac tccgacggct ccttcttcct ctacagcaag ctcaccgtgg 600
acaagagcag gtggcagcag gggaacgtct tctcatgctc cgtgatgcat gaggctctgc 660
acaaccacta cacgcagaag agcctctccc tgtctccggg taaatgagtg cgacggccgc 720
gactctagag gat 733

<210> 2
<211> 5
<212> PRT
<213> Homo sapiens

<220>
<221> Site
<222> (3)
<223> Xaa equals any one of the twenty naturally ocurring L-amino acids

<400> 2
Trp Ser Xaa Trp Ser
1               5

<210> 3
<211> 86
<212> DNA

<213> Homo sapiens

<400> 3
gcgcctcgag atttccccga aatctagatt tccccgaaat gatttccccg aaatgatttc 60
cccgaaatat ctgccatctc aattag 86

<210> 4
<211> 27
<212> DNA
<213> Homo sapiens

<400> 4
gcggcaagct ttttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120
gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg 32

<210> 7
<211> 31
<212> DNA
<213> Homo sapiens

<400> 7
gcgaagcttc gcgactcccc ggatccgcct c 31

<210> 8
<211> 12
<212> DNA
<213> Homo sapiens

<400> 8
ggggactttc cc 12

<210> 9
<211> 73
<212> DNA
<213> Homo sapiens

<400> 9
gcggcctcga ggggactttc ccggggactt tccggggact ttccgggact ttccatcctg 60
ccatctcaat tag 73

<210> 10
<211> 256
<212> DNA
<213> Homo sapiens

<400> 10
ctcgagggga ctttcccggg gactttccgg ggactttccg ggactttcca tctgccatct 60
caattagtca gcaaccatag tcccgcccct aactccgccc atcccgcccc taactccgcc 120
cagttccgcc cattctccgc cccatggctg actaattttt tttatttatg cagaggccga 180
ggccgcctcg gcctctgagc tattccagaa gtagtgagga ggcttttttg gaggcctagg 240
cttttgcaaa aagctt 256

<210> 11
<211> 558
<212> DNA
<213> Homo sapiens

<220>
<221> SITE
<222> (546)
<223> n equals a,t,g, or c

<400> 11
gaattcggca cgagctgggc tgcagttggc gattccgcgc ggtgaaagca gccagtgccc 60
agggtctttt cctgagtgca cctgggcctg ccgcccggcg atgccatggg gtcgtgcgct 120
gcttttctac ttgccgcgct ctcactgctc ggtgtactgg gagggtaccc tgggaggcgt 180
gcctttattc ttccgaaccg ccgctcactg agacagtggc tagaagtgtc tcttggacct 240
gtgagttagc cttaacctgt tatgccccca gagccctcag tggagcgccc gtactttgcc 300
ggcatgacgt ttgatttccc ggtgataatc cgacgagttt gacagattga ggtagtgagc 360
aaagttgccc gtcagttggt ggccacttga cttcgtgcgg accctggcct tgctcttgga 420
agagatagtg ttcttagggc tggtttcact gtctcttaag actgaarggt ggarctggga 480
tatagatgtg ttgtttcttt tcaaatcaaa cctgcttarg tcgtcactcg aaggggggcc 540
cggtanccaa ttcgccct 558

<210> 12
<211> 715
<212> DNA
<213> Homo sapiens

<400> 12
cggatttcga gtgcttttct ccttacctcc accctccccc atgttttaat gcagccctcc 60
aaaaatattt actgagtgtg gactctagac cagggcctgt gctaggatac aaagatgaat 120
gaggcaccac ccttatcttc gagtagtata tgttttattt tattttattt ttttcccctg 180
ctgcctccct tgagtagtac atgttttagt aaggggaaca gacactaaag agtcctggta 240
atgatgagca aagtactgca tgagtaagta tctggggggc aagtgtcccc actaggactc 300
ctgtcagatc tggaaaaggc ctgaggaatc tgatacatga cttaatgcag cgtatacttg 360
cagcctggaa aactaagtaa tgacaaaata gacattcttg tcagtgtgag ccattctctg 420
agtccmaggg gagtacataa ttcaaaccag aattggtcat tttggagttt gcactcttag 480
cagtatacag tggagtgaaa tttaagaatc aatttaattt cttttcagtt tttatgtaca 540
taaaacctgc ttactacaag agacccagtt tattattttg tgttggttaa cattcataag 600
tatatttcat cataataagg ctccgtgaaa ttagtcattt tatcatttgc caataaagac 660
atatatctga aaataaatgt tcctgaacct gaaaaaaaaa aaaaaaaaaa ctcga 715

<210> 13
<211> 838
<212> DNA
<213> Homo sapiens

<400> 13
gaattcggca cgagccaaaa caaaagaaac ctttggaggc atgtgtcaga acagagaaag 60
tgtcctggtt ttgcttatag aatcaaatat gttctcattc tacctactgt tttcattcta 120
catagtgttt tccttcttta tagttttacg tcctcttcct aggaatgagt ctattaagaa 180
aataggtgtt atcttttagc tttggcattt gactttcagg ataatagagc tatctgctac 240
tgacagaaaa gctttgacaa gtgtttaata ctctgggatt accttcatct tacttttgca 300
atcattatgt gaacattgtc ttccgtccac atctayaggc tagtawgtaa caccgttgac 360
taaatccaaa ctttaggcta gggaaaaagg gtatactttc tgggtttcgg ttgtagatta 420
tgtttagatc taaycaaaac aggacagtgg tccaaacaga aaattgctat tttctgtatc 480
ttgtaaatct aggatttgag tttttaagat gaatttatgg ttccctttct gatatcattt 540
ctcatctgca gctcctaatg cctggtacct tgggtatgga gtgaggagag acaatggaca 600
gttttatata agaaatggaa gtaatgatac tatctttcct ggaatatttg caggccccag 660
aggagatgat gagcaaggac tgttggcctg tattacacac aacagggttg tagttactat 720
cccagcaagg aaagggtgta tctttcttct ttcatgcaaa ttatctatga tgacctaaca 780
gtttgattat agtgagtgga ctaaccacaa caataaaaaa aaaaaaaaaa aactcgaa 838

<210> 14
<211> 513
<212> DNA
<213> Homo sapiens

<400> 14
ctgcaggaat tcggcacgag ggaacaactc catgtttttg taaaggccta gagaacatat 60
atccagtgcc tttccttttt gcctttgtat tcatcatttt ggcaaattac tggaagatga 120
cggttctggc caaaaggctg gttttgtttt tgggtcacat tttcttgctt ctctgcgtta 180
gaatcttgga ttagatgatg gacatggtga agatctcagc aacctcattc actagaagat 240
catgtggatt ggaatcatac aatggggaac aaatggaaaa gagtactttt gaaatagtgc 300
tggagaccac tgtgaccaca gaatgtcaag acacgtgctg ccattactgt tactatttgg 360
aaaatacatt cttgtaaatg caaccttagg gggtttgagg gggaagtctg ttgggaaatg 420
aattgcaaga aaaatattac accctgaaaa aaaaaaaaaa aaaaaaaaaa aaaaaaaaaa 480
aaaaaaaaaa aaaaaaaaaa aaaaaaaact cga 513

<210> 15
<211> 712
<212> DNA
<213> Homo sapiens

<220>
<221> SITE
<222> (565)
<223> n equals a,t,g, or c

<400> 15
gaattcggca cgagctggac aggaccggag aggaccccgc gtaaccgcgg aacagacact 60
cccggcagcg gccgccgccg cggcactgct acgggacgag ccggagcgct tggccatggc 120
ggcccgatcc gcactggcgc tgctgctgct gctgccagtc ctgctcctgc cggtgcagag 180
ycgctcagag cccgagacca ccgcgcccac ccctacccca atcccgggtg gcaactcgtc 240
aktgagcagg cccctgccca gcatcgagct ccacgcctgc ggcccatacc ccaaaccagg 300
cctgctcatc ctgctggccc cgctggccct gtggcccatt ctcctgtagg gacgcccagc 360
cagccacctc taagtcgccg ctgggactgg cctgccccat tgagcaacag agacgcttga 420
cagccgcccg cctccattcc ttgacttcac ccagaaatgg gtccagaaaa ctgaatccca 480

ccagcactgg tttggagcaa ccggacaccg aggtttcacc tccagggrtt ccatggaaga 540
gcctcaatgg agatgccaca tcctnactga gttaaagatg ggctgaggaa cttgggtacc 600
cacaagtytg ccttgggrat caaaagaaaa tatttacctt tagtttggtt cattaaatgc 660
atgaagtcaa aatatgaaaa aaaaaaaaaa aaaaaaaaaa aaaaaaactc ga 712

<210> 16
<211> 652
<212> DNA
<213> Homo sapiens

<400> 16
gaattcggca cgagcaacag tggggcactc tgctcccagg caggtcccac tgggctgagc 60
cgcacagcct ggctttgggc ttccctgact gcaccaccca catcasctgc ctctagccct 120
taamatacaa aacttccccc agtcactggc cgccaggctg agttggggga tgtgttacat 180
ccctgggtcc actggggggc agtgttggcc atggtgttgg tgctggctct gccgagaggc 240
gttggagtgg ctgtgtgggg cggtgagcgc cggcccagcc tgatggaacc cactgtacca 300
ggcccaggcc tcagcctctg agaaggactt ccctgtgtca ctcactcata catgtcctca 360
ggacgtgaag acatttcagc agaccaaagt ttccttcgaa tttccttcga atcgtccaga 420
tacttggaga catctcctcc tcacctgtgg ggtgctgggg cagtcctagg cgtgggggca 480
gatgggtgga cagctgctgc tgccctgctg ggggtgggca gcccttggag cacacagtgg 540
tgaagacatt cctgaatatg tctcaggctg tagaaatctt attttgtgga aagattttag 600
agaatcatca aaataaactt ttaccaaata aaaaaaaaaa aaaaaaactc ga 652

<210> 17
<211> 742
<212> DNA
<213> Homo sapiens

<400> 17
ggtcgaccca cgcgtctgat atgatctcct tatccttctc cctttgattg atcttttct 60
ttgagctgat ttgagctttc ttcttttctc tgtagttggc ggaatcagct cagttacatt 120
ttttactaag ttacccacat tctgacactc cttgacagtk ttaagatctt cttctaacac 180
acttgaatag aatggatact ggaatctatt ttgacagctg ttgaaaatct attctgttgt 240
tacaggaggt taaggaggtt atttgtaaca ctgggattat ttaatgaacc ttttgaaaag 300
gtgtgcagac tgttcaggca aatagtattt tttagaatta aatgattttg gttttcacag 360
ttaaattatc aaatgtaatg cttttaagaa ttatacacct agtaatattt ttcattaatt 420
tctccaccag tgtagtaata gtacattaca atgttctcaa ttaccggtgc cttctaaaat 480
gcaggtgtag agtcyttaaa tacagctagt ctatkgccag ctgtcccata gataaccttc 540
tcyttaaaar tgaccttkgr gcaattycat aaagaataaa tatttctagt tttttgttgc 600
tgaactgcta aaagatggtt ctatacatgt aacaggtggc tttagttggg ttgctttcac 660
tgaaatttga ttcaaataaa gcattgcatt attttacctt tggaattata aaaaaaaaaa 720
aaaaaaaaaa aaaagggcgg cc 742

<210> 18
<211> 1219
<212> DNA
<213> Homo sapiens

<400> 18
aacgcactca atattcagaa gtttgaattc taccactctc aaacacagtt caaaagatag 60
ctgtttgaga atgcttctta actaatacta gtacaatatc ttcaataatg tatgtacctt 120
atagaaaatc ttgaacagta caagattttc ataattaagg catgcaaaac tgcttgggct 180
ctttgattcc aggtgtcctc ttctcccttc tgcttttgcc atctatgttc aatataattc 240
taacccagtc taagtatgga gaaaattcct accctgcctg cttttatagc tcatcaaatt 300
tccctgtatc agctatcact tttctggtag gtgtagtctg atttctgtct gtcatgcctt 360
tgccacaatc ctttctttga agagtaggta aaagatctat taaagtgtta atcacattgc 420
tctaatatat aaagcctcca gtggtttccc atatcactct gtaaaatgcc ccttgccagc 480

ctctcccatc aacctcgctt tttctgttct tgtatatgca catctcttcc tgagccttta 540
ttgccatcct catgtgggga tgtttctgtc tcagagatag tctttattca ggtcccactc 600
tgcagtcctc tccagagggg ctgctttcac caccccttct aagtaagcct ctctaaacac 660
ctctatcata ttctatccct tagccagcac taattttttc ataatgctta ccactaactg 720
aaatttactt tatcatttaa tctcttcctc attagaatgt aagctcgtaa gggaggggca 780
gctctgtagt ttattcatta ttgtatgtcc ctcacctaat cctatgagtg tctggcccat 840
attagggtat gtaataaata ttacttgagg aatgaatgaa tttaacatac taccaattct 900
ctgagtgact ctttttaaag ccttcatcat cattcacact ttcttgtctt tcatatgggc 960
atgtccaatc acccttccat gaatatctgt acctgttaca aagagaggac taggttcctg 1020
gagttcatag atgtaaacaa catcctaggg ktagcaaact ggtgggccct gagccaaatc 1080
ctggcctgca catgtatttt gtttgakttg tacaatgttt gttataaatg aactggctga 1140
taatattttt taattggaaa tgtttacatt aaaaacctar acttctagct gctcttaaaa 1200
aataaaaata cggctgggc 1219

<210> 19
<211> 874
<212> DNA
<213> Homo sapiens

<220>
<221> SITE
<222> (461)
<223> n equals a,t,g, or c

<400> 19
ggtcgaccca cgcgtccgag caattgaatc atctgcccaa ggataagctg ctggtgagag 60
cagagttggg atttgaagtc gagttagacc ccagtgatca cagtcttgac gattaaattc 120
ttccagcttt catttttcac tgagataatg gtagtgatag tactgacctc taatgtgtgc 180
atttgtgggt atgtggtcca ttcagcttta atccccagaa gacaaggctt attccttttc 240
ttatttttgg tcatgtttta tttttccatt gcttttaaca ggattaccaa aggcacactc 300
agtagtcagt aaacacattt ctaggaaagg tgttgtgtca tcatgccaca tattcatact 360
ttcctgggtt ggaaaataga tcatcagtaa aaacatacag gaaaaatgaa tcttgccaat 420
gcaattgtta acctacaacc ataatatacc ttaagtatat ntttgcacat aagtataaca 480
tgcgatttaa aacaataaac cagattgaga tctaaggagc attttgtaag taattactaa 540
tgtttatttt agagagatca cacaacttca aataaaaact gacatagatt gaacaccttg 600
agaataaact ttagtgccaa atggaaaata attttttaca agtaaatttg aagaacaatg 660
tgaactttct ataattatat acagraaata tactgatttg ccaaaatgag taattttgat 720
atattaatat ttcacttata agaatgcata ccacctgatc caggatggga tccaggaaca 780
gaaaaagaac attagktaaa aatgacagaa atctgaatat agtatagagt agctaaaaac 840
aaaccaaaaa aaaaaaaaaa aaaaaagggc ggcc 874

<210> 20
<211> 464
<212> DNA
<213> Homo sapiens

<220>
<221> SITE
<222> (21)
<223> n equals a,t,g, or c

<400> 20
caaacccttc agtggatgag nccaagtcgc agaaagcatt ctgttgacag atgaacagcc 60
gaaagctggc cagaccctcc tgkatgcact cccwgcccck tktatcagaa acacaggcaa 120
ggaaattgga actgccaccc agcccagcat ggtggctcaa ttggttggtt gcgttgtcag 180
ttgtctcttc gttttgttaa ggtttttaat aagtacgttt ggcataatgt cttttaatgg 240
gtttgtaata tttgtaacgg ttttagcagc ctataacttt tcagctggtg cttttactta 300
gggaaaaaaa caatttgtaa atacagaaca ttgtttaaaa gacataacca tagaacatag 360

cttcctgttt gtggattttg tttcctatat attcaaagta aaatgactta caggaaaaaa 420
ataaaaaaaa aaaaaaaaaa aaaaaaatcg gggggggggc ccgg 464

<210> 21
<211> 637
<212> DNA
<213> Homo sapiens

<400> 21
gattttcctg cttgcatcat ttctagcaca gagctggagg aaatggcgag gtgcaggtgg 60
ccgctggccm tgctgttcta catgggagca agacagctgc taggtgaagg ggaatgacca 120
ggcagccaca gggaggacat gtggcctcag gaagcctggg tgtgtatcct ggttctgcta 180
ggaacacgtg tggggctttg tgtgggtgac tctctggctc cccaagcctc cctttcctac 240
tgttatatcc ttaaagtgcc tctgaggcca aagcctttgt ggcaattgtc aaatgagtcc 300
atatgcagtg agtaccgtgt tgagggagga caaggtcacc aagagctgag aatgtttctc 360
cgactgatga gacctagata ttgggtacat ggaggtcccc ggtccctttg tgattcctgc 420
agcctgttgc ctccttgcct ggaccccgcc tcagctcaga aagccaattc cctagattcc 480
aaaggccttc ccagaccaat tagcatgtcc tgcagctgtc agctccctgt gcctagcctg 540
gacctcagct catgtctagc acccagtctc ccaaccccac acatattcac aaataaaaga 600
aaataacaaa tgaaaaaaaa aaaaaaaaaa aaaaaat 637

<210> 22
<211> 752
<212> DNA
<213> Homo sapiens

<400> 22
gaattcggca cgaggggatt acaggcatga gccaccatgc ccggccatat aaagcattta 60
ggatagttag ttgctatttt tatttattta ttattgttgt tgttattata ttactacttt 120
atcccatttc acaaggatgg catgttgcca acattgtctt tctaaagaat atctctgatc 180
acatccttgt tctattaaaa accttttgaa agctccctct tacctttaga agaaattgga 240
acttcatgat tcctcatggt ctggctccag cactgagtct ggaatgctag tgtgagatga 300
ggccttagaa gtcatccagc tgaactcctg gaatttttat agatgaataa atgtagcatc 360
cagacatttt tcytgttgca cccctgtamg ccatgtcctc ttccagactc ctggataaga 420
ctgrcagaca tcaccattct cttaaaccag aactacactt gccttcatcc atttgatcac 480
ctggttccag gtaactcatg agctttgtag cttcccttct ctcagacctt ccaaggaaga 540
caatggcata attttcccca tatgctctaa ttagcaacct ttccctgccc ttctgtgggt 600
gggcagggcc ggacacagtg ggtcacacct gcaacctgta atcccagcac tttgggaggc 660
tgaggtgggc agattgcctg agctcaggag ttcaagacag tctgggtaac atggcaaaat 720
cctgtctcaa aaaaaaaaaa aaaaaaactc ga 752

<210> 23
<211> 492
<212> DNA
<213> Homo sapiens

<220>
<221> SITE
<222> (486)
<223> n equals a,t,g, or c

<400> 23
aagctggact cgcgcgcttg caggtcgaca ctagtggatc cmaaagaatt cggcacgagc 60
aaggacccag aagtagggtt ttggcctagg taacggggca gagatgtggt tcgagattct 120
ccccggactc tccgtcatgg gcgtgtgctt gttgattcca ggactggcta ctgcgtacat 180
ccacaggttc actaacgggg gcaaggaaaa aagggttgct cattttgggt atcactggag 240
tctgatggaa agagataggc gcatctctgg agttgatcgt tactatgtgt caaagggttt 300

ggagaacatt gattaaggaa gcattttcct gattgatgaa aaaaataact cagttatggc 360
catctacccc tgctagaagg ttacagtgta ttatgtagca tgcaatgtgt tatgtagtgc 420
ttaataaaaa taaaatgaaa aaaawrmaaa aaaaaaaaaa aaaaaaaaaa aaaaaaaaaa 480
aaaaanaaaa aa 492

<210> 24
<211> 532
<212> DNA
<213> Homo sapiens

<400> 24
actcatataa gaaagcagta cgccgcagta ccggtccgaa ttccgggtcg acccacgcgt 60
ccgcccacgc gtccgcacct cccttggctg tggggagggg cttccatgcc ctgtgtggct 120
ctcgggtggg ctgtcgcacc acactgctct tcctttctct tcacgaatca cgcaagcctc 180
ctagtcagtt ctgatgagat aacctggata tcttggttgc cggtgaagga tttacatgct 240
tattatggtt tttttgttgt tgttgttgtt tggttttttt tttgatggga gcctcagatc 300
gccgctgttg ctaatcatcc atcttggccc tgcccccaca tttctgcaaa tttaaatatg 360
agatttgtcc ccttaggtgc acagtccaga ccccatccag tccagctcct tttaaagcca 420
catggaaagt cagctgagaa tggtttggga gcccaggtgc gctgtcttcc gccctgccct 480
ctccctgaaa taaagaacag cttgacagaa aaaaaaaaaa aaaagggcgg cc 532

<210> 25
<211> 920
<212> DNA
<213> Homo sapiens

<220>
<221> SITE
<222> (907)
<223> n equals a,t,g, or c

<220>
<221> SITE
<222> (914)
<223> n equals a,t,g, or c

<220>
<221> SITE
<222> (920)
<223> n equals a,t,g, or c

<400> 25
   gtcggaattc ccgggtcgac ccacgacgtc cgcaaaatta acatcaaaaa ggtatatact 60
   ttttaaaaaa aatttacttt tattgatgtg tactcttcct attgatgagt taattccata 120
   aatctctact tagtttaact tattggatca aattatcttc agcatgtata tctggggaaa 180
   aaaggtccga attttcacat ttatatttaa acttcaattt tttatattta aacttcaatt 240
   ttttagcaac agctgaatag ctttgcggag gagtttaata gttacacatt catgctaata 300
   tacatttcct ttaaacatcc acaaattctt aaaaagrttg aatcagtaaa tttcatttca 360
   gctaaaaatg gagtctaata tattgtttca aaagatacat ttttacccac cataaatgtt 420
   acaatatctg aatatgcttt gtcaaactat ccctttatgc aatcgtcttc atattgtttt 480
   tatgattcta atcaagctgt atgtagagac tgaatgtgaa gtcaagtctg agcacaaaaa 540
   gataatgcac gatgagattg cctaccattt tataggatat ttactatgta tttatacgtt 600
   aagacctcta tgaatgaatg tatcagagaa tgtctttgta actaactgtt taattcaatc 660
   tgtaataaaa atctaactaa ctaactcatt tatttctatt aaaaaggtat tgtcctttag 720
   gcggggaatg ggaatccttg ctgcactgtt gcagtcattc tgaaaggacc tttccctgta 780
   cttacctttc aacatgcttc aatcttatca acgctacatt ttgtattttt caaacaagta 840
   taaattctgc aataaagaga tgtagttttt ttttaaacaa aaaaaaaaaa aaaaaaaaaa 900
   aaaaaanggg gggncccccn 920

<210> 26
<211> 917
<212> DNA
<213> Homo sapiens

<220>
<221> SITE
<222> (434)
<223> n equals a,t,g, or c

<400> 26
gaattcggca cgaggtttca ttgccctcaa cattctctgt tcttcaccga atcatgtctg 60
tttcctccaa cctctggcaa acactgatct tgttactgtc tttgtggttt tgcctttttc 120
cagaatgtca tatagttgga atcatacaat tgtgcagact ttttagattg ccttctttca 180
cttagtaaca tttaagtttc ctccacccct tttcatggct tgatagttca tttcttttaa 240
ttgctcaata ataaatattc cattatctag atagaacggt ttatctacct agtgaaggac 300
atctcaattg cctccaagtt taggcaaata taaacaaagc tgctatcagg atttttcaca 360
gaggaaaaga cagtgggatc caaaactgaa tggtctatca ataaatgacg catggtacat 420
ctacacccat granccattg tgcatccatg agaaaaatcc agatgtagga aggtatgtat 480
aattttgcag aaaagagtat gtaactggaa acaccaarga aaaaaggaaa tggatctata 540
tatttaggtg gagatattta tgtggctgca gaagaaatat attattattc atactagata 600
gttaatgttt gcctttggtg ggcaagaaag gtaaaaaggg agaagggagc ccaaccaaaa 660
gaggaagagg aagaaaaaaa aactgcacta agaaaaatct tttaaaagta tgtgatcaca 720
gccaggtgca gtggctgaca aatgtaatcc cagctacttg ggaggctgag gcaggagaat 780
cgcttgaacc caggaggctg aagttgcagt gagctgagat catgccattg cactccagcc 840
tggtgacaga gactctgttt caaaaaaaaa aaaaaagtat atgatcacat ctgtgttaac 900
ttacagacta gtctcga 917

<210> 27
<211> 662
<212> DNA
<213> Homo sapiens

<400> 27
gaattcggca cgaggttccc atggcacttt atatgtgtgc atagagagcc agggagcagt 60
ggggttcagg gtgggcccat gctatgtgct gcagagctgg tgggtcacag tctccccagg 120
tgatggtggt gttaataatc atcctaggcc cgtggggtgg ggtgaggatt gatgcatgag 180
aaagttgagg cgggggccct ggcatggagc agggctcagg ccgcttgtca cccaggctca 240
tgtcagccct ccggagcctg tgggtgtata ggggaagcgc aggggttctt cagccagagg 300
gacaggttca rggcctgctg atgccccttg ctggttttgg gaccttgagc aagtcccctt 360
gccttttggt gctgtgcctc ggtttcttct tctataagaa ggaggtgatg atgtaaccca 420
cccacccagc ccctctaccc cgcgcatcag ggtagcaggc gagctagcac tgtggcacca 480
ggagtggagc tggcccctgg cgggcccacg ctggagaggc atcgccatct ctgctgcccc 540
cctgtggcgt catcatatca acctgccagt ccccctcacc tggtgttaat ctcccagagg 600
atggggactg rttctgcata ttctttgcta aacaaagacg ctagtttggc tgtggctctc 660
ga 662

<210> 28
<211> 699
<212> DNA
<213> Homo sapiens

<400> 28
gattcggcac gagaaacttt taaatcttta gttatttctt aatacttaga acacttaaac 60
aaaactttac aaaacaaaag agcagaataa ttagatcctt tcaggagaat atgacttttt 120
tttcctaagc acactggacc atagaggaag accaaaggaa tgtacagttg cctgctcctt 180

cctgacttgc tgtatttgac tctgtcccca ctggtggtgg caatgctatt aaccccacac 240
tttaacgtgg caaatcccca gaatctgttg gctggtctct ggctagagaa tgagcacagt 300
ttcaccctta tggctccaga aagagcaaga acacaccact gccagccaga agagagaaaa 360
gtcttgttct gtctctttcc cattgtccca aatagccaag cacaggttca accaccccaa 420
atgccaccct tctgctgtgc agcagccaag gaaaagaccc aggaggagca gctccaagaa 480
cctctgggca gtcagtgccc agatacttgc cccaattctt tgtgtccaag ccacactcag 540
ctgacaaaag ccaacacttt gtctctcttt tttttttttt cttttttttt gagcagagtt 600
tcactcttgt cacccaggct ggagtgcaat ggcaggatct tggctcattg caacctccac 660
ctcccgggtt caagcaattc tcctgtctca gcctctcga 699

<210> 29
<211> 1637
<212> DNA
<213> Homo sapiens

<220>
<221> SITE
<222> (726)
<223> n equals a,t,g, or c

<220>
<221> SITE
<222> (727)
<223> n equals a,t,g, or c

<220>
<221> SITE
<222> (728)
<223> n equals a,t,g, or c

<220>
<221> SITE
<222> (899)
<223> n equals a,t,g, or c

<220>
<221> SITE
<222> (901)
<223> n equals a,t,g, or c

<400> 29
aaatgtgcca cgtcttctaa gaagggggag tcctgaactt gtctgaagcc cttgtccgta 60
agccttgaac tacgttctta aatctatgaa gtcgagggac ctttcgctgc ttttgtaggg 120
acttctttcc ttgcttcagc aacatgaggc ttttcttgtg gaacgcggtc ttgactctgt 180
tcgtcacttc tttgattggg gctttgatcc ctgaaccaga agtgaaaatt gaagttctcc 240
agaagccatt catctgccat cgcaagacca aaggarggga tttgatgttg gtccactatg 300
aaggctactt agaaaaggac ggctccttat ttcactccac tcacaaacat aacaatggtc 360
agcccatttg gtttaccctg ggcatcctgg aggctctcaa aggttgggac cagggcttga 420
aaggaatgtg tgtaggagag aagagaaagc tcatcattcc tcctgctctg ggctatggaa 480
aagaaggaaa aggtaaaatt cccccagaaa gtacactgat atttaatatt gatctcctgg 540
agattcgaaa tggaccaaga tcccatgaat cattccaaga aatggatctt aatgatgact 600
ggaaactctc taaagatgag gttaaagcat atttaaagaa ggagtttgaa aaacatggtg 660
cggtggtgaa tgaaagtcat catgatgctt tggtggagga tatttttgat aaagaagatg 720
aagacnnnta tgggtttata tctgccagag aatttacata taaacacgat gagttataga 780
gatacatcta cccttttaat atagcactca tctttcaaga gagggcagtc atctttaaag 840
aacattttat ttttatacaa tgttctttct tgctttgttt aattattttt atatatttnt 900
nctgactcct atttaaagaa ccccttaggt ttctaagtac ccatttcttt ctgataagtt 960
attgggaaga aaaagctaat tggtctttga atagaagact tctggacaat ttttcacttt 1020
cacagatatg aagctttgtt ttactttctc acttataaat ttaaaatgtt gcaactggga 1080

atataccacg acatgagacc aggttatagc acaaattagc accctatatt tctgcttccc 1140
tctattttct ccaagttaga ggtcaacatt tgaaaagcct tttgcaatag cccaaggctt 1200
gctattttca tgttataatg aaatagttta tgtgtaactg gctctgagtc tctgcttgag 1260
gaccagagga aaatggttgt tggacctgac ttgttaatgg ctactgcttt actaaggaga 1320
tgtgcaatgc tgaagttaga aacaaggtta atagccaggc atggtggctc atgcctgtaa 1380
tcccagcact ttgggaggct gaggcgggcg gatcacctga ggttgggagt tcgagaccag 1440
cctgaccaac acggagaaac cctatctcta ctaaaaatac aaaagtagcc gggcgtggtg 1500
atgcgtgcct gtaatcccag ctacccagga aggctgaggc ggcagaatca cttgaacccg 1560
gaggcggagg ttgcggtaag ccgagatcac ctccagcctg gacactctgt ctcgaaaaaa 1620
aaaaaaaaaa aactcga 1637

<210> 30
<211> 2142
<212> DNA
<213> Homo sapiens

<400> 30
aattcggcac agagacgcgg gtccccgggt ctgacaggag cagcctgtgg gcaccgcggc 60
ggtagttgga ggcgggagag ggtccgtagc cgcgccgccc tgccccgcca tgggcctcct 120
gtcggacccg gttcgccggc gcgcgctcgc ccgcctagtg ctgcgcctca acgcgccgtt 180
gtgcgtgctg agctacgtgg cgggcatcgc ctggttcttg gcgctggttt tcccgccgct 240
gacccagcgc acttacatgt cggagaacgc catgggctcc accatggtgg aggagcagtt 300
tgcgggcgga gaccgtgccc gggcttttgc ccgggacttc gccgcccacc gcaagaagtc 360
gggggctctg ccagtggcct ggcttgaacg gacgatgcgg tcagtagggc tggaggtcta 420
cacgcagagt ttctcccgga aactgccctt cccagatgag acccacgagc gctatatggt 480
gtcgggcacc aacgtgtacg gcatcctgcg ggccccgsgt gctgccagca ccgagtcgct 540
tgtgctcacc gtgccctgtg gctctgactc taccaacagc caggctgtgg ggctgctgct 600
ggcactggct gcccacttcc gggggcagat ttattgggcc aaagatatcg tcttcctggt 660
aacagaacat gaccttctgg gcactgaggc ttggcttgaa gcctaccacg atgtcaatgt 720
cactggcatg cagtcgtctc ccctgcaggg ccgagctggg gccattcagg cagccgtggc 780
cctggagctg agcagtgatg tggtcaccag cctcgatgtg gccgtggagg ggcttaacgg 840
gcagctgccc aaccttgacc tgctcaatct cttccagacc ttctgccaga aggggggcct 900
gttgtgcacg cttcagggca agctgcagcc cgaggactgg acatcattgg atggaccgct 960
gcagggcctg cagacactgc tgctcatggt tctgcggcag gcctccggcc gcccccacgg 1020
ctcccatggc ctcttcctgc gctaccgtgt ggaggcccta accctgcgtg gcatcaatag 1080
cttccgccag tacaagtatg acctggtggc agtgggcaag gctttggagg gcatgttccg 1140
caagctcaac cacctcctgg agcgcctgca ccagtccttc ttcctctact tgctccccgg 1200
cctctcccgc ttcgtctcca tcggcctcta catgcccgct gtcggcttct tgctcctggt 1260
ccttggtctc aaggctctgg aactgtggat gcagctgcat gaggctggaa tgggccttga 1320
ggagcccggg ggtgcccctg gccccagtgt acccctcccc ccatcacagg gtgtggggct 1380
ggcctcgctc gtggcacctc tgctgatctc acaggccatg ggactggccc tctatgtcct 1440
gccagtgctg ggccaacacg ttgccaccca gcacttccca gtggcagagg ctgaggctgt 1500
ggtgctgaca ctgctggcga tttatgcagc tggcctggcc ctgccccaca atacccaccg 1560
ggtggtaagc acacaggccc cagacagggg ctggatggca ctgaagctgg tagccctgat 1620
ctacctagca ctgcagctgg gctgcatcgc cctcaccaac ttctcactgg gcttcctgct 1680
ggccaccacc atggtgccca ctgctgcgct tgccaagcct catgggcccc ggaccctcta 1740
tgctgccctg ctggtgctga ccagcccggc agccacgctc cttggcagcc tgttcctgtg 1800
gcgggagctg caggaggcgc cactgtcact ggccgagggc tggcagctct tcctggcagc 1860
gctagcccag ggtgtgctgg agcaccacac ctacggcgcc ctgctcttcc cactgctgtc 1920
cctgggcctc tacccctgct ggctgctttt ctggaatgtg ctcttctgga agtgagatct 1980
gcctgtccgg gctgggacag agactcccca aggaccccat tctgcctcct tctggggaaa 2040
taaatgagtg tctgtttcag carmaaaaaa aaaaaaaaaa aaaaaaaaaa aatgaccctc 2100
gagggggggc ccgggtaccc aattggccct atgaagaggc ga 2142

<210> 31
<211> 1564
<212> DNA
<213> Homo sapiens

<400> 31
ggcacgagcc ttaggggaac gtggctttcc ctgcagagcc ggtgtctccg cctgcgtccc 60
tgctgcagca accggagctg gagtcggatc ccgaacgcac cctcgccatg gactcggccc 120
tcagcgatcc gcataacggc agtgccgagg caggcggccc caccaacagc actacgcggc 180
cgccttccac gcccgagggc atcgcgctgg cctacggcag cctcctgctc atggcgctgc 240
tgcccatctt cttcggcgcc ctgcgctccg tacgctgcgc ccgcggcaag aatgcttcag 300
acatgcctga aacaatcacc agccgggatg ccgcccgctt ccccatcatc gccagctgca 360
cactcttggg gctctacctc tttttcaaaa tattctccca ggagtacatc aacctcctgc 420
tgtccatgta tttcttcgtg ctgggaatcc tggccctgtc ccacaccatc agccccttca 480
tgaataagtt ttttccagcc agctttccaa atcgacagta ccagctgctc ttcacacagg 540
gttctgggga aaacaaggaa gagatcatca attatgaatt tgacaccaag gacctggtgt 600
gcctgggcct gagcagcatc gttggcgtct ggtacctgct gaggaagcac tggattgcca 660
acaacctttt tggcctggcc ttctccctta atggagtaga gctcctgcac ctcaacaatg 720
tcagcactgg ctgcatcctg ctgggcggac tcttcatcta cgatgtcttc tgggtatttg 780
gcaccaatgt gatggtgaca gtggccaagt ccttcgaggc accaataaaa ttggtgtttc 840
cccaggatct gctggagaaa ggcctcgaag caaacaactt tgccatgctg ggacttggag 900
atgtcgtcat tccagggatc ttcattgcct tgctgctgcg ctttgacatc agcttgaaga 960
agaataccca cacctacttc tacaccagct ttgcagccta catyttcggc ctggggcytt 1020
accatcttca tcatgcacat cttcaagcat gctcagttat gaggagtcaa atcctaagga 1080
tccagcggca gtgacagaat ccaaagaggg aacagaggca tcagcatcga aggggctgga 1140
gaagaaagag aaatgatgca gctggtgccc gagcctctca gggccagacc agacagatgg 1200
gggctgggcc cacacaggcg tgcaccggta gagggcacag gaggccaagg gcagctccag 1260
gacagggcag ggggcagcag gatacctcca gccaggcctc tgtggcctct gtttccttct 1320
ccctttcttg gccctcctct gctcctcccc acaccctgca ggcaaaagaa acccccagct 1380
tcccccctcc ccgggagcca ggtgggaaaa gtgggtgtga tttttagatt ttgtattgtg 1440
gactgatttt gcctcacatt aaaaactcat cccatggcma aaaaaaaaaa aaaaaaaaaa 1500
aaaaaaaaaa aaaaaaaaaa aaaacaaaaa aaaaaaaaaa aaaaaaaaaa aaaaaggggg 1560
gggg 1564

<210> 32
<211> 1631
<212> DNA
<213> Homo sapiens

<400> 32
cttaggggga gccctggtgc tacttgcttg aagttttcag tgtaagtacc ctgatgcctt 60
ttggaccttg ggatcagatc aagagttttg gagatcaggt accaaggaaa taaggacagt 120
ctagctgcct caagtgaggg gccctttgca tagctctcct tccccctcac tgaagctggg 180
tagcctattg gggttgagag ggaaaatgtg aaatctcaga atttatctcc cttagaagag 240
agccagtaac ttatgtacaa ggatgaaaga aaggtcgcag cagtagcttt ggggaaaggg 300
aggaagatat ggcacttctc caaccccgga aaacattgct tttgaaaact gctgataaaa 360
tatgagccgg ttattacttc tgtttgggag actgtgctct ctgtggtgcc tctcttggct 420
ctactccaca gataccagac ctcttctaag aggatgagca gaccagcttt gaggttgacc 480
tgtttctctt tgtctgcctt cccaaaacac cagcccccag gaagacatta agcagcctta 540
agcttaaatt cctactccct cttccaaatt tggctcactt gccttagatc caaggcaggg 600
aaaggaaaag aagggggtc tctggcttta ttactcccct aagtctttac tctgacttcc 660
ccaaacccag aaagattttc tccacagtgt tcatttgaaa gaggagtatt ttgtcccatt 720
ttccccttcc tcattatcaa acagccccag tcttccttgt ctctgctaag aaagtagagg 780
catgatgatc tgcctctcaa ctgccctaag tcctagctaa gtatcagggg aaaaaaaaaa 840
aaaaaaagcc taacaaatgg gattagacta gggctgcaag tagtgaggat tttgttgata 900
cctctgctgg gatgtgtgct ttcccatatc ttgccttcag gaattacact gtgccttttc 960
cccagggata tgggctctgt ctacccagtg ctccagtttc ccggtaactg ctcttgaaca 1020
ttgtggacaa gggcaggtct tcatattttt gatcatccct ttctcccagt gaaatcccat 1080
agcccttacc tagagtctag ggcacaaaga cttcggggaa gatacactga gattgacctg 1140
aggagacatc tacacacacc agtggcagct gccccagggc ctgcttcccc ttcctaagtc 1200
tgtcatcctc tggaagggat gggtggtgct ccaatctctg gtgcctaaaa acccaagttt 1260
atttctctct taacactggc aataaccagt ccacaccact gttgcctttt aaaacctctt 1320
aataatctca tgctgtgttt gttttgattc caatccaatt atcaccaggg ctgtgtgggt 1380

aaatgctttt aaatgctctc tcatcttgtt cttccccctc accccccact cttaggtatg 1440
tatgatgcta atcttgtccc taagtaagtt tcttcctgct ccttttgtat cttcctttct 1500
tgtctttcct cctacctttt gtctcttggt gttttgggac tttttttttt ttttttggcc 1560
ttttgtacaa agattagttt caatgtagtc tgtagcctcc tttgtaaacc aattaaaaag 1620
tttttaata a 1631

<210> 33
<211> 978
<212> DNA
<213> Homo sapiens

<220>
<221> SITE
<222> (2)
<223> n equals a,t,g, or c

<220>
<221> SITE
<222> (27)
<223> n equals a,t,g, or c

<400> 33
angagttgca tgcaagsgta agttggnccc ytsgrggatc tttagagcgg ccgccctttt 60
tttttttttt tgcatgtctg agtttgtgga ataagattca tatttactac aagtaatgga 120
attggagatt cagaggggag aaagtcactt atcacattag tgtaattttc tgatggtagg 180
attatggaga gttttaggtt ttcccttttt ttccccaact tctctcccct cagtatttta 240
aaaataacat tgtgtgggtt gttttttttt gtttttgatt gttttttgtt tttcaaacag 300
gtctcactcc tatccatgta ggctagagtg cagtagtgca atcttggctc actgcagcct 360
cgacttcctg agctcaggtg atcttcccac ctcagtctcc tgagtagctg ggactccagg 420
tgtgtgccac catgcctggc taaatttttg tatttttatt agatacaggg tctcaccatg 480
ttgcccagac tggtcttgaa ctcctgggcc tgcccacctc agcctcccaa agtgctagaa 540
ttacaggcat gtgccaccat atccagccta ataacattgt ttttaatgtt cattaagtca 600
tcccaccctc tcagtcttgc agaagcctct caagagggac agaatcagtt gcaaagtacc 660
atttctgacc ctgagacatg gatattattt gttcatttaa atgtcacctg aaaaacccac 720
tcactcaaat ggtctgtgaa gcttgcaaaa acaggaatgc ttaccctcct gggtcctgaa 780
tttttggttc tcttggactc tttgaaattc ttctttctca gaaaggagcc ctctttctat 840
ttcccctcaa agttgtgact tgaccctcac atccctttct tctccagggc cccttgataa 900
gattctttta aaatttcttt ggagggcatc ccttttagga agacggacgc gtgggtcgac 960
cgggaattcc ggacggta 978

<210> 34
<211> 898
<212> DNA
<213> Homo sapiens

<220>
<221> SITE
<222> (402)
<223> n equals a,t,g, or c

<220>
<221> SITE
<222> (452)
<223> n equals a,t,g, or c

<400> 34
gaattcggca cgagattatg tagtagatgt cactagaatt cttgaaattt gtcttcaagt 60
catggcagta tttcagtatc gctcctttgg gattgcctga gtgatactca agagttagac 120

tagttttatc tgggttcttt gaagaaccgg ggacacctca ctggcttatg ttgaatttct 180
gcactgcagg gaccaactat aaatggtgtt tttggttttt tacgtgttaa gagctttaaa 240
atgtaattct tcctatcatt catgcacaaa tgttctcaca caaattgctt cacagattga 300
taaaactttg aataattttt ccctgaagaa atgttgaact tttctgcaag ctgttggaat 360
kggagcgcgt gttgaaaggc ytgaakggga ccgtactgta cngcctawtt cttttaaaaa 420
aaattawgat ttcyattttt watycattta cngatgactg aatakgtyca ggccagaaaa 480
tatcccctta tttcaaaatg cagcaatcta taaacaaaat acttgccatt tttctaaatg 540
acaccttttt ctataatttg tatagaaaat taagtgcaag ggccaggcac cgtgtaacgc 600
ctgtaatccc agcactttgg gaggccaagg cgggtggatc gcctgaggtc agtagttcaa 660
gaccaccctg gccaacatgg cgaaactcca tctctactaa aaatacaaaa caattagcca 720
ggtgtggtgg cagacgcctg taatcccagc tacttgggag gctgaggcat gagaatcact 780
tgaacccagg aggcagaggt ggcagtgagc tcagatggcg ccattgcact ccagcctggg 840
taacaagagt gaaaactgaa gctgtctcaa aaaaaaaaaa aaaaaaaaaa aactcgga 898

<210> 35
<211> 754
<212> DNA
<213> Homo sapiens

<220>
<221> SITE
<222> (311)
<223> n equals a,t,g, or c

<400> 35
cagcctcatc tcctgttggc cccttgtatg taccctgtgt ttgagttgta atgaacccct 60
gcttgtccat aatctttctt ttaactcctg tgcttctctc tcatcctttg cagagccttc 120
actttctgct taaagtggac cttgacttct ctttatcttg ctccatttgc acctgaaact 180
tgtcctcaac tgcagtgcta attccttggt aatgttttat aactttgtca ggcagctaga 240
cactgtaagt atagaacatg ctgggaaatc caaattaaaa atgacagttg gcacaaagct 300
gacttctggg nagggaccaa ggaaaagtag ccagagtggc aggatagctg cttccatcac 360
ggattgccag caatgtaaag cgtagactcc agaggaacag tgctaactta aattaactat 420
gcaggcatca gtacttctgg ttctgatggc ccggggattt ctaagtagta gtgagtctca 480
gcattatttg ttatacagtc tactgctaga tgaacaaggc taagtctaca gagaaggtaa 540
attatagaaa ttaggccccg tctctgctaa gaatacaaaa aattagccgg gcgcggtggt 600
ggggtcctgt ggtcccagct actcgggagg tgacgcagga gaatggcgtg aacccgggag 660
gcggagcttg cggtgggccg agatagcgcc actgcagtct ggcctgggcg aaagagcgag 720
actccgtctt aaaaaaaaaa aaaaaaaact cgta 754

<210> 36
<211> 699
<212> DNA
<213> Homo sapiens

<220>
<221> SITE
<222> (483)
<223> n equals a,t,g, or c

<400> 36
gaattcggca cgagcggcac gagccacctt ctcagtccag tctatgggta tgacagttta 60
tctgctgaaa acccatcctt gcttctttgt tgcctaccag atgcaggtcg cactcataat 120
cctccttccc ggactcagga acagcaagac tgttactatg ccattgtccc ctgccctcct 180
tcccaccctc cttttttttc cctctcccac tcccttcttt cacccctttc tttctgtttt 240
atgctgcttc aagtattaat tttaaaattg ttctacaaga atgcgattta tcagaaggat 300
gtgaaccaag cagaatttct tagtatttct ttgccttagg gcattcccct tgtgtggktt 360
aaaatttgtc ccccattcct ttttgcctgt ggaacttatc cttattcttc aagagactcc 420
tamtcctaat agcactttga atttaacctc cctggtagtt cttctcagcc aaatttcacc 480

ttnctgaaaa caggattctc tgttctccat gtctggctaa tttttgtatt ttttgtggag 540
acaaagtctc actatgttgc ccaggcaggt ctcaaacacc tggccttaag ccatcctccc 600
accttggcct cccaagtgct gggattataa gcatgtgcca ctggacccag ccagagaccc 660
tgtctcttta aaaaaaaaaa aaaaaaaaaa aaactcgta 699

<210> 37
<211> 971
<212> DNA
<213> Homo sapiens

<400> 37
gccaccgagc cgcagttcct gggtcgcgcg gcagctgtga gcgccgaggg caaggcggtg 60
cagaccgcca tcctgggcgg cgccatgagc gtggtgtcgg cctgcgtgct cctgacccag 120
tgcctcaggg atctggcgca accccgacgg ggcgccaaga tgtcggacca cagggagagg 180
ctgaggaact cggcctgcgc cgtgtctgaa ggctgcaccc tgctatctca ggctttaagg 240
gagaggtctt cgcccaggac tttaccgcca gtgaattcca attctgtgaa ttagcacccc 300
acccccatac cccttcttcc acccccagac taaaggaaga tacttactct ctgcccctct 360
ccatttatac caaagaaatc ataggtgaaa ccccctaccc tccccaacgt taaatgctcg 420
agaggaatct tccacaaggc agggccatgc acgcaacctg cacacgcact tggagggccc 480
aggtgtctct ccaccagccc ccatgcagta gggactggaa gatatgtcat ctgctggttg 540
tgttatcact cccaccccct accccagccc gtsttccgga atttctcaac taaatttsat 600
tattgggcag gaaggaggtc atgggttcat ttcatttttg ttttttgtgt ttttaattaa 660
aagaaaggtt acctcagttt tcactcctta gacatggatg tagctacctt tttttgtatg 720
tctttttttt tttaagcaat cgtgttgaat taggagtata cttggtgtgg aaagagtatg 780
aatttgccat gtgatttgca aatgggggga agctactgtg agcgtgtgtt tttttaattt 840
acactataga gtgatttttt tttcccccaa cgtcaagttt ttaccttgca tgtactggag 900
tatttatttc atctattaaa atgttatgtt tctcagaaaa aaaaaaaaaa aaaaaaaaaa 960
aaaaaactcg a 971

<210> 38
<211> 872
<212> DNA
<213> Homo sapiens

<220>
<221> SITE
<222> (2)
<223> n equals a,t,g, or c

<400> 38
tngcagttct ccacaccgaa gaggacggtg ggcgccaaca gacaggcgat taatgcggct 60
cttacccagg caaccaggac tacagtatac attgtggaca ttcaggacat agattctgca 120
gctcgggccc gacctcactc ctacctcgat gcctactttg tcttccccaa tgggtcagcc 180
ctgaccyttg atgagctgag tgtgatgatc cggaatgatc aggactcgct gatgcagctg 240
ctgcagctgg ggctggtggt gctgggctcc caggagagcc aggagtcaga cctgtcgaaa 300
cagctcatca gtgtcatcat aggattggga gtggctttgc tgctggtcct tgtgatcatg 360
accatggcct tcgtgtgtgt gcggaagagc tacaaccgga agcttcaagc tatgaaggct 420
gccaaggagg ccaggaagac agcagcaggg gtgatgccct cagcccctgc catcccaggg 480
actaacatgt acaacactga gcgagccaac cccatgctga acctccccaa caaagacctg 540
ggcttggagt acctctctcc ctccaatgac ytggactctg tcagcgtcaa ctccctggac 600
gacaactctg tggatgtgga caagaacagt caggaaatca aggagcacag gccaccacac 660
acaccaccag agccagatcc agagcccctg agcgtggtcc tgttaggacg gcaggcaggc 720
gcaagtggac agctggaggg gccatcctac accaacgctg gcctggacac cacggacctg 780
tgacaggggc ccccactctt ctggacccct tgaagaggcc ctaccacacc ctaactgcac 840
ctgtctccct ggagatgaaa atatatgacg ct 872

<210> 39

<211> 608
<212> DNA
<213> Homo sapiens

<220>
<221> SITE
<222> (10)
<223> n equals a,t,g, or c

<220>
<221> SITE
<222> (16)
<223> n equals a,t,g, or c

<400> 39
ccatacgcan accgcntctc cccgcgcgtt ggccgattct tatggcagct ggcacgacag 60
gtttcccgat ggaaagcggg cagtgagcgc aacgcaatta atgtgagtta gctcactcat 120
taggcacccc ggctttacac tttatgcttc cggctcgtat gtkgtgtgga attgtgagcg 180
gataacaatt tcacacagga aacagctatg accatgattt acgccaagct cgaaattaac 240
cctcactaaa gggaacaaaa gctggagctc cacgcggtgg cggccgctct agaactagtg 300
gatcccccgg gctgcaggaa ttcggcacga gtttgggtgg agtttccaag gtgaaagttt 360
ctgaattggt caatcagtga cgcctttgta aagatggctc atgtggtggt cgctcgcaat 420
gaatgcctga taagggcttt tctgtttctt ttgcactgtg taagtttgct cccatcgcct 480
ggggaagtta atatcagaca cacacttttt acggtagaag agaggttgac tactccaagg 540
gcactgaaac tctcactgag ccttattgtt tctctacacg cgamttgcag aaagcaggag 600
tgctcgta 608

<210> 40
<211> 855
<212> DNA
<213> Homo sapiens

<220>
<221> SITE
<222> (850)
<223> n equals a,t,g, or c

<220>
<221> SITE
<222> (851)
<223> n equals a,t,g, or c

<400> 40
ctgtaatagc acacaactca gaactcttca gcatttgtgt gattccttac ctctggctga 60
taaaactcta atgggttgtg gcttactttg tttccatttt ctttggcttt gtgcaatttt 120
tgtgtaactt tacttgtacc tatattttct gtttacagtt ctttttaagg ggaggggtag 180
ggttctaaga tcttgttgtt tattgtagat aaaaattttt tcgtgttgta gaaaagcatg 240
ggttatgcgt ttgactgaaa aagacactgt attatttacc aaaggggtat tgtttttgca 300
tttgtttata aatgcattat tttggtactg taaatttgga cataatttct gagtttatta 360
ctactggcat ttttcttttc cctttttttt ttttttaacc gtaagtgcac gatgcaggtg 420
cataggcccc agaccaaact agaccaccag catgttcatg tccagacctc ggcagtggcg 480
tgcactgctt gtgcacctca gttcctccag tgttggtttg tttgtttttt aattcagcat 540
cctgctggtt ttactttcca agcaagatct gttgcgactc ccaaatgcgt tttaatgagc 600
tcatccttat ttgcctttct tcttacgtat tttgtgtatt agattgtgca ggagatattc 660
tagaaggcat taatggtttg cattcaaaac gatgtggttt gtccaagtta ttttctgtct 720
ttattactga gacggattaa tctccttatt tttttcttga tgatttgaag ttgtaacagt 780
tgtccagcta ttgcttaata aaattttgca gatcaaaaaa aaaaaaaaaa amctcggggg 840
gggccccggn nccca 855

<210> 41
<211> 1042
<212> DNA
<213> Homo sapiens

<400> 41
acggcccgta attcccgggt cgayccacgc gkccgtgctt cctagaaggt cgtgtcacgt 60
ggaacctctt aatctcagca tccggagctc caggaaggga aaatttcaag tcagatagaa 120
ttctatatat accatttctt tggaaccttc agccctcaag attccaacat catgacctca 180
gtttcaacac agttgtcctt agtcctcatg tcactgcttt tggtgctgcc tgttgtggaa 240
gcagtagaag ccggtgatgc aatcgccctt ttgttaggtg tggttctcag cattacaggc 300
atttgtgcct gcttgggggt atatgcacga aaaagaaatg gacagatgtg actttgaaag 360
gcctactgag tcaaacctca ccctgaaaac ctttgcgctt tagaggctaa acctgagmtt 420
tggtgtgtga aaggttccaa gaatcagtaa ataagggagt ttcacatttt tcattgtttc 480
catgaaatgg caacaaacat acatttataa attgaaaaaa aaatgttttc tttacaacaa 540
ataatgcaca gaaaaatgca gcctataatt tgctagttag gtagtcaaag aagtaagatg 600
gctgaaattt acataagtaa tatttcataa tcttagaatt ctctcaaagc atgtgaaata 660
ggaagaagga agttcttgcc cagaatctta ggaaatcacc actgttcggt tataatcact 720
gcctcctgaa tcgttgagga gtcttttaaa ttagattttt gttttgttgt ctcccaagtt 780
aatattatat ttagatatca gagagtcagg yaaaaaggaa aacttttatc tctagggaaa 840
aaacatttag aaaaatgtat tcagtgtatc taatactgaa atgcggaaaa aaatttaatg 900
ttaaaaaaaa actatagaca ttgacatgga aaagagattt aatgttttga aaaaaaactt 960
tatattaact gagtaacatc ctcctgatga gaagtactat attaaatata aacccattat 1020
gttataagtt aaaaaaaaaa tt 1042

<210> 42
<211> 702
<212> DNA
<213> Homo sapiens

<220>
<221> SITE
<222> (515)
<223> n equals a,t,g, or c

<220>
<221> SITE
<222> (614)
<223> n equals a,t,g, or c

<220>
<221> SITE
<222> (673)
<223> n equals a,t,g, or c

<220>
<221> SITE
<222> (677)
<223> n equals a,t,g, or c

<400> 42
gggacaatga actccttctg gtctaagtta ttggtgctgc ccctgctggc tccgctgtcc 60
atggcccgag cctctgcctg tcagagatgg tagagccacc aggacatgga gtcattgctg 120
acacagggaa acatgagatg tcttaggttt ggtgtatgtg aaacatgcat gagaaataga 180
ggccaaaagt tccactgtgg agcgcagaca gaatggtctg aatgctcttg cagttactac 240
gtcagtagtt tgtcatctaa tatatattat acatctataa cctatgtatt taccttattg 300
tgataatact gttttgtttt gttttttttc taattttgct ttgtgcaaag ccaaatccct 360
ttcagcagca ttgagctaaa aaaaaaaaaa agtgcatgtt tagggctggg cacggtggct 420

catgcctata atctcagtac ttcgggaggc cgaggcaggc ggatcacaag gtcaggagtt 480
cgagaccagc ctggccaata tggtgaaatc acgtntctac taaaaataca aaaattagct 540
gggcatggtg gtgggtgcct atagtcccag ctatgcggga ggctgaggca ggaaaaaccg 600
cttgaaccct ggangcggaa attcccagtt gagccaagat cgcgccactg cactcccagc 660
ctggttgaca gancganact cttgtctcca acaaccagca ac 702

<210> 43
<211> 642
<212> DNA
<213> Homo sapiens

<220>
<221> SITE
<222> (593)
<223> n equals a,t,g, or c

<400> 43
aattcggcac gagcggcggg gtcgactgac ggtaacgggg cagagaggct gttcgcagag 60
ctgcggaaga tgaatgccag aggacttgga tctgagctaa aggacagtat tccagttact 120
gaactttcag caagtggacc ttttgaaagt catgatcttc ttcggaaagg tttttcttgt 180
gtgaaaaatg aacttttgcc tagtcatccc cttgaattat cagaaaaaaa tttccagctc 240
aaccaagata aaatgaattt ttccacactg agaaacattc agggtctatt tgctccgcta 300
aaattacaga tggaattcaa ggcagtgcag caggttcagc gtcttccatt tctttcaagc 360
tcaaatcttt cactggatgt tttgaggggt aatgatgaga ctattggatt tgaggatatt 420
cttaatgatc catcacaaag cgaagtcatg ggagagccac acttgatggt ggaatataaa 480
cttggtttac tgtaatagtg tgctgttcat ggaaaccgag ggctgcatct tgtttatagt 540
catctttgta ctgtaatttg atgtacacaa cattaaaagt actgacacct ganaaaaaaa 600
aaaaaaaaaa aaaaaaaaaa aaagcggccg ccgaattaag cc 642

<210> 44
<211> 1219
<212> DNA
<213> Homo sapiens

<220>
<221> SITE
<222> (25)
<223> n equals a,t,g, or c

<220>
<221> SITE
<222> (26)
<223> n equals a,t,g, or c

<400> 44
aattcccggg tcgacccacg cgtcnnctaa aatccccaaa ctgacaggta aatgtagccc 60
tcagagctca gcccaaggca gaatctaaat cacactattt tcgagatcat gtataaaaag 120
aaaaaaaaga agtcatgctg tgtggccaat tataattttt ttcaaagact ttgtcacaaa 180
actgtctata ttagacattt tggagggacc aggaaatgta agacaccaaa tcctccakct 240
cttcagtgtg cctgatgtca cctcatgatt tgctgttact tttttaactc ctgcgccaag 300
gacagtgggt tctgtgtcca cctttgtgct ttgcgaggcc gagcccaggc atctgctcgc 360
ctgccacggc tgaccagaga aggtgcttca ggagctctgc cttagacgac gtgttacagt 420
atgaacacac agcagaggca ccctcgtatg ttttgaaagt tgccttctga aagggcacag 480
ttttaaggaa aagaaaaaga atgtaaaact atactgaccc gttttcagtt ttaaagggtc 540
gtgagaaact ggctggtcca atgggattta cagcaacatt ttccattgct gaagtgaggt 600
agcagctctc ttctgtcagc tgaatgttaa ggatggggaa aaagaatgcc tttaagtttg 660
ctcttaatcg tatggaagct tgagctatgt gttggaagtg ccctggtttt aatccataca 720
caaagacggt acataatcct acaggtttaa atgtacataa aaatatagtt tggaattctt 780

tgctctactg tttacattgc agattgctat aatttcaagg agtgagatta taaataaaat 840
gatgcacttt aggatgtttc ctatttttga aatctgaaca tgaatcattc acatgaccaa 900
aaattgtgtt tttttaaaaa tacatgtcta gtctgtcctt taatagctct cttaaataag 960
ctatgatatt aatcagatca ttaccagtta gcttttaaag cacatttgtt taagactatg 1020
tttttggaaa aatacgctac agaatttttt tttaagctac aaataaatga gatgctacta 1080
attgttttgg aatctgttgt ttctgccaaa ggtaaattaa ctaaagattt attcaggaat 1140
ccccatttga atttgtatga ttcaataaaa gaaaacacca agtaagttat ataaaataaa 1200
aaaaaaaaaa aaaactcga 1219

<210> 45
<211> 437
<212> DNA
<213> Homo sapiens

<220>
<221> SITE
<222> (422)
<223> n equals a,t,g, or c

<220>
<221> SITE
<222> (423)
<223> n equals a,t,g, or c

<220>
<221> SITE
<222> (427)
<223> n equals a,t,g, or c

<220>
<221> SITE
<222> (437)
<223> n equals a,t,g, or c

<400> 45
   gaattcggca cgagggcggc accagggagc ctgggcgccc ggggctccgc cgcgacccca 60
   tcgggtagac cacagaagct ccgggaccct tccggcacct ctggacagcc caggatgctg 120
   ttggccaccc tcctcctcct cctccttgga ggcgctctgg cccatccaga ccggattatt 180
   tttccaaatc atgcttgtga ggacccccca gcagtgctct tagaagtgca gggcacctta 240
   cagaggcccc tggtccggga cagccgcacc tcccctgcca actgcacctg gctcacaaaa 300
   agagtgcaac aaatgcttct attccatagc tacggcattg ctcagtaagt tgaggtcaaa 360
   aataaaggaa tcatacatct caaaaaaaaa aaaaaaaaaa aaaaaaaaaa aaaaaaaaaa 420
   annaaanaaa aaaaaan 437

<210> 46
<211> 533
<212> DNA
<213> Homo sapiens

<220>
<221> SITE
<222> (305)
<223> n equals a,t,g, or c

<400> 46
gaattcggca cgaggaccct atcttacaaa aaagaagaag aagaagaaaa ccatgacagg 60
tgtctttaag ctgcccttgc tgttctgggt tcatgaagca tctgtgggag gttgcccata 120
tgtaaaatta gttgagtttg aagaaatgtt aacgttatat ggtattcttt taattttgtt 180

ttaaaaataa tttttctcat tcaaatcctg aattagaagt tgtttggtat aaatattgaa 240
aattgttgag gggagaattt attcaaagtt taatcatttg ctttatctat gttatactta 300
gctantagtt actggaagtg tcaagtttta tttttagatc ttaactagag tctaaagtaa 360
ttactaaaag ctagttttca aataatatgt aagagtaaag tcctgagtta aaagatttag 420
catactgaat taacttagtt gactgatgct gtacttacat gggcctccta tttcttgtgg 480
ccaagatagc atcaacagaa aaaaaamaaa aaaaaactcg aggggggccc cgg 533

<210> 47
<211> 1849
<212> DNA
<213> Homo sapiens

<220>
<221> SITE
<222> (222)
<223> n equals a,t,g, or c

<220>
<221> SITE
<222> (1300)
<223> n equals a,t,g, or c

<400> 47
gtttttaaaa aattaaacaa ggctttgtgt tcctagaaga gcttcatttc agtgaatctg 60
gtgacctcca tctgcttgct gtcataaccc gacacggact tatttttgtc attagcaagg 120
gggaaaaggc caaaggacaa gggcctcttc tcccattggt tttcctgtgg gcagaagggc 180
tgaggaagat ggcccagccc gtgggggctg ctgggtcacc ancagygggt agggtgcaat 240
ctggtgtgtg ttccagcagt gagacggtgt tattgtgaag gtggcattca tctgcggacc 300
aaaacccagc catcggggaa gggtcagggc ttctgtggaa cttggaacgt gccaggacca 360
cctgcaaaag ccagggtgcg ttgatcattc tcagatcatt gattggcctc cacttgggta 420
tgtgaattat tcatgtccca gaagaccaaa aagtgctctg gttctgagat gagtatttta 480
ttcgtgttct gtttccgaaa cacttagcaa agaaggtcac agtgatgtgg agtcgccgca 540
cccatctttg aagatagcca gtgtccctgg atgaggtgat gatttcccgt cccaaggact 600
ctgtgaagtt tagagtacag tttgttgggg tccaaaagac accatctcta ccccacccaa 660
ataaaaatgc actcatctct gtagaacatc tgctgtcaaa ggccagcctg tcgttagggc 720
atggcttatg cttgacaaac cagtaacaac tgtgggatgg cgatggtggg atgtgtcgca 780
agcaattcac tagacaatct tcacatgaat gtcggtagcc agggtctctc ccgagggatg 840
gctttagtct tgatgaatgt gaaccatgtc ggaattgtta ggtagaaacc tgggctggga 900
ggcctcggac cccaggctcc atccctggct tccccagcct gcggccgcaa gcaaaaccaa 960
gcgcgagatg cagctagcac ccttcatatc catccccgtt ctcagcggga caacaccatg 1020
gacagccgtt ttcagagcct ccagcatttg cacaccacta ctcaccctct ctgctgctgg 1080
catgttggta gagtcatccc tgtaatcaag aaatggcctg tggaatgtta ttgttcaacg 1140
ttgtttacag ctcttaaaac atggtgagga atgcctaagt cttagtgacc aaacgtgacc 1200
ttgaaagcag acatagcatg acagaccttc ctagagtgtt tggtcgggtt cacagtgacc 1260
gagagtcagg tccagcacac acctgggaaa gggatgctgn cccaaggggg accaaaaggg 1320
ccggacgtta cagggtgaaa ccctctgacc cctcgcgaca ccgtaggact tgacttttgt 1380
ttagtctttc taagaaatag atcatggagc caagtgaagt gcactttgtc aaatgtaagg 1440
gtctgctttg ttcttgttgc ttttctgttt tttaaccttt tgttccgcca tttaaaaaaa 1500
gaaaaaaaaa aagcttatgt ttcttgtcaa atgcagaaat gttccttccg ccactcactg 1560
aagttttgca ttctggcttg tgcagttttt attgtctgtg tcagacgtac agccagacat 1620
gttctctatt ggcatttttc cgattctgtt cagatgacag cgaccgcctt ttcattcccc 1680
ccgccacctg tactcaccct cacgctcttt gaagaaaaaa aaaaaaatca ccttgtgtgt 1740
tgtagctcat ttgtttcaag agagaatcaa cagatcatat tcagtgtctt gaataaattg 1800
ctctattttg atattagaaa aaaaaaaaaa aaaaaaaaaa aaaaaaaaa 1849

<210> 48
<211> 926
<212> DNA

<213> Homo sapiens

<400> 48
   ctcaaccaca actagaattt gcacaatata agcttgaaac gaaattcaaa agtggtttaa 60
   atgggagcat cttggctgag aggaaagaac ccctccgatg cctaataaag ttctctagcc 120
   cacatcttct ggaagcattg aaatccttag caccagcggg tattgcagat gctccacttt 180
   ctccactgct cacttgcata cccaacaaga gaatgaatta ttttaaaatt agagataaat 240
   aagacgtgcg tggtttctta agcacagctc ctccttcttg atattgcaca tgcacttcag 300
   ttcatggcta gctgtatagc ttccgtctgt aaacttgtat tttcaagaat ccttggtatt 360
   gaatttttag aaatgctcac ataattgttg ggactgattc attcctccac gatatgcctc 420
   ctctctctga tatcctgcta actgtagccg ttgtggcatt tgagatgaca ggacatatat 480
   atatatggcc ccacacttga ccttgagtgc ctgaatgctc tgaaatcaag catatggcac 540
   agcgctcaag acttttgggt ttgtgtcctt ttttctatgg ctgtctcttc tcaattctgg 600
   agaggtctgg ttccagtggc tggtttcyar ggattgattc ttaagctctg gatcacagag 660
   agaagcaaca aggaactata ctcaactcaa aactttttag gagaatcatg aaattggtct 720
   attcaaagga tggagttgag tccatwmtgt tattgttgca agaggttgca tatttggtga 780
   gtcagttata taaaatagtg ttcttattgt aaatatgata cttctcataa tctattttat 840
   catgtgtata acattcaaac tgacaaatat attgacttat gaataaaggt gtcaaaaaac 900
   aaaaaaaaaa aaaaaaaaaa ctcgta 926

<210> 49
<211> 1593
<212> DNA
<213> Homo sapiens

<400> 49
gcggacgcgt gggctgtgct ccctgcagtc aggactctgg gaccgcaggg gctcccggac 60
cctgactctg cagccgaacc ggcacggttt cgtggggacc caggcttgca aagtgacggt 120
cattttctct ttctttctcc ctcttgagtc cttctgagat gatggctctg ggcgcagcgg 180
gagctacccg ggtctttgtc gcgatggtag cggcggctct cggcggccac cctctgctgg 240
gagtgagcgc caccttgaac tcggttctca attccaacgc tatcaagaac ctgcccccac 300
cgctgggcgg cgctgcgggg cacccaggct ctgcagtcag cgccgcgccg ggaatcctgt 360
acccgggcgg gaataagtac cagaccattg acaactacca gccgtacccg tgcgcagagg 420
acgaggagtg cggcactgat gagtactgcg ctagtcccac ccgcggaggg gacgcaggcg 480
tgcaaatctg tctcgcctgc aggaagcgcc gaaaacgctg catgcktcam gctatgtgct 540
gccccgggaa ttactgcaaa aatggaatat gtgtgtcttc tgatcaaaat catttccgag 600
gagaaattga ggaaaccatc actgaaagct ttggtaatga tcatagcacc ttggatgggt 660
attccagaag aaccaccttg tcttcaaaaa tgtatcacac caaaggacaa gaaggttctg 720
tttgtctccg gtcatcagac tgtgcctcag gattgtgttg tgctagacac ttctggtcca 780
agatctgtaa acctgtcctg aaagaaggtc aagtgtgtac caagcatagg agaaaaggct 840
ctcatggact agaaatattc cagcgttgtt actgtggaga aggtctgtct tgccggatac 900
agaaagatca ccatcaagcc agtaattctt ctaggcttca cacttgtcag agacactaaa 960
ccagctatcc aaatgcagtg aactcctttt atataataga tgctatgaaa accttttatg 1020
accttcatca actcaatcct aaggatatac aagttctgtg gtttcagtta agcattccaa 1080
taacaccttc caaaaacctg gagtgtaaga gctttgtttc tttatggaac tcccctgtga 1140
ttgcagtaaa ttactgtatt gtaaattctc agtgtggcac ttacctgtaa atgcaatgaa 1200
acttttaatt atttttctaa aggtgctgca ctgcctattt ttcctcttgt tatgtaaatt 1260
tttgtacaca ttgattgtta tcttgactga caaatattct atattgaact gaagtaaatc 1320
atttcagctt atagttctta aaagcataac cctttacccc atttaattct agagtctaga 1380
acgcaaggat ctcttggaat gacaaatgat aggtacctaa aatgtaacat gaaaatacta 1440
gcttattttc tgaaatgtac tatcttaatg cttaaattat atttcccttt aggctgtgat 1500
agtttttgaa ataaaattta acatttaata tcaaaaaaaa aaaaaaaaaa aaaaaaaaaa 1560
ctcgaggtcg acggtatcga taagcttgat atc 1593

<210> 50
<211> 978
<212> DNA
<213> Homo sapiens

<400> 50
gaattcggca cgagatgagt ttggccacgt gatgcaccag ctctgctccc aggtgggtgc 60
gggcccgggc aggggcaggg gcaggggcag gggcaggggc tgcctgtggt cagcgaggcc 120
caagcctggg gcttcggctt ccggctctct ctgcacccgt ccggtggctc cttcatccaa 180
tgccacccaa agatggtgac tccctgtcat gcccgtgtcc tggggctgcc ccagcaaaac 240
accacagacc agggcttaca caaggtgcgt gtatttcctc atggtcctag aggctggagt 300
cggaggtcac agtgtcagca gggttggctc cctcgargtc cctccttggc ttgtggccgc 360
caacaacttc ccgcatctca tgtggtcgtc cttctgtgtg ggtccccaty tygtcttctt 420
acrggacccc agtctgccgg atccgggccc gcccaacaac ctcacttgac ctagtgacct 480
ccttagacat ctgtctctaa gtagtcacat ctgggattac ggcgtgagcc atgttcccgc 540
ggaatttctt ttttatagta ttggataaag tttggtgttt ttacagagga gaagcaatgg 600
gtcttagctc tttctctatt atgttatcat cctccctttt ttgtacaata tgttgtttac 660
ctgaaaggaa ggtttctatt cgttggttgt ggacctggac aaagtccaag tctgtggaac 720
ttaaaacctt gaaggtctgt cataggactc tggacaatct cacaccttag ctattcccag 780
ggaaccccag ggggcaactg acattgctcc aagatgttct cctgatgtag cttgagatat 840
aaaggaaagg ccctgcacag gtggctgttt cttgtctgtt atgtcagagg aacagtcctg 900
ttcagaaagg ggctcttctg agcagaaatg gctaataaac tttgtgctga tctggaaaaa 960
aaaaaaaaaa aaactcga 978

<210> 51
<211> 433
<212> DNA
<213> Homo sapiens

<220>
<221> SITE
<222> (424)
<223> n equals a,t,g, or c

<220>
<221> SITE
<222> (430)
<223> n equals a,t,g, or c

<220>
<221> SITE
<222> (431)
<223> n equals a,t,g, or c

<400> 51
   cggccgctct agaactagtg gatcccccgg gctgcaggaa ttcggcacga ggcgggaagg 60
   cttattccaa ggtaagaggg gctgtgtgaa ggggcagtgg gatggaatgg ggggtggcat 120
   gggacaggca caagggaagc ctccagcccc ttttctgcca caagcaagag gcactcagcc 180
   ctacctgaga tgtgttattt tttagaaata tctttattga tggtctttgc actcaatata 240
   aaggcagcat atggttgttg caatataaat ggtacagaag tccacagagc aaaagggcca 300
   gtttctgtcc cctttcctct ctccaggcct ctttctggga ccccattatt ggatagatta 360
   agacctttcc agaccttgta aaaaaaaaaa aaaaaaactc ggggggggsc ccggaaacca 420
   attngccccn naa 433

<210> 52
<211> 861
<212> DNA
<213> Homo sapiens

<400> 52
gaattcggca cgagcctgag tcaacttgat atccaagctt tttacttcaa ttatctggca 60
agattacata gactgtcaaa gtttgtgaaa gtttagcaag aaaactgtct tactcacaga 120

accacaggac taactgactg aaccacactc caccatttgc ccctatttcc aggcgttatg 180
gtcaccctgt agtttctaat ctgtatagat gtgtagagca tgcctcttcc ctcttccttt 240
cccctccctg ttttcctttc ctcttgccct ttcttaatgt ctgtytctat tggcttcttg 300
atcttggtct ttaatgttca tccttaagct tgcttctctc ttcagactac tgattcagcc 360
tcttgcattt tctttcaact tgggccaaaa aaacaggcaa cattttcttc ctccactacc 420
tcatcatcat ccaatttatt cctttagttt atattaccac aactctccta aacgtcccaa 480
gtctattatt aagtctaaca acttagcttc gaacctcaat ccaagcatct gacaacacac 540
tgaaatgtgc aagcaagagt cccwatggcc gggtgcagtg gctcatgcct gtaatcccag 600
cactttggga ggccaaggtg ggatcacctg aggtcgggag ttcgggacca gcctggccag 660
tatggtgaag ccatgtctmw actaaaaata caaaattagc cggacattgt ggtgcacgtc 720
tgtcatccca gcaaggcagg cgaatcgctt gaacccggga ggcggaggtt gcggtgagcc 780
gggatcgtgc cattgcactc cagcctggtc aacagagcga gactccgcct cattaaaaaa 840
aaaaaaaaaa aaaactcgta g 861

<210> 53
<211> 510
<212> DNA
<213> Homo sapiens

<220>
<221> SITE
<222> (380)
<223> n equals a,t,g, or c

<220>
<221> SITE
<222> (396)
<223> n equals a,t,g, or c

<400> 53
gatcccccgg gctgcaggaa ttccgcacga gtgaaaaccg cctccaccaa cacccccgtt 60
tgcctacacc accccccttt tacttagtat gtttattttt tgtgtgtctc ttgccttcct 120
cccacgtttt atttcccctc agagctgtga atgggcaggt ctgtctctgg tttggcatca 180
ctgagttttt cccatgcatt ggccccaggg ctgctaggat gtgagacaaa tctccctaca 240
atgggcttgc tcccattgtc tgtacagttt aatagatgct ggcatgtcgg aggttaccca 300
tgagtcaaaa tccgctctcc atgcttactc ttgacacccc attgaagcca ctcattgtgt 360
gtgcgtctgg gtgtgaagtn ccagctccgt gtggtncctg tgcttgtact gyccctgctt 420
tgcagttcct ttgcacttac tcatcgagtg ctgttttgaa atgctgacat tatataaacg 480
taaaagaaaa aaaaaaaaaa aaaactcgta 510

<210> 54
<211> 309
<212> DNA
<213> Homo sapiens

<220>
<221> SITE
<222> (301)
<223> n equals a,t,g, or c

<220>
<221> SITE
<222> (305)
<223> n equals a,t,g, or c

<400> 54
gaattccccg ggataaattt catttcccaa agatgagtag gtatgaaaaa taatactcag 60
aagagattgt tcttgtgggg agaactgctt ctacaggatc tagctttgat tttgtatctt 120

tcaatctttt taaaatcaac tttaacgaat ttaaacctat tttaagtgta caagtaataa 180
gtttgacaat tgtatgtgac ttctaccaca ataaaatata gaacattttt atcattctat 240
aaaaaaaaaa aaaaaaaaac tcgagggggg gcccggtacc caattcgccc tatagtgagt 300
ngtancgtc 309

<210> 55
<211> 1585
<212> DNA
<213> Homo sapiens

<400> 55
ggaatttctt aaatatgttc atgtataata cttgatcaaa atatttttgg gttttttgtt 60
ttgttttaat gggttagaaa atgtttacaa tcttggtctt atatgatcac caatggaata 120
gtaacttcca ggtttatatc aatatgagct gactttaact gagttgtttg ggatagggaa 180
gaagcagtcc ctctacagta tacaactact gcttgccagc tggatcaaaa taatcatgtt 240
ttatgaaaat atctccctta agcagtgtta aggttggttt gcagtgtgta agtggcacat 300
tgaactggaa gttttcttga aagctgcttc atctattaag aagcaatttt caaattgtag 360
cgaattatat tatcccctct tttaaagaaa cagtcgttat atgctgatgt ttcttaaaat 420
aactaaaatg tkcctcttaa tgtgatttta aatggagtta tttgtaggtc ctttcttagt 480
agtaaagaat cttctagagg gaaacatttg tgcttttagg gataatcttc cttgtgcctc 540
actacatccc taagtgggta tgactcttgt tattaccaca tgctttttta gtatatttca 600
caaatttact tttaaatatt attttagata cggtgtaaca tgtgcaattc agaataattt 660
tataacaggt catgaaaaac ataactttag ttaggattca caatatttgt wctccacata 720
atgagagaat gaatgagcct ttggagatac tgatataagg caattatttt ttgcaatgtt 780
gaatgtgttt tttagtttga ttcttttttt ttcccccaat agggcactac ctgccatatc 840
atcttgtatt actttttgat gtaaagcgac taatatttac actatgccat atttttttta 900
attatagttg taaattatga aagatccttg aattttctac agatctacaa ctactaatgt 960
aacagacaag ggcaatcttg gtatttaaat ctgagcatgg cagttctacc ataaaaagta 1020
ctctattttt ctaatttcta ggatttttaa aataacattt ctgtaagtct gacatactaa 1080
tagtcactca agcagtacca tttattttag tttgcatata ttttcactgt ttttaattta 1140
atgtattgag tctaatagga ctgttttgca ataattrgaa taaagattta tttcttctaa 1200
tcaaagatgc ataacagcta ttatctaggg gaccmccaaa tgtgatttca aaattttgtt 1260
aactattaca aatgtaatcc ttatatagaa attttaattt tgtaaagtag tgtataatat 1320
tgtaatatta aattcttgtt cttaaattca aatatgtatt gatcttcaat gtgctgtgtt 1380
aaatcttgct tctctgaaaa gttggagaca agatttgtct tcctttttac agtttgtaat 1440
tttcactgtt ttattcctgt taaaaaaaaa aaaaagtcat ttgtaaccca tgcagaccat 1500
tgtttgatct atgctaactt atcaacttgg ctattcaata aagttaattg aaaagaaaaa 1560
aaaaaaaaaa aaaaaaaaaa ctcga 1585

<210> 56
<211> 874
<212> DNA
<213> Homo sapiens

<220>
<221> SITE
<222> (468)
<223> n equals a,t,g, or c

<220>
<221> SITE
<222> (501)
<223> n equals a,t,g, or c

<220>
<221> SITE
<222> (546)
<223> n equals a,t,g, or c

<400> 56
aggggaatct cggtgctgcg acgagtgtgg ggccagccgt ggaggctcca ggtgttctct 60
ctgccccagc agagcccggc aggagcccca acaggaagcc agcgcggcat ggctgccacc 120
gacttcgtgc aggagatgcg cgccgtgggc gagaggctgc tgctcaagct gcagagactg 180
ccccaggctg agcccgtgga gatcgtggcc ttctcagtca tcatcctttt cacagctact 240
gttctgctgt tgctgctgat agcctgcagc tgctgctgca ctcactgctg ctgccctgag 300
cggagaggca ggaaggtcca ggtgcagccg acaccaccat gacggacggg cgatggctga 360
ggagaagctg gagaggagat ggccaatgcc atgacacagg ccatcagcct ggccctgcag 420
cccttacccc tcaagaccag gctcccctgg ccccagctct ggcccagncc caggtacctg 480
gacactgaca acttgagccc ntaccaagga aacaagggct ggtataggtg caaacctctc 540
atctgnccag tggacactgg gtgctgggga gtcagctgtt tcaaagactg ggtcaactgc 600
ctgggcttct tcgcctacct gcacttttta acaaaacaag gaagtagggg tccccatacc 660
ttgatggaga acagtcccca cctgtgggca attggccctt ggggctctgc tgatacatgc 720
caaagaggag caaggcaatc agaggggctt tgtgcaatag cttctgcatc cgagctcccg 780
ccagagcgtg agcatgtcag tattctagtc cagtatttgc cagtttccaa gtaaaagctt 840
ttgtgttaaa aaaaaaaaaa aaaaaaaact cgta 874

<210> 57
<211> 1169
<212> DNA
<213> Homo sapiens

<220>
<221> SITE
<222> (2)
<223> n equals a,t,g, or c

<220>
<221> SITE
<222> (9)
<223> n equals a,t,g, or c

<400> 57
gngcggccnc cctttttttt tttttttata ttttatcaat tttattgaaa tattccaagg 60
atcccaaccc catttaaaaa taaaaattgt aaagcactcc attcaataaa agcacataag 120
tccccctcaa taattagtat gacaattcac gatacagctc ttactctggg agagtttatt 180
ttacccttta ttccaaaagg cacaaagtca tctgaggcct cagatattaa ccccactgca 240
tgttaatgac acaccactga ggtgcagctc aatgtaatta ttaaagctta taacacactt 300
ccccaagaat ttatagattc tttctataaa taataattta aaaaatactg caccttaaga 360
ccaatacagg cttaacaaaa gacctgaaat ttctgcaagg gcagttttgt ttcttgatag 420
aagtacaact tttgaaagtc tattcccagc aaaagaaaca ctagacccag cttggccaaa 480
gaaacaaaat aaaacaagtg atttctaaca cgctaaaaga gtacattttc atcagctcca 540
aagaaagcag tcctggtcat tcagaaggct cctatgatcc caccagtctg cagtcattag 600
aaatatatgc tttacaggcc acaggctgct ctggatttgg tttcagacac cagtgaccag 660
aagaagccag ttttgcgtgt gaggggtgtg ggcccccgct gccttgggcc tgctcaccgg 720
ggtggatgga cccccgccgg gtcacagcct gctgtcacgt ctggactgtt ggcctcttct 780
gcatctgggc tgttgggctc tcctgctctc tgtccctcag tcacgtcatt gtctggctgt 840
ccggtgctgg ctgcactctc atttgtgagg ataacccctt ccttcttctt ttctcccaat 900
acctccagcc ccatcatcct gagataatga agccgttcat tcttgggcac aaaagttcga 960
atggaggcct ttccccgcca tccgcataag acgatgggac actgcagagc gtctggattc 1020
gcagaatctg gttcatactt cagcacgatg cttccctttg ccaggtcctt tgcttgactg 1080
taggtctcac tgctgagttt tctaaaaaag ggattttcct gggtcaacag tatcttaaca 1140
tcttccattg atacagtaat aattctttg 1169

<210> 58
<211> 1066
<212> DNA

<213> Homo sapiens

<400> 58
gaattcggca cgagcaaatg ttgaaccaat tatgttttgg tggtggtgtt cttagctgtt 60
gaatcctgaa tggtttataa agtgaactag ctggcttaat gcagccagcg ttctgggcag 120
cagaacatat tcattcttac tgtaaattct atttgctgct tccaaaggtg atgattttca 180
agcagacatg ttctatatgg tctgtgtttt aggatctggt gcccagcctc tatcagagct 240
tgcctacctg gcaaagctgc ctacccttca agtgggaaaa tataatccac tgtttaacaa 300
ggctcaccct ctccaccctg tcctaacgac cttttgtgaa tgtgctgtga tattttcttg 360
ctcaatagca aggtggtagc tctgctttca ttttaagaaa gtggaggctg agggcattgt 420
atcaatactg ttgcaactcc aagaagtttt ccttgtaaaa ttaaaggaaa gatcttgtta 480
ttgattaacc attttcttat gccttgctat tgacatattc atgctctttc tacgtctagt 540
ggctgaaaat gtttgcattt gttcatttga ctaatggtgt gatttttgky ycwatattat 600
tagacctgta atgttttaaa atgtatttta ttaaatttgg actggatgta tgkcctctag 660
caatacgagg tactttctaa actattaagg gaggggttgt aycctcatgt tgagataaga 720
tgatggtcgt ttaaattttg caattttttt tggcctgcag ggatattttg tgtttatgtg 780
tccaaaaaag gaataaattg gcattcttgt gccaaaagtt gtttttcctg tcaattgtct 840
aataagtatg cagtacactg taatggcaac atacatggtt gctttataaa aacagtttcc 900
tcagtatgag aaattttaca aagaacagtg gaaaaacttt gtgtttttaa ctcttgggtc 960
tccctatttt taaaaattgc tatttggtat acaattatta tgtgtcaatt aaaactaaaa 1020
taaaactttt aaaaaaraaa aaaaaaaaaa aaaaaaaaaa ctcgta 1066

<210> 59
<211> 772
<212> DNA
<213> Homo sapiens

<400> 59
gaattcggca cgagctttcc tgagcctcag tttctccaac ggtgggaggt ggtagaaatt 60
gatatagtac ttaccactga gggtaaaatg agatataacc tgtgtaaata ctgtacacca 120
cagtcattca atagtggcag cttaaaaaaa ttattctacg attacccttg cttcagtgat 180
tcttcttggt gttattgaag ggtgagatct cggtggggat ctcccaggtg tttccataat 240
cccagcgatc accccaggga gaacctctct ccttaggctg ctagaggaca tgtgccatag 300
gaccagatag gagggagggg cagcggtggg aatgcgtttt cagagctacc tttggccaag 360
ccgtatcctt gtggggacct attgcattgc tgctgaagtg ctgttcccat cagccctggc 420
ttcgtgtggc cctgtctggc aagggggtgc tcctacaaag tcatggcagc ctggtgccaa 480
aaccatcatc ccataggacc tgctgtagct ttgccagaag cctggcccaa ggggtggagg 540
cccctggagc tctgacccac cacgtggagg gtgggaaatg ccacagagca ggttctctag 600
aagggatttg tcagaagcta aactggggtg ccccctgggc tcaggcctgc acagtttctc 660
cctgaccacc cagctgggat ggatatagag acaggtgtca tgttgcagaa agcctgccct 720
aagaggccct actggtgttt tcctttatta aaaaaaaaaa aaaaaaactc ga 772

<210> 60
<211> 1198
<212> DNA
<213> Homo sapiens

<220>
<221> SITE
<222> (1189)
<223> n equals a,t,g, or c

<220>
<221> SITE
<222> (1191)
<223> n equals a,t,g, or c

<400> 60

tcgacccacg cgtccgattt aattcttatt ccccacagtt taggtatttt tcattagtac 60
atcaatttga cacactgaat gcaagactat taaggaagaa cgattaaata ttattttatt 120
ttgtgaagag ttggcagcag attacatctc aagaacttgc agagagagga aggtagatgg 180
acaatcctaa attgtaagat gttacaaaaa acagtgaagt aagagtactc ctgaagacta 240
aaatagagag gctggggttt gagccatttt actgagtagc ttagctggaa cctgatatca 300
gaagtagcct ttaacaaaaa gcctcttggc aattgtatgg tactaacaac tagagtactg 360
aagtgtaagt tgaaaccaag ttgcagtggg aaatcaaagg tgaggtagct tatttgaaac 420
cagcaaatga gacaggttgg acagttttaa aatctcttct aacaaagaaa ctgcacggta 480
gcaaggacta gcggttctca aagcccttct ttttcagtgt tctcattcac cttggcaccc 540
aagtatgttt aacaggccat gcattaaaaa taaatacaaa aatataaaag ccgcttaaag 600
ggaacttaca aactgacaat ctctcctctg tatttgtgtt catagtggct gggagtttaa 660
ttatatgcac aaaagttagg agccacttgt ttctgcacag actgtaggag caagatgagg 720
agatgggcag gttttggtaa gagcccccag ttctggtgga caggcatact tgtggcattg 780
ggtgcggcat tgctgggagg accacgtctt gggaggcgat tgacttttgg tttgtaattt 840
ccctttaaac aagaagagat ggctcacatt ttccatatat atctcaatga atgtactgta 900
ttactgtttt aaaaatttga tgaaataata atgaattggt ctccttttgt tatctggtcc 960
ttgtttaatt tgtttaaggg tttttgtata caaaagttta catttttatg tatatttttc 1020
ttgtgtaaaa actgatgtaa tatgtgtatg aaacactgta tgtattatct gtatatagtg 1080
tgacaaaatc atttttcttt ctttcttttg gatgtattaa taaatcttgc tgtgaagtaa 1140
aaaaaaaaaa aaaaaaactc gagggggggc ccggtaccca ataaccctnt natgatct 1198

<210> 61
<211> 558
<212> DNA
<213> Homo sapiens

<400> 61
ctgcaggaat tcagcacgag ytggcatgtg acaacccagg gctgcctgaa aatggatacc 60
aaatcctgta caagcgactc tacctgccag gagagtccct caccttcatg tgctacgaag 120
gctttgagct catgggtgaa gtgaccatcc gctgcatcct gggacagcca tcccactgga 180
acgggcccct gcccgtgtgt aaagtagcag aagcggcagc agagacgtcg ctggaagggg 240
ggaacatggc cctggctatc ttcatcccgg tcctcatcat ctccttactg ctgggaggag 300
cctacattta catcacaaga tgtcgctact attccaacct ccgcctgcct ctgatgtact 360
cccaccccta cagccagatc accgtggaaa ccgagtttga caaccccatt tacgagacag 420
gggaaaccag agagtatgag gtttctatct aaagagagct acacttgaga aggggacttg 480
tgaactcaac cacaatctcc tcgagggggg gccggtaccc aattcgscct atagtgagtc 540
gtattacaat taatgggc 558

<210> 62
<211> 616
<212> DNA
<213> Homo sapiens

<400> 62
gaattcggca cgagtcttga cagcctggtc accaagggtt tggaaaaagg ttctattgga 60
gtggagattg atgggtggaa aaaggagaga ggggagttgg acctgatacc aaagagatgt 120
tttcagccat caaccagctg caaaacaaga tgggcttcct tttcctacat attcttccaa 180
gcatcataaa tactcggtct gctccccaac ccacatcctg caggatgcag ccagagcaac 240
agccccactc cactctgaaa ccagtcatcc tagggatgat gatcatttct tagcttccct 300
gttggaggtc ggttggggtt ggctgatcgc tgcttggttc actcctgcac tggctgggcg 360
ttggctgcat ggtaaagctg ttccctgtct catcctgttg ggataaacag agtatcctag 420
gcatattttc tccagagcag tggcagacac aaagggtcaa cagaaaccct caaggttttg 480
tcatgcctac tcttgcaact agcacattgt catttcagcc tcatgctatt gaccaaagca 540
agtcacttga ccaaattcaa agccacaaaa ctcgtgccga attcgatatc aagcttatcg 600
ataccgtcga cctcga 616

<210> 63

<211> 811
<212> DNA
<213> Homo sapiens

<400> 63
gaattcggca cgaggagctt ccatcttttc tgatgtgagt ggtgtcagga atgactatgg 60
tggtggtagt ggcagtggcg atggttttct ggaggctgaa aggttaaagt cccaatgcag 120
aagtgatgtc agggctagtg ggtggcggta gcaggtgcag taaagtcagg ttcagatgct 180
tcaatggtga ctcccttctc gtgttagtcc tacagcatca tttcagactt tgttcttggt 240
gcttagctcc aagcctcttc ctcctgctgt cctgtcaggt tgtgtccact atgatggagc 300
aagaccctgt catctatgat gatgatgacg acttgcctaa ttatttttct gtttaagcta 360
gccatagtgg atcctgttat ttgtgcctaa gagctcttac tgacaaagaa cgtgttaccg 420
gaagtgggat gctacaagta acaacactaa aagtagaatt gactaagtgc agcaggcagg 480
cctttgagca aggaggggac acacattaca ggctggaaag ctggtgactc ttgtaatgca 540
gtggcaaaat tttgcttcaa ctactatata caatacttga agatgcacac tgcaagctga 600
gtgaggctgt gataagaggg gaaatagtgg ggagcattca gaatgttggt ttacattgat 660
gacttcttgc tctttcagca gtcttgatag agcagctata cccacaccag agtcctccag 720
ctgacaagag aggtaaggag agaaactgct ttgccaggag gggccctctg ctgcagctgg 780
aggtccaagt tgaccgagag cccaaatttt g 811

<210> 64
<211> 993
<212> DNA
<213> Homo sapiens

<220>
<221> SITE
<222> (370)
<223> n equals a,t,g, or c

<400> 64
ggcacgagcc caaagtgctg ggattacagg gagttgatga aagtggagat gtttttagag 60
ctacctatgc agcattcaga tgttctccta tttctggtct gctggaaagc catgggatcc 120
aaaaagtctc catcacattt ttscccagag gtagggggga ttatscccag ttttgggatg 180
ttgaatgtca ccctcttaag gagcctcaca tgaaacacac gttgagattc caactctctg 240
gacaaagcat cgaagcagaa aatgagcctg aaaacgcatg cctttccacg gattccctca 300
ttaaaataga tcatttagtt aagccccgaa gacaagctgt gtcagargct tctgctcgca 360
tacctgacan gcagcttgat gtgactgctc gtggagttta tgccccagag gatgtgtaca 420
ggttcctgcc gactagtgtg ggggaatcac ggacacttaa agtcaatctg cgaaataatt 480
cttttattac acactcactg aagtttttga gtcccagaga gccattctat gtcaaacatt 540
ccaagtactc tttgagagcc cagcattaac atcaacatgc ccgtgcagtt caaaccgaag 600
tcccgcaggc aaatttgaag ctttgcttgt cattcaaaca gatgaaggca agagtattgc 660
tattcgacta attggtgaag ctcttggaaa aaattaacta gaatacattt ttgtgtaaag 720
taaattacat aagttgtatt ttgttaactt tatctttcta cactacaatt atgcytttgt 780
atatatattt tgtatgatgg atatctataa ttgtagattt tgtttttaca agctaatact 840
gaagactcga ctgaaatatt atgtatctag cccatagtat tgtacttaac ttttacaggt 900
gagaagagag ttctgtgttt gcattgatta tgatattctg aataaatatg gaatatattt 960
taaaaaaaaa aaaaaaaaaa aaaaaaaaaa att 993

<210> 65
<211> 689
<212> DNA
<213> Homo sapiens

<400> 65
gaattcggca cgagctaagg tgggcgggtc acttaagcct cgaactcctg gcctcaagca 60
atcctcctgc ctttccttcc caaagctatg aaattgcaga caggagccac catgcctggc 120
tggtttttgg gggccatggc aagtgcaggc ttgtcagagg aattggagaa gcagggatta 180

gttaggaaaa cctctccact tcttgtgttt catgccaggt agtgtttgta acttcagaac 240
ccgcccttac cttacctacc taccatgtta tgctcatttc acctactgtc ccctgctgta 300
tagggagtgc cttgagggca gagatcatgt tagttttgtt ccctcttctg tacagagggt 360
ggagcccagt acctggcaca gctgaaggag gaatgtgctg ctgctgtctc tgtatttcca 420
ggtactcctt gttgacctct agccaagaca aggaacctcc ttatgagatg tcatcttctg 480
agctctcttg atggagggaa taccacggtg atgattgaat atgaaaagtc ttggcacagt 540
ggctcacacc tgtaatccca acactttggg tggccgaggt gggaggattg cttgaagcca 600
ggcattgaga ccatccttgg ccaccaaacg agaccccatc tctacaaaaa aagaaaaaca 660
aaaccaaaaa aaaaaaaaaa aaactcgta 689

<210> 66
<211> 942
<212> DNA
<213> Homo sapiens

<400> 66
gaattccagg actgctggga ccccctgcac ctcctggcca cggagagatc ctgctcccag 60
ggaccagcgt ctgggtggga cacagttcac tcctctctcc acttcatgtt ctttttcttc 120
agcagatggc tcaagttcct tgtttttctc cttgctttct gacagccgta gcttctgaaa 180
cctgccattt ttggtctcct gatgcctgat ttcctaattg tcctgactgt gtcttctagg 240
aagcattaag tctgaactga cttattaggg aacttcagaa agttaaacac acaaaaccct 300
ttctttgact cctatcttaa ggacatggag atacagttac atatatttat acacaaggat 360
attcatatgg caaaaacggg gagaaggcac aatttaagag cccaatgggg actgggattg 420
tgtatgcatc tgtacaatga catgttatga agtcattctg tttttataa aactttttag 480
tgacatggga aaatacaaag aatgtaaaga atttaaaaag cagcgtacaa aacmatatat 540
gtgatccaat ttgtggtgga aatattttat ctatatatat ccattttaaa mcaccaarga 600
aaatacacag ttaacagtag ttatctttgg aaggcaggat tataagtgat cttagttttc 660
ttccttccac ttttgttacc gatatcagaa aaaaactctg tctctacgaa aataaaataa 720
aatgaaataa aataaaatta gctgggtgca gtggctcatg cctgttgcct cagctcctca 780
ggaggctgag gcgggagaat cacttgggcc cggcaggtcg aggctgcagt gagctaggat 840
cgtgccactg cactctagcc tgggtggcag caagaccttg tctcaaaaaa aaaaaaaaaa 900
aaaggaattc gatatcaagc ttatcgatac cgtcgacctc ga 942

<210> 67
<211> 2309
<212> DNA
<213> Homo sapiens

<220>
<221> SITE
<222> (13)
<223> n equals a,t,g, or c

<220>
<221> SITE
<222> (652)
<223> n equals a,t,g, or c

<220>
<221> SITE
<222> (677)
<223> n equals a,t,g, or c

<400> 67
ggtaagagag aangtgtgaa gtcacctgtc tgttctagtc attccaatgg acattgtact 60
ggcccaggag gaaagaacca gatgtggttg tccagtcatc caaagcaagt ctctagcaca 120
aagcccgttc cactgaactg cccttctcca gtgcctcctc tgtatttgga tgatgatgga 180
ctcccctttc ccacggatgt gatccagcat aggttacggc aaatcgaagc agggtacaaa 240

caagaggtgg agcagctacg tcgacaggtg cgtgasttca gatgaggctg gacatccgtc 300
actgctgtgc ccctccagca gagcccccca tggactatga ggatgatttt acatgtttga 360
aggagtcaga tggcagtgat actgaggatt ttggctctga tcacagtgaa gactgccttt 420
cagaagcaag ctgggaacct gttgataaga aagagactga ggtgactcgc tgggttccag 480
accatatggc atcacactgc tataactgtg actgtgaatt ctggttggcc aaacgaagac 540
accattgcag aaattgtggg aatgtatttt gtgctggatg ctgccacctg aagctgccca 600
ttcctgatca gcaactctat gacccagttc tcgtctgtaa ctcatgttac gnaacacatt 660
caagtctctc gtgccanggg aactcatgag ccaacagctg aagaaaccca ttgctacagc 720
ttccagttga atgccgggga gaaacctgtc caattttagc aggtttgaag ggaggatctt 780
cttcagttgt agtttggaag gttccttggt gtggctcatg aaatcacaga gctcagagat 840
accatcttga gaaatcctcc ttggtatcat gaaactggag cagaggaatt gcaatttagc 900
aggaggtcct ctactggtga taccctcacc ttggggtaat ggtcctaacc cagacccagg 960
gtctggaaag cttaatgttg agttggtgac tccagcctct ttctcctgga ggtcacaaga 1020
tgatgattgc gtagatgttg cctggtgcaa agtgccccaa acagcaatag aaaggcatat 1080
gtataaccaa actccaagtg ataaccagac ccatctctcc tccaccttga caaaagcaga 1140
ttatagtata caaggtagga attcctgtcc tatttgagat gaactatatc ctgtacctct 1200
gtgctctgtg tctgcatgaa ggctcagcct ttagaggcac tccttctagt tgcattagta 1260
ctgtctttct gtggagtttg gtttgaagac tggctcagca agtggaggtt tcaatgtatt 1320
tttcagttgg ctcatcagcc agcattggtg aatattcagt ttaggggaac agttctaggg 1380
agtgagacat ttttgggagc agaggaaaac tctgctgatg ttcggtcctg gcaaacattg 1440
agttattttg agctgtgaag gcagtcgtct ctgttacaca gtggcagctc ttgagttatg 1500
cactgtgaag aatgagaagg gaaaagcaaa aattatcctt gtgaaatatc tgctgattgt 1560
gccctactct ttgcacctga cttttcctag ttgtcctggt gctaacacag gagctacacc 1620
ttgatcctct cctggcatga aaataaaaca aaggttttcg ttgttgttgt tccattgccc 1680
atttccccca tgttgtcttt cccttggctg atgcctcctc tgggtcacat tgcttcttat 1740
cctgaacact tgacaccttg agggtagaat ttagcgtttg gtttttacct cctagcatat 1800
gctgtttggt atgtgagggt ttcagtacaa atgctgctgt ctatttctgt gcacttaaca 1860
atggaaccca aacagaagag aataaagcct tgataccaaa attgggaaag aacatgtgtc 1920
catttggacc aaacgttgtt ggtttttaaa aattttattt ttgttttttt gtttttgttt 1980
ttgttttttt tcatcttaat atgtaccagt ggcacttaac caaaagatac agtgatatag 2040
ccatgtatct gtctacttag cgtggctgtt ttgagggact gtcccatcag tgaacaaact 2100
gcatggcctt ggagagagac tctgggctct tggctcagat gtgttcatca aatactcctt 2160
tcagagctgt tgtgggtgta agtgacatga tgtggccaaa aatccaaact gtgcagttgc 2220
gttgtgacaa acatgcaatg tgctgtaaaa attcaataca gtttaaataa aatctctata 2280
ttagtaaaaa aaaaaaaaaa aaactcgag 2309

<210> 68
<211> 814
<212> DNA
<213> Homo sapiens

<220>
<221> SITE
<222> (421)
<223> n equals a,t,g, or c

<400> 68
tacgagtttt tttttttttt tttagccata attaccaaaa acattagtgc aggacaccat 60
tttaaaaaac tatttaaaat agtcttcaga gaaaaaatat taagtattac agtttaggag 120
tatattgact ttgggccaac ggattccaat attttacaaa aaggcaatat ccacgcaaca 180
tattccagat tcgggttgtg gagaagctgc agggcttgag gtgactctat cacaactgct 240
ttccgtacgg aggagccact gccaactgtg tggacgagaa tacttaagca cgtgcttcat 300
tgctccactg ccacaggtgg atatttcagg ggaattatta ttaatttcaa agttttttta 360
aaargytatg ataagtaaat aaaagtaatg gtaggaktca cggtcggaga gcttatcgcc 420
naagtctttc tatagccttc ccccggaagc cccagttcag gcatcggtca cccgaagtgt 480
caccctctga tctttccccc atcccatctg aggaagttaa agagatccct cacaggtacc 540
gtggctctcg gtgccctcgc acttccaaca gccggttcgg gcccaggaga ctcgctccga 600
cctccaccac aatggcggcc agtgtgggcc gcgcaaccag aagtgcggcc gcgcacctga 660
cccagcttcc gcctgcacct agagctcagc gcaccagccc ggctcagcca gacgaaggca 720

aacgaagaga tgcggatccc tggaggactg gccccaccgt gaacaaaaca ggaagcattc 780
caggaagact gcgggggtgg gctcgtgccg aatc 814

<210> 69
<211> 788
<212> DNA
<213> Homo sapiens

<220>
<221> SITE
<222> (370)
<223> n equals a,t,g, or c

<400> 69
gaattcggca cgaggcaatt ttcaatgaac cttgaatggt aggaagaatt gaagaagaaa 60
tcagagcatt tttgccttgc agaaggcagc tgctgtgatg gcaggaggct gaaatggaca 120
tggcctggca gaagagtatt atggggtggt tgtgttgtga gccatctggc ctgtacaatt 180
tggagaaaca atactttttt ttttcttctc tgcaagctgg gcttcctgtg attgtgtcct 240
caggctgcac aaaaatagcg tatggctttg ctgtgtattc accttcatct taaaatagct 300
agaacatttt ccctcttctt ttaaaaagtt tttaaaatga gggttagact cttgtaggaa 360
aaggtagaan tcttaataac agtactcatg ttgacaaacc tttctcgtca aaattcctat 420
gtaatcaaga ctcttattaa atatgaacaa atgtaatgta tggaaattaa tgtttaccct 480
caaggtaaaa gctgaaatgg atttataaag aattatttta aacagcaata atgtttgagg 540
ggtgggggaa gtgagaaaaa tgaaatttta aatcacatgt ttatgactat gaagctagac 600
tttaaaaata ggtcagttag ggtatgactc ttataataca aaagtttatt tggtatacaa 660
aggatttata gctaatgtat tttttaatta tattcactaa tacttgtaaa agatcattca 720
atttataaag tttccaaaat aaacctgttt aaagtgtcaa aaaaaaaaaa aaaaaaaaaa 780
aaactcga 788

<210> 70
<211> 791
<212> DNA
<213> Homo sapiens

<400> 70
gaattcggca cgagctcaag gctaaaatct tgatctctcc tgaatatgag gaggtgtgtt 60
aggcatgttt tggggattgg attaatagtg ttaaaaaatt tgtattttca caaaaatagc 120
atgtacccat cacccaaact cagcagcttt caagaagctt ttcttttttt ctttcttatt 180
ttaaaaaatc ctttaacctt atgtagttag tatatctttt ttaaaaagta gaaaatcatg 240
taaccttagg atttttagtt ttaatgtaga gtttcacaaa tttccatctt tagtaagaca 300
aaagggtcac atattggctg tctccttcaa ctatactttc ttcagtataa aatatgttta 360
ccatggttgt cattatcgag cacgtaactg catgttagac tctatgctaa gtgttttaca 420
taatcattta aagctcacta aggccctagg agtaattatt atcctcccat caaaaaggta 480
agtgaaatgt taacctgaag tttgactact ttaggtctct gagctagtaa gtacaatagc 540
caggtttcaa accaagatcc ttttaactgc agcacctgtg ccttatctgg tagcgtcatc 600
ttggttcata catttaaaaa agagttatct atgtgccggg tgccctggct catgcctgta 660
atcccagcac tttgggaggc cgaggagggc ggatcaccag gtcaggagtt tgagactgac 720
caataaggtg aaatcctgtc tctactaaaa aaaaaagggg gggcccgtac ccaatcgccc 780
aaaaagatcg t 791

<210> 71
<211> 804
<212> DNA
<213> Homo sapiens

<400> 71
gaattcggca cgagcggcac gagcttgaaa tggcgtcttc tgatgaacac tcatccatcc 60

ttcaaggtct actctctcat cacagcttgt gactcttcca ctttttgaac tggtgtttcc 120
cattcccagt tcacagagcc ctttctcatt gaactattta tctgagttcc ctctgccgga 180
acatgagcca tgcctagagt agccacctag tagtgagtga cagctctgtg ctggatgcac 240
ataaatggtc tcccttaact gccatgagsc ctaaagaagg tttgctacag ctattttaca 300
gatggggaaa actgacagag agatattaat gaattgccca catgcaaata tgtgctgagt 360
cttggatttg catctttatc gtgactccac ggagacccac cctctaagac cagagccagt 420
gtcctattca tcttttgtct ctgcagcgtt cagcatggca ctgtcttggc ttacaaaatc 480
tgctctatgc ttgctgactg ctgaatgaat gaatgaatga ataggtagtc acaaagaatg 540
tttagaatgt ttctcagaca ggctgagaaa aaacacaacg aaacattatt tccgtttgga 600
aagttttttt atttttgtgt tcagtactga agtaaaacaa aaatctgaat aacagctgca 660
ccgttaaaaa tgaaattacc aatatatgaa ctctaggcat catgcatata taattttttg 720
tagataactt ttcttctcat tttccttctc attctcttca tctttttctt tttgtttgag 780
caaaaaaaaa aaaaaaaaac tcga 804

<210> 72
<211> 783
<212> DNA
<213> Homo sapiens

<400> 72
gaattcggca cgagctaaaa cttacaatga catgttgttg cttgctctgt aagctccaag 60
gcattttttt tttcagtttt aattcaagtg ttctaaaaag tattttgggt acaaccagaa 120
ctctctctgc tccttggatt ggagtcagtg tgaaaggaac acagtgggct ctggggtcag 180
ctagacctgg atgtggatca cagctcacct cttcattggg aggcctcagg caagttattt 240
gccaacctca cctacaaaag catgatgcta agctcwtttc agtttagttg tggatatcag 300
agcatatgta tacaatgcct gccatagtga gtgcctggcc cttggcagac tgtcaaatgg 360
agctatggag cagcagcggg agtaatatta ttatctagac cttatctgtc cttttaaact 420
cagttcagat tccttctcct ttttaaatta ctgcaacctg attttacctg cccctgcctc 480
caagttgctg tatcagttag cctctgaaca attcatttag caattttaat tatatattgc 540
ttcttgacac tgctttgtga tcttaaaaac tctgcttcaa atacgtactt ggttgctttt 600
cctgagtgct gttaattcct gctctaacgg actaaagtaa tttgaaggca ggactaggtt 660
ttatgcatgg cacacagtct ggtgccttac atgtaactac tcacaaactt ttttgatcca 720
aaatttagaa acttcacacg cattcataag aaatcaataa aaaaaaaaaa aaaaaactcg 780
tag 783

<210> 73
<211> 1523
<212> DNA
<213> Homo sapiens

<220>
<221> SITE
<222> (1)
<223> n equals a,t,g, or c

<220>
<221> SITE
<222> (8)
<223> n equals a,t,g, or c

<220>
<221> SITE
<222> (15)
<223> n equals a,t,g, or c

<400> 73
   ngggggngcc ccccnttttt tttttttttt tttttttttt ttcagttcta ctattattta 60
   tttttttaaa tatttttgaa aaaatataat ttttttacaa tattttcaac ttaaacacta 120
   ttcacactga acacgtatgg cagcttaacc tacccaaata tgaagtttaa gaagccaaaa 180
   ctgttctagc tttgttaaaa gttgtgctgc agactctcgt gatggttaac aaagcaagga 240
   aaagcaccac tcaaatcata atgttacagt atctttgttc agctggatta tgggttggta 300
   ttggtcatat gttagactcc atacaggcat agctatgatg cagtgaatcc cttagaagtt 360
   acaattctca aattacatac ttcctcagat gtaacattag aactcaatat ttctaacaat 420
   aacataccag aaaaggctgg actggcactc atctgctgac taacttgtag cctcagtaat 480
   atgacatact tgcctttaac aaattatctc aaattaacta acagaccttc agaaaatgga 540
   gattcttttt gatggggaca taatcaaatt taagtctgag aaatatgctt aacagttgga 600
   actcaaatta aatgtactga ttttaaagtt tagacattaa caagtgatag attagcctca 660
   aaaaaagaca atttggtaag gtttaggtct tttaatttgg tgcttgttca caacttgact 720
   ggtgcttctt tccttgctgt cttcacatca agccatgggg ccaattctat tttcagtaaa 780
   tgtttgacag ctttttactt agtaacagtc tcagcacttt tattaagcat gcaagactaa 840
   caaaaacttt ggcaatgcat aagtgtaaca cagtgacaag agagctttta caattaagtc 900
   ttctaatact gccttcacag tgtggaaatt gtgctacatc caccaaaaga gggccccgtc 960
   tactcaaata tttccgtact tcaccccagg aacaaactcc tttgcatttg gattcagatt 1020
   gctcttgacc acaagatctt ccagagaaga gccatcactg ataacaaggt cattaaactg 1080
   gtcttggatt tggtccatag tttgtgggag atctcgagct ggaataaacc attcatgctc 1140
   ttcttcctct tccagcattt cttggaaaca gcgttcaata aattcttctt cccataactc 1200
   ctcttctatt tgtctgttga attcttcttc attttccatc cacatgtact ctgcaaatgg 1260
   attgtcatct tcatgagaat gaccgttaat aatcacatct tcattgatga tgcttgggct 1320
   agtactgctg cgacttggat ctttcatggc tgatgttggt tgtcgttttt aacccaatgc 1380
   acagcagcgg ggacggcagc caacgaatcc tgtcggcctc cgcggatctc cacaggcagc 1440
   gccgctcccc cgctcgacgt gcgcttcgcc cgccgcctcc cttctcccgg acgcgtgggc 1500
   ggacgcgtgg gcggacgcgt ggg 1523

<210> 74
<211> 758
<212> DNA
<213> Homo sapiens

<400> 74
gaattcggca cgagacasgg tttcaccctg ttggccagga tggtctcaat ctcttgacct 60
cgtgatctgc ctgcctcggc ctcccaaagt gctaggatta caggcatgag ccactgtgcc 120
cggcctttgt tttttgagac cttttttatt ttgttgtcac ccaggctgaa gtgcagtggc 180
acaaacacag ttcactacag ccttgacctc ctgggctcaa gcaattctgc ctcagtccca 240
caagtaggtg ggcttacaaa tgcacagcat gacacctggc ttatttttgt attttgtgtg 300
tgtgtgtgtg agccactgcg caggccttgg gcagctttct tgatctctgt tacctcatct 360
ataaaatgat gataataata gcttctccct tattggggaa ttgtaatgat taaatgagat 420
aacatgtaaa atgctcagta caggccaggc atggtggctc acgcttgcaa tcccagcact 480
ttgggaggct gaggctgcta gatctcttga ggccagcagt taagaccagc ctggccaata 540
tggtgaaacc ctgtgtctac caaaaaatac agaaagtcag ccaggcatgg tggtgcatgc 600
ctgtggtccc agctactcag aggctgaggt gggagaatca cttgagcccg ggagacagaa 660
gttgaagtga gccaagatgg cgccactgca ctctagcatg ggctacagag tgagagcctc 720
tctcaaaaaa aaaaaaaaaa aaaaaaaaaa aactcgta 758

<210> 75
<211> 1096
<212> DNA
<213> Homo sapiens

<400> 75
ccccacggct cccatggcct cttcctgcgc taccgtgtgg aggccctaac cctgcgtggc 60
atcaatagct tccgccagta caagtatgac ctggtggcag tgggcaaggc tttggagggc 120
atgttccgca agctcaacca cctcctggag cgcctgcacc agtccttctt cctctacttg 180
ctccccggcc tctcccgctt cgtctccatc ggcctctaca tgcccgctgt cggcttcttg 240
ctcctggtcc ttggtctcaa ggctctggaa ctgtggatgc agctgcatga ggctggaatg 300
ggccttgagg agcccggggg tgcccctggc cccagtgtac cccttccccc atcacagggt 360
gtggggctgg cctcgctcgt ggcacctctg ctgatctcac aggccatggg actggccctc 420

tatgtcctgc cagtgctggg ccaacacgtt gccacccagc acttcccagt ggcagaggct 480
gaggctgtgg tgctgacact gctggcgatt tatgcagctg gcctggccct gccycacaat 540
acccaccggg tggtaagcac acaggcccca gacaggggct ggatggcact gaagctggta 600
gccctgatct acctagcact gcagctgggc tgcatcgccc tcaccaactt ctcactgggc 660
ttcctgctgg ccaccaccat ggtgcccact gctgcgcttg ccaagcctca tgggccccgg 720
accctctatg ctgccctgct ggtgctgacc agcccggcag ccacgctcct tggcagcctg 780
ttcctgtggc gggagctgca ggaggcgcca ctgtcactgg ccgagggctg gcagctcttc 840
ctggcagcgc tagcccaggg tgtgctggag caccacacta cggcgccctg ctcttcccac 900
tgctgtccct gggcctctac ccctgctggc tgcttttctg gaatgtgctc ttctggaagt 960
gagatctgcc tgtccgggct gggacagaga ctccccaagg accccattct gcctccttct 1020
ggggaaataa atgagtgtct gtttcagcar mwaaaaaaaa aaaaaaaaaa aaaaaaaaaa 1080
aaaaaaaagg gcggcc 1096

<210> 76
<211> 1230
<212> DNA
<213> Homo sapiens

<400> 76
   cacgagtgcc gctaaccttc ttcatccttt ggtggcaaag tagaaagatt ccagaattaa 60
   ctcgaccttt ctaaagacct gggctcagag gcagctggca ctgactgagc acccactatg 120
   tgccaggcac tgtgctgaat gcattagatc atcaattatg aatttgacac caaggacctg 180
   gtgtgcctgg gcctgagcag catcgttggc gtctggtacc tgctgaggaa gcactggatt 240
   gccaacaacc tttttggcct ggccttctcc cttaatggag tagagctcct gcacctcaac 300
   aatgtcagca ctggctgcat cctgctgggc ggactcttca tctacgatgt cttctgggta 360
   tttggcacca atgtgatggt gacagtggcc aagtccttcg aggcaccaat aaaattggtg 420
   tttccccagg atctgctgga gaaaggcctc gaagcaaaca actttgccat gctgggactt 480
   ggagatgtcg tcattccagg gatcttcatt gccttgctgc tgcgctttga catcagcttg 540
   aagaagaata cccacaccta cttctacacc agctttgcag cctacatctt cggcctgggc 600
   cttaccatct tcatcatgca catcttcaag catgctcagc ctgccctcct atacctggtc 660
   cccgcctgca tcggttttcc tgtcctggtg gcgctggcca agggagaagt gacagagatg 720
   ttcagttatg aggagtcaaa tcctaaggat ccagcggcag tgacagaatc caaagaggga 780
   acagaggcat cagcatcgaa ggggctggag aagaaagaga aatgatgcag ctggtgcccg 840
   agcctctcag ggccagacca gacagatggg ggctgggccc acacaggcgt gcaccggtag 900
   agggcacagg aggccaaggg cagctccagg acagggcagg gggcagcagg atacctccag 960
   ccaggcctct gtggcctctg tttccttctc cctttcttgg ccctcctctg ctcctcccca 1020
   caccctgcag gcaaaagaaa cccccagctt cccccctccc cgggagccag gtgggaaaag 1080
   tgggtgtgat ttttagattt tgtattgtgg actgattttg cctcacatta aaaactcatc 1140
   ccatggccag ggcgggccac tgtgctcctg gaaaaaaaaa aaaaaaaaaa aaaaaaaaaa 1200
   aaaaaaaaaa aaaaaaaaaa ggggaggggc 1230

<210> 77
<211> 911
<212> DNA
<213> Homo sapiens

<400> 77
tcgacccacg cgtccgtctt cctaaaaggg atgccctcca aagaaatttt aaaagaatct 60
tatcaagggg ccctggagaa gaaagggatg tgagggtcaa gtcacaactt tgaggggaaa 120
tagaaagagg gctcctttct gagaaagaag aatttcaaag agtccaagag aaccaaaaat 180
tcaggaccca ggagggtaag cattcctgtt tttgcaagct tcacagacca tttgagtgag 240
tgggtttttc aggtgacatt taaatgaaca aataatatcc atgtctcagg gtcagaaatg 300
gtactttgca actgattctg tccctcttga gaggcttctg caagactgag agggtgggat 360
gacttaatga acattaaaaa caatgttatt aggckggata tggtggcaca tgcctgtaat 420
tctagcactt tgggargctg aggtgggcag gcccargart tcaagaccag tctgggcaac 480
atggtgagac cctgtatcta ataaaaatac aaaaatttag ccaggcatgg tggcacacac 540
ctggagtccc agctactcag gagactgagg tgggaagatc acctgagctc aggaagtcga 600
ggctgcagtg agccaagatt gcactactgc actctagcct acatggatag gagtgagacc 660

tgtttgaaaa acaaaaaaca atcaaaaaca aaaaaaaaca acccacacaa tgttattttt 720
aaaatactga ggggagagaa gttggggaaa aaaagggaaa acctaaaact ctccataatc 780
ctaccatcag aaaattacac taatgtgata agtgactttc tcccctctga atctccaatt 840
ccattacttg tagtaaatat gaatcttatt ccacaaactc agacatgcaa aaaaaaaaaa 900
aaagggcggc c 911

<210> 78
<211> 488
<212> DNA
<213> Homo sapiens

<220>
<221> SITE
<222> (324)
<223> n equals a,t,g, or c

<220>
<221> SITE
<222> (438)
<223> n equals a,t,g, or c

<220>
<221> SITE
<222> (484)
<223> n equals a,t,g, or c

<400> 78
accgcagggg ctcccggacc ctgactctgc agccgaaccg gcacggtttc gtggggaccc 60
aggcttgcaa agtgacggtc attttctctt tctttctccc tcttgagtcc ttctgagatg 120
atggctctgg gcgcacggga gctacccggg tctttgtcgc gatggtagcg gcggctctcg 180
gcggccaccc tctgctggga gtgagcgcca ccttgaactc ggttctcaat tccaacgcta 240
tcaagaacct gcccccaccg ctgggcggcg ctgcggggca cccaaggctc tgcagtcagc 300
gccgcgccgg gaatcctgta cccngggcgg gaataagtac cagaccattg acaactacca 360
gccgtacccs ttgcgcaaaa gaacraaaga aatttgccgc actgaaataa atttacttgc 420
gcctaattcc ccaccccncc cggaaagggg aaacccccgg ggcgtttttc caaattcttt 480
tttnttcc 488

<210> 79
<211> 753
<212> DNA
<213> Homo sapiens

<220>
<221> SITE
<222> (745)
<223> n equals a,t,g, or c

<220>
<221> SITE
<222> (752)
<223> n equals a,t,g, or c

<400> 79
gaattcggca cgagcggcgg gggtccatcc accccggtga gcaggcccaa ggcagcgggg 60
gcccacaccc ctcacacgca aaactggctt cttctggtca ctggtgtctg aaaccaaatc 120
cagagcagcc tgtggcctgt aaagcatata tttctaatga ctgcagactg gtgggatcat 180
aggagccttc tgaatgacca ggactgcttt ctttggagct gatgaaaatg tactctttta 240
gcgtgttaga aatcacttgt tttattttgt ttctttggcc aagctgggtc tagtgtttct 300

tttgctggga atagactttc aaaagttgta cttctatcaa gaaacaaaac tgcccttgca 360
gaaatttcag gtcttttgtt aagcctgtat tggtcttaag gtgcagtatt ttttaaatta 420
ttatttatag aaagaatcta taaattcttg gggaagtgtg ttataagctt taataattac 480
attgagctgc acctcagtgg tgtgtcatta acatgcagtg gggttaatat ctgaggcctc 540
agatgacttt gtgccttttg gaataaaggg taaaataaac tctcccagag taagagctgt 600
atcgtgaatt gtcatactaa ttattgaggg ggacttatgt gcttttattg aatggagtgc 660
tttacaattt ttatttttaa atggggttgg gatccttgga atatttcaat aaaattgata 720
aaatataaaa aaaaaaaaaa agggnggccg cnc 753

<210> 80
<211> 2138
<212> DNA
<213> Homo sapiens

<400> 80
tggatgatga tggactcccc tttcccacgg atgtgatcca gcataggtta cggcaaatcg 60
aagcagggta caaacaagag gtggagcagc tacgtcgaca ggtgcgtgac tcagatgagr 120
ctggacatcc gtcactgctg tgcccctcca gcagagcccc catggactat gaggatgatt 180
ttacatgttt gaaggagtca gatggcagtg atactgagga ttttggctct gatcacagtg 240
aagactgcct ttcagaagca agctgggaac ctgttgataa gaaagagact gaggtgactc 300
gctgggttcc agaccatatg gcatcacact gctataactg tgactgtgaa ttctggttgg 360
ccaaacgaag acaccattgc agaaattgtg ggaatgtatt ttgtgctgga tgctgccacc 420
tgaagctgcc cattcctgat cagcaactct atgacccagt tctcgtctgt aactcatgtt 480
acgraacaca ttcaagtctc tcgtgccagg gaactcatga gccaacagct gaagaaaccc 540
attgctacag cttccagttg aatgccgggg agaaacctgt ccaattttag caggtttgaa 600
gggaggatct tcttcagttg tagtttggaa ggttccttgg tgtggctcat gaaatcacag 660
agctcagaga taccatcttg agaaatcctc cttggtatca tgaaactgga gcagaggaat 720
tgcaatttag caggaggtcc tctactggtg ataccctcac cttggggtaa tggtcctaac 780
ccagacccag ggtctggaag cttaatgttg agttggtgac tccagcctct ttctcctgga 840
ggtcacaaga tgatgattgc gtagatgttg cctggtgcaa agtgccccaa acagcaatag 900
aaaggcatat gtataaccaa actccaagtg ataaccagac ccatctctcc tccaccttga 960
caaaagcaga ttatagtata caaggtagga attcctgtcc tatttgagat gaactatatc 1020
ctgtacctct gtgctctgtg tctgcatgaa ggctcagcct ttagaggcac tccttctagt 1080
tgcattagta ctgtctttct gtggagtttg gtttgaagac tggctcagca agtggaggtt 1140
tcaatgtatt tttcagttgg ctcatcagcc agcattggtg aatattcagt ttaggggaac 1200
agttctaggg agtgagacat ttttgggagc agaggaaaac tctgctgatg ttcggtcctg 1260
gcaaacattg agttattttg agctgtgaag gcagtcgtct ctgttacaca gtggcagctc 1320
ttgagttatg cactgtgaag aatgagaagg gaaaagcaaa aattatcctt gtgaaatatc 1380
tgctgattgt gccctactct ttgcacctga cttttcctag ttgtcctggt gctaacacag 1440
gagctacamc ttgatcctct cctggcatga aaataaaaca aaggttttcg ttgttgttgt 1500
tccattgccc atttccccca tgttgtcttt cccttggctg atgcctcctc tgggtcacat 1560
tgcttcttat cctgaacact tgacaccttg agggtagaat ttagcgtttg gtttttacct 1620
cctagcatat gctgtttggt atgtgagggt ttcagtacaa atgctgctgt ctatttctgt 1680
gcacttaaca atggaaccca aacagaagag aataaagcct tgataccaaa attgggaaag 1740
aacatgtgtc catttggacc aaacgttgtt ggtttttaaa aaatttattt ttgttttttt 1800
gtttttgttt ttgttttttt tcatcttaat atgtaccagt ggcacttaac caaaagatac 1860
agtgatatag ccatgtatct gtctacttag cgtggctgtt ttgagggact gtcccatcag 1920
tgaacaaact gcatggcctt ggagagagac tctgggctct tggctcagat gtgttcatca 1980
aatactcctt tcagagctgt tgtgggtgta agtgacatga tgtggccaaa aatccaaact 2040
gtgcagttgc gttgtgacaa acatgcaatg tgctgtaaaa attcaataca gtttaaataa 2100
aatctctata ttagtaaaaa aaaaaaaaaa aaactcga 2138

<210> 81
<211> 1327
<212> DNA
<213> Homo sapiens

<220>

<221> SITE
<222> (5)
<223> n equals a,t,g, or c

<220>
<221> SITE
<222> (7)
<223> n equals a,t,g, or c

<220>
<221> SITE
<222> (9)
<223> n equals a,t,g, or c

<220>
<221> SITE
<222> (10)
<223> n equals a,t,g, or c

<220>
<221> SITE
<222> (1205)
<223> n equals a,t,g, or c

<400> 81
aaccnangnn taccggtccg gaattcccgg gtcggaccca cgcgtccgcg gcgggcgacg 60
cacgtcgagc gggggagcgg cgctgcctgt ggagatccgc ggaggccgac aggattcgtt 120
ggctgccgtc cccgctgctg tgcattgggt taaaaacgac aaccaacatc agccatgaaa 180
gatccaagtc gcagcagtac tagcccaagc atcatcaatg aagatgtgat tattaacggt 240
cattctcatg aagatgacaa tccatttgca gagtacatgt ggatggaaaa tgaagaagaa 300
ttcaacagac aaatagaaga ggagttatgg gaagaagaat ttattgaacg ctgtttccaa 360
gaaatgctgg aagaggaaga agagcatgaa tggtttattc cagctcgaga tctcccacaa 420
actatggacc aaatccaaga ccagtttaat gaccttgtta tcagtgatgg ctcttctctg 480
gaagatcttg tggtcaagag caatctgaat ccaaatgcaa aggagtttgt tcctggggtg 540
aagtacggaa atatttgagt agacggggcc ctcttttggt ggatgtagca caatttccac 600
actgtgaagg cagtattaga agacttaatt gtaaaagctc tcttgtcact gtgttacact 660
tatgcattgc caaagttttt gttagtcttg catgcttaat aaaagtgctg agactgttac 720
taagtaaaaa gctgtcaaac atttactgaa aatagaattg gccccatggc ttgatgtgaa 780
gacagcaagg aaagaagcac cagtcaagtt gtgaacaagc accaaattaa aagacctaaa 840
ccttaccaaa ttgtcttttt ttgaggctaa tctatcactt gttaatgtct aaactttaaa 900
atcagtacat ttaatttgag ttccaactgt taagcatatt tctcagactt aaatttgatt 960
atgtccccat caaaaagaat ctccattttc tgaaggtctg ttagttaatt tgagataatt 1020
tgttaaaggc aagtatgtca tattactgag gctacaagtt agtcagcaga tgagtgccag 1080
tccagccttt tctggtatgt tattgttagr aatattgagt tctaatgtta catctgaggr 1140
agtatgtaat tgagrattgt aacttctaag gggttcactg catcatrgct atgcctgtat 1200
ggrgntctwa ccatatgacc mataccamcc cwtaatccca gctgraccaa rgrtacckgt 1260
aaccattwwg gatttgaggg gkggcctttc ccyggcyttg kttwacccmt ccacggagaa 1320
tctggca 1327

<210> 82
<211> 758
<212> DNA
<213> Homo sapiens

<400> 82
gaattcggca cgagacacgg tttcaccctg ttggccagga tggtctcaat ctcttgacct 60
cgtgatctgc ctgcctcggc ctcccaaagt gctaggatta caggcatgag ccactgtgcc 120
cggcctttgt tttttgagac cttttttatt ttgttgtcac ccaggctgaa gtgcagtggc 180
acaaacacag ttcactacag ccttgacctc ctgggctcaa gcaattctgc ctcagtccca 240

caagtaggtg ggcttacaaa tgcacagcat gacacctggc ttatttttgt attttgtgtg 300
tgtgtgtgtg agccactgcg caggccttgg gcagctttct tgatctctgt tacctcatct 360
ataaaatgat gataataata gcttctccct tattggggaa ttgtaatgat taaatgagat 420
aacatgtaaa atgctcagta caggccaggc atggtggctc acgcttgcaa tcccagcact 480
ttgggaggct gaggctgcta gatctcttga ggccagcagt taagaccagc ctggccaata 540
tggtgaaacc ctgtgtctac caaaaaatac agaaagtcag ccaggcatgg tggtgcatgc 600
ctgtggtccc agctactcag aggctgaggt gggagaatca cttgagcccg ggagacagaa 660
gttgaagtga gccaagatgg cgccactgca ctctagcatg ggctacagag tgagagcctc 720
tctcaaaaaa aaaaaaaaaa aaaaaaaaaa aactcgta 758

<210> 83
<211> 48
<212> PRT
<213> Homo sapiens

<220>
<221> SITE
<222> (48)
<223> Xaa equals stop translation

<400> 83
Met Gly Ser Cys Ala Ala Phe Leu Leu Ala Ala Leu Ser Leu Leu Gly
1               5                   10                  15

Val Leu Gly Gly Tyr Pro Gly Arg Arg Ala Phe Ile Leu Pro Asn Arg
            20                  25                  30

Arg Ser Leu Arg Gln Trp Leu Glu Val Ser Leu Gly Pro Val Ser Xaa
        35                  40                  45

<210> 84
<211> 38
<212> PRT
<213> Homo sapiens

<220>
<221> SITE
<222> (38)
<223> Xaa equals stop translation

<400> 84
Met Asn Glu Ala Pro Pro Leu Ser Ser Ser Ser Ile Cys Phe Ile Leu
1               5                   10                  15

Phe Tyr Phe Phe Pro Leu Leu Pro Pro Leu Ser Ser Thr Cys Phe Ser
            20                  25                  30

Lys Gly Asn Arg His Xaa
        35

<210> 85
<211> 53
<212> PRT
<213> Homo sapiens

<220>
<221> SITE
<222> (53)
<223> Xaa equals stop translation

<400> 85
Met Cys Gln Asn Arg Glu Ser Val Leu Val Leu Leu Ile Glu Ser Asn
1               5                   10                  15

Met Phe Ser Phe Tyr Leu Leu Phe Ser Phe Tyr Ile Val Phe Ser Phe
            20                  25                  30

Phe Ile Val Leu Arg Pro Leu Pro Arg Asn Glu Ser Ile Lys Lys Ile
        35                  40                  45

Gly Val Ile Phe Xaa
    50



<210> 86
<211> 26
<212> PRT
<213> Homo sapiens

<220>
<221> SITE
<222> (26)
<223> Xaa equals stop translation

<400> 86
Met Thr Val Leu Ala Lys Arg Leu Val Leu Phe Leu Gly His Ile Phe
1               5                   10                  15

Leu Leu Leu Cys Val Arg Ile Leu Asp Xaa
            20                  25



<210> 87
<211> 78
<212> PRT
<213> Homo sapiens

<220>
<221> SITE
<222> (43)
<223> Xaa equals any one of the naturally occurring L-amino acids

<220>
<221> SITE
<222> (78)
<223> Xaa equals stop translation

<400> 87
Met Ala Ala Arg Ser Ala Leu Ala Leu Leu Leu Leu Leu Pro Val Leu
1               5                   10                  15

Leu Leu Pro Val Gln Ser Arg Ser Glu Pro Glu Thr Thr Ala Pro Thr
            20                  25                  30

Pro Thr Pro Ile Pro Gly Gly Asn Ser Ser Xaa Ser Arg Pro Leu Pro
        35                  40                  45

Ser Ile Glu Leu His Ala Cys Gly Pro Tyr Pro Lys Pro Gly Leu Leu
    50                  55                  60

Ile Leu Leu Ala Pro Leu Ala Leu Trp Pro Ile Leu Leu Xaa
65                  70                  75

<210> 88
<211> 38
<212> PRT
<213> Homo sapiens

<220>
<221> SITE
<222> (38)
<223> Xaa equals stop translation

<400> 88
Met Cys Tyr Ile Pro Gly Ser Thr Gly Gly Gln Cys Trp Pro Trp Cys
1               5                   10                  15

Trp Cys Trp Leu Cys Arg Glu Ala Leu Glu Trp Leu Cys Gly Ala Val
            20                  25                  30

Ser Ala Gly Pro Ala Xaa
        35

<210> 89
<211> 44
<212> PRT
<213> Homo sapiens

<220>
<221> SITE
<222> (40)
<223> Xaa equals any one of the naturally occurring L-amino acids

<220>
<221> SITE
<222> (44)
<223> Xaa equals stop translation

<400> 89
Met Leu Leu Arg Ile Ile His Leu Val Ile Phe Phe Ile Asn Phe Ser
1               5                   10                  15

Thr Ser Val Val Ile Val His Tyr Asn Val Leu Asn Tyr Arg Cys Leu
            20                  25                  30

Leu Lys Cys Arg Cys Arg Val Xaa Lys Tyr Ser Xaa
        35                  40

<210> 90
<211> 60
<212> PRT

<213> Homo sapiens

<220>
<221> SITE
<222> (60)
<223> Xaa equals stop translation

<400> 90
Met Gln Asn Cys Leu Gly Ser Leu Ile Pro Gly Val Leu Phe Ser Leu
1               5                   10                  15

Leu Leu Leu Pro Ser Met Phe Asn Ile Ile Leu Thr Gln Ser Lys Tyr
            20                  25                  30

Gly Glu Asn Ser Tyr Pro Ala Cys Phe Tyr Ser Ser Ser Asn Phe Pro
        35                  40                  45

Val Ser Ala Ile Thr Phe Leu Val Gly Val Val Xaa
    50                  55                  60

<210> 91
<211> 55
<212> PRT
<213> Homo sapiens

<220>
<221> SITE
<222> (55)
<223> Xaa equals stop translation

<400> 91
Met Val Val Ile Val Leu Thr Ser Asn Val Cys Ile Cys Gly Tyr Val
1               5                   10                  15

Val His Ser Ala Leu Ile Pro Arg Arg Gln Gly Leu Phe Leu Phe Leu
            20                  25                  30

Phe Leu Val Met Phe Tyr Phe Ser Ile Ala Phe Asn Arg Ile Thr Lys
        35                  40                  45

Gly Thr Leu Ser Ser Gln Xaa
    50                  55

<210> 92
<211> 51
<212> PRT
<213> Homo sapiens

<220>
<221> SITE
<222> (51)
<223> Xaa equals stop translation

<400> 92
Met Val Ala Gln Leu Val Gly Cys Val Val Ser Cys Leu Phe Val Leu
1               5                   10                  15

Leu Arg Phe Leu Ile Ser Thr Phe Gly Ile Met Ser Phe Asn Gly Phe

            20                  25                  30

Val Ile Phe Val Thr Val Leu Ala Ala Tyr Asn Phe Ser Ala Gly Ala
        35                  40                  45

Phe Thr Xaa
    50

<210> 93
<211> 156
<212> PRT
<213> Homo sapiens

<220>
<221> SITE
<222> (156)
<223> Xaa equals stop translation

<400> 93
Met Trp Pro Gln Glu Ala Trp Val Cys Ile Leu Val Leu Leu Gly Thr
1               5                   10                  15

Arg Val Gly Leu Cys Val Gly Asp Ser Leu Ala Pro Gln Ala Ser Leu
            20                  25                  30

Ser Tyr Cys Tyr Ile Leu Lys Val Pro Leu Arg Pro Lys Pro Leu Trp
        35                  40                  45

Gln Leu Ser Asn Glu Ser Ile Cys Ser Glu Tyr Arg Val Glu Gly Gly
    50                  55                  60

Gln Gly His Gln Glu Leu Arg Met Phe Leu Arg Leu Met Arg Pro Arg
65                  70                  75                  80

Tyr Trp Val His Gly Gly Pro Arg Ser Leu Cys Asp Ser Cys Ser Leu
                85                  90                  95

Leu Pro Pro Cys Leu Asp Pro Ala Ser Ala Gln Lys Ala Asn Ser Leu
            100                 105                 110

Asp Ser Lys Gly Leu Pro Arg Pro Ile Ser Met Ser Cys Ser Cys Gln
        115                 120                 125

Leu Pro Val Pro Ser Leu Asp Leu Ser Ser Cys Leu Ala Pro Ser Leu
    130                 135                 140

Pro Thr Pro His Ile Phe Thr Asn Lys Arg Lys Xaa
145                 150                 155

<210> 94
<211> 61
<212> PRT
<213> Homo sapiens

<220>
<221> SITE
<222> (61)
<223> Xaa equals stop translation

<400> 94
Met Ser His His Ala Arg Pro Tyr Lys Ala Phe Arg Ile Val Ser Cys
1               5                   10                  15

Tyr Phe Tyr Leu Phe Ile Ile Val Val Val Ile Ile Leu Leu Leu Tyr
            20                  25                  30

Pro Ile Ser Gln Gly Trp His Val Ala Asn Ile Val Phe Leu Lys Asn
        35                  40                  45

Ile Ser Asp His Ile Leu Val Leu Leu Lys Thr Phe Xaa
    50                  55                  60

<210> 95
<211> 71
<212> PRT
<213> Homo sapiens

<220>
<221> SITE
<222> (71)
<223> Xaa equals stop translation

<400> 95
Met Trp Phe Glu Ile Leu Pro Gly Leu Ser Val Met Gly Val Cys Leu
1               5                   10                  15

Leu Ile Pro Gly Leu Ala Thr Ala Tyr Ile His Arg Phe Thr Asn Gly
            20                  25                  30

Gly Lys Glu Lys Arg Val Ala His Phe Gly Tyr His Trp Ser Leu Met
        35                  40                  45

Glu Arg Asp Arg Arg Ile Ser Gly Val Asp Arg Tyr Tyr Val Ser Lys
    50                  55                  60

Gly Leu Glu Asn Ile Asp Xaa
65                  70

<210> 96
<211> 37
<212> PRT
<213> Homo sapiens

<220>
<221> SITE
<222> (37)
<223> Xaa equals stop translation

<400> 96
Met Val Phe Leu Leu Leu Leu Leu Phe Gly Phe Phe Phe Asp Gly Ser
1               5                   10                  15

Leu Arg Ser Pro Leu Leu Leu Ile Ile His Leu Gly Pro Ala Pro Thr
            20                  25                  30

Phe Leu Gln Ile Xaa

<210> 97
<211> 60
<212> PRT
<213> Homo sapiens

<220>
<221> SITE
<222> (60)
<223> Xaa equals stop translation

<400> 97
Met Leu Cys Gln Thr Ile Pro Leu Cys Asn Arg Leu His Ile Val Phe
1               5                   10                  15

Met Ile Leu Ile Lys Leu Tyr Val Glu Thr Glu Cys Glu Val Lys Ser
            20                  25                  30

Glu His Lys Lys Ile Met His Asp Glu Ile Ala Tyr His Phe Ile Gly
        35                  40                  45

Tyr Leu Leu Cys Ile Tyr Thr Leu Arg Pro Leu Xaa
    50                  55                  60


<210> 98
<211> 44
<212> PRT
<213> Homo sapiens

<220>
<221> SITE
<222> (44)
<223> Xaa equals stop translation

<400> 98
Met Ser Val Ser Ser Asn Leu Trp Gln Thr Leu Ile Leu Leu Leu Ser
1               5                   10                  15

Leu Trp Phe Cys Leu Phe Pro Glu Cys His Ile Val Gly Ile Ile Gln
            20                  25                  30

Leu Cys Arg Leu Phe Arg Leu Pro Ser Phe Thr Xaa
        35                  40


<210> 99
<211> 32
<212> PRT
<213> Homo sapiens

<220>
<221> SITE
<222> (32)
<223> Xaa equals stop translation

<400> 99
Met Cys Cys Arg Ala Gly Gly Ser Gln Ser Pro Gln Val Met Val Val

1                   5                   10                  15

Leu Ile Ile Ile Leu Gly Pro Trp Gly Gly Val Arg Ile Asp Ala Xaa
            20                  25                  30

<210> 100
<211> 180
<212> PRT
<213> Homo sapiens

<400> 100
Met Tyr Ser Cys Leu Leu Leu Pro Asp Leu Leu Tyr Leu Thr Leu Ser
1               5                   10                  15

Pro Leu Val Val Ala Met Leu Leu Thr Pro His Phe Asn Val Ala Asn
            20                  25                  30

Pro Gln Asn Leu Leu Ala Gly Leu Trp Leu Glu Asn Glu His Ser Phe
        35                  40                  45

Thr Leu Met Ala Pro Glu Arg Ala Arg Thr His His Cys Gln Pro Glu
    50                  55                  60

Glu Arg Lys Val Leu Phe Cys Leu Phe Pro Ile Val Pro Asn Ser Gln
65                  70                  75                  80

Ala Gln Val Gln Pro Pro Gln Met Pro Pro Phe Cys Cys Ala Ala Ala
                85                  90                  95

Lys Glu Lys Thr Gln Glu Glu Gln Leu Gln Glu Pro Leu Gly Ser Gln
            100                 105                 110

Cys Pro Asp Thr Cys Pro Asn Ser Leu Cys Pro Ser His Thr Gln Leu
        115                 120                 125

Thr Lys Ala Asn Thr Leu Ser Leu Phe Phe Phe Phe Ser Phe Phe Leu
    130                 135                 140

Ser Arg Val Ser Leu Leu Ser Pro Arg Leu Glu Cys Asn Gly Arg Ile
145                 150                 155                 160

Leu Ala His Cys Asn Leu His Leu Pro Gly Ser Ser Asn Ser Pro Val
                165                 170                 175

Ser Ala Ser Arg
            180

<210> 101
<211> 212
<212> PRT
<213> Homo sapiens

<220>
<221> SITE
<222> (45)

<223> Xaa equals any one of the naturally occurring L-amino acids

<220>
<221> SITE
<222> (195)
<223> Xaa equals any one of the naturally occurring L-amino acids

<220>
<221> SITE
<222> (212)
<223> Xaa equals stop translation

<400> 101
Met Arg Leu Phe Leu Trp Asn Ala Val Leu Thr Leu Phe Val Thr Ser
1               5                   10                  15

Leu Ile Gly Ala Leu Ile Pro Glu Pro Glu Val Lys Ile Glu Val Leu
            20                  25                  30

Gln Lys Pro Phe Ile Cys His Arg Lys Thr Lys Gly Xaa Asp Leu Met
        35                  40                  45

Leu Val His Tyr Glu Gly Tyr Leu Glu Lys Asp Gly Ser Leu Phe His
    50                  55                  60

Ser Thr His Lys His Asn Asn Gly Gln Pro Ile Trp Phe Thr Leu Gly
65                  70                  75                  80

Ile Leu Glu Ala Leu Lys Gly Trp Asp Gln Gly Leu Lys Gly Met Cys
                85                  90                  95

Val Gly Glu Lys Arg Lys Leu Ile Ile Pro Pro Ala Leu Gly Tyr Gly
            100                 105                 110

Lys Glu Gly Lys Gly Lys Ile Pro Pro Glu Ser Thr Leu Ile Phe Asn
        115                 120                 125

Ile Asp Leu Leu Glu Ile Arg Asn Gly Pro Arg Ser His Glu Ser Phe
    130                 135                 140

Gln Glu Met Asp Leu Asn Asp Asp Trp Lys Leu Ser Lys Asp Glu Val
145                 150                 155                 160

Lys Ala Tyr Leu Lys Lys Glu Phe Glu Lys His Gly Ala Val Val Asn
                165                 170                 175

Glu Ser His His Asp Ala Leu Val Glu Asp Ile Phe Asp Lys Glu Asp
            180                 185                 190

Glu Asp Xaa Tyr Gly Phe Ile Ser Ala Arg Glu Phe Thr Tyr Lys His
        195                 200                 205

Asp Glu Leu Xaa
    210


<210> 102
<211> 621
<212> PRT
<213> Homo sapiens

<220>
<221> SITE
<222> (137)
<223> Xaa equals any one of the naturally occurring L-amino acids

<400> 102
Met Gly Leu Leu Ser Asp Pro Val Arg Arg Arg Ala Leu Ala Arg Leu
1               5                   10                  15

Val Leu Arg Leu Asn Ala Pro Leu Cys Val Leu Ser Tyr Val Ala Gly
            20                  25                  30

Ile Ala Trp Phe Leu Ala Leu Val Phe Pro Pro Leu Thr Gln Arg Thr
        35                  40                  45

Tyr Met Ser Glu Asn Ala Met Gly Ser Thr Met Val Glu Glu Gln Phe
    50                  55                  60

Ala Gly Gly Asp Arg Ala Arg Ala Phe Ala Arg Asp Phe Ala Ala His
65                  70                  75                  80

Arg Lys Lys Ser Gly Ala Leu Pro Val Ala Trp Leu Glu Arg Thr Met
                85                  90                  95

Arg Ser Val Gly Leu Glu Val Tyr Thr Gln Ser Phe Ser Arg Lys Leu
            100                 105                 110

Pro Phe Pro Asp Glu Thr His Glu Arg Tyr Met Val Ser Gly Thr Asn
        115                 120                 125

Val Tyr Gly Ile Leu Arg Ala Pro Xaa Ala Ala Ser Thr Glu Ser Leu
    130                 135                 140

Val Leu Thr Val Pro Cys Gly Ser Asp Ser Thr Asn Ser Gln Ala Val
145                 150                 155                 160

Gly Leu Leu Leu Ala Leu Ala Ala His Phe Arg Gly Gln Ile Tyr Trp
                165                 170                 175

Ala Lys Asp Ile Val Phe Leu Val Thr Glu His Asp Leu Leu Gly Thr
            180                 185                 190

Glu Ala Trp Leu Glu Ala Tyr His Asp Val Asn Val Thr Gly Met Gln
        195                 200                 205

Ser Ser Pro Leu Gln Gly Arg Ala Gly Ala Ile Gln Ala Ala Val Ala
    210                 215                 220

Leu Glu Leu Ser Ser Asp Val Val Thr Ser Leu Asp Val Ala Val Glu
225                 230                 235                 240

Gly Leu Asn Gly Gln Leu Pro Asn Leu Asp Leu Leu Asn Leu Phe Gln
                245                 250                 255

Thr Phe Cys Gln Lys Gly Gly Leu Leu Cys Thr Leu Gln Gly Lys Leu
            260                 265                 270

Gln Pro Glu Asp Trp Thr Ser Leu Asp Gly Pro Leu Gln Gly Leu Gln
        275                 280                 285

Thr Leu Leu Leu Met Val Leu Arg Gln Ala Ser Gly Arg Pro His Gly
    290                 295                 300

Ser His Gly Leu Phe Leu Arg Tyr Arg Val Glu Ala Leu Thr Leu Arg
305                 310                 315                 320

Gly Ile Asn Ser Phe Arg Gln Tyr Lys Tyr Asp Leu Val Ala Val Gly
                325                 330                 335

Lys Ala Leu Glu Gly Met Phe Arg Lys Leu Asn His Leu Leu Glu Arg
            340                 345                 350

Leu His Gln Ser Phe Phe Leu Tyr Leu Leu Pro Gly Leu Ser Arg Phe
        355                 360                 365

Val Ser Ile Gly Leu Tyr Met Pro Ala Val Gly Phe Leu Leu Leu Val
    370                 375                 380

Leu Gly Leu Lys Ala Leu Glu Leu Trp Met Gln Leu His Glu Ala Gly
385                 390                 395                 400

Met Gly Leu Glu Glu Pro Gly Gly Ala Pro Gly Pro Ser Val Pro Leu
                405                 410                 415

Pro Pro Ser Gln Gly Val Gly Leu Ala Ser Leu Val Ala Pro Leu Leu
            420                 425                 430

Ile Ser Gln Ala Met Gly Leu Ala Leu Tyr Val Leu Pro Val Leu Gly
        435                 440                 445

Gln His Val Ala Thr Gln His Phe Pro Val Ala Glu Ala Glu Ala Val
    450                 455                 460

Val Leu Thr Leu Leu Ala Ile Tyr Ala Ala Gly Leu Ala Leu Pro His
465                 470                 475                 480

Asn Thr His Arg Val Val Ser Thr Gln Ala Pro Asp Arg Gly Trp Met
                485                 490                 495

Ala Leu Lys Leu Val Ala Leu Ile Tyr Leu Ala Leu Gln Leu Gly Cys
            500                 505                 510

Ile Ala Leu Thr Asn Phe Ser Leu Gly Phe Leu Leu Ala Thr Thr Met
        515                 520                 525

Val Pro Thr Ala Ala Leu Ala Lys Pro His Gly Pro Arg Thr Leu Tyr
    530                 535                 540

Ala Ala Leu Leu Val Leu Thr Ser Pro Ala Ala Thr Leu Leu Gly Ser
545                 550                 555                 560

Leu Phe Leu Trp Arg Glu Leu Gln Glu Ala Pro Leu Ser Leu Ala Glu
                565                 570                 575

Gly Trp Gln Leu Phe Leu Ala Ala Leu Ala Gln Gly Val Leu Glu His
            580                 585                 590

His Thr Tyr Gly Ala Leu Leu Phe Pro Leu Leu Ser Leu Gly Leu Tyr
        595                 600                 605

Pro Cys Trp Leu Leu Phe Trp Asn Val Leu Phe Trp Lys
610                 615                 620

<210> 103
<211> 287
<212> PRT
<213> Homo sapiens

<220>
<221> SITE
<222> (263)
<223> Xaa equals any one of the naturally occurring L-amino acids

<400> 103
Met Ala Leu Leu Pro Ile Phe Phe Gly Ala Leu Arg Ser Val Arg Cys
1               5                   10                  15

Ala Arg Gly Lys Asn Ala Ser Asp Met Pro Glu Thr Ile Thr Ser Arg
            20                  25                  30

Asp Ala Ala Arg Phe Pro Ile Ile Ala Ser Cys Thr Leu Leu Gly Leu
        35                  40                  45

Tyr Leu Phe Phe Lys Ile Phe Ser Gln Glu Tyr Ile Asn Leu Leu Leu
    50                  55                  60

Ser Met Tyr Phe Phe Val Leu Gly Ile Leu Ala Leu Ser His Thr Ile
65                  70                  75                  80

Ser Pro Phe Met Asn Lys Phe Phe Pro Ala Ser Phe Pro Asn Arg Gln
                85                  90                  95

Tyr Gln Leu Leu Phe Thr Gln Gly Ser Gly Glu Asn Lys Glu Glu Ile
            100                 105                 110

Ile Asn Tyr Glu Phe Asp Thr Lys Asp Leu Val Cys Leu Gly Leu Ser
        115                 120                 125

Ser Ile Val Gly Val Trp Tyr Leu Leu Arg Lys His Trp Ile Ala Asn
    130                 135                 140

Asn Leu Phe Gly Leu Ala Phe Ser Leu Asn Gly Val Glu Leu Leu His
145                 150                 155                 160

Leu Asn Asn Val Ser Thr Gly Cys Ile Leu Leu Gly Gly Leu Phe Ile
                165                 170                 175

Tyr Asp Val Phe Trp Val Phe Gly Thr Asn Val Met Val Thr Val Ala
            180                 185                 190

Lys Ser Phe Glu Ala Pro Ile Lys Leu Val Phe Pro Gln Asp Leu Leu
        195                 200                 205

Glu Lys Gly Leu Glu Ala Asn Asn Phe Ala Met Leu Gly Leu Gly Asp
    210                 215                 220

Val Val Ile Pro Gly Ile Phe Ile Ala Leu Leu Leu Arg Phe Asp Ile
225                 230                 235                 240

Ser Leu Lys Lys Asn Thr His Thr Tyr Phe Tyr Thr Ser Phe Ala Ala
                245                 250                 255

Tyr Ile Phe Gly Leu Gly Xaa Tyr His Leu His His Ala His Leu Gln
            260                 265                 270

Ala Cys Ser Val Met Arg Ser Gln Ile Leu Arg Ile Gln Arg Gln
        275                 280                 285

<210> 104
<211> 32
<212> PRT
<213> Homo sapiens

<220>
<221> SITE
<222> (32)
<223> Xaa equals stop translation

<400> 104
Met Ser Arg Leu Leu Leu Leu Phe Gly Arg Leu Cys Ser Leu Trp Cys
1               5                   10                  15

Leu Ser Trp Leu Tyr Ser Thr Asp Thr Arg Pro Leu Leu Arg Gly Xaa
            20                  25                  30

<210> 105
<211> 77
<212> PRT
<213> Homo sapiens

<400> 105

<210> 106
<211> 45
<212> PRT
<213> Homo sapiens

<220>

<221> SITE
<222> (45)
<223> Xaa equals stop translation

<400> 106
Met Val Phe Leu Val Phe Tyr Val Leu Arg Ala Leu Lys Cys Asn Ser
1               5                   10                  15

Ser Tyr His Ser Cys Thr Asn Val Leu Thr Gln Ile Ala Ser Gln Ile
            20                  25                  30

Asp Lys Thr Leu Asn Asn Phe Ser Leu Lys Lys Cys Xaa
        35                  40                  45


<210> 107
<211> 42
<212> PRT
<213> Homo sapiens

<220>
<221> SITE
<222> (42)
<223> Xaa equals stop translation

<400> 107
Met Asn Pro Cys Leu Ser Ile Ile Phe Leu Leu Thr Pro Val Leu Leu
1               5                   10                  15

Ser His Pro Leu Gln Ser Leu His Phe Leu Leu Lys Val Asp Leu Asp
            20                  25                  30

Phe Ser Leu Ser Cys Ser Ile Cys Thr Xaa
        35                  40


<210> 108
<211> 70
<212> PRT
<213> Homo sapiens

<220>
<221> SITE
<222> (70)
<223> Xaa equals stop translation

<400> 108
Met Thr Val Tyr Leu Leu Lys Thr His Pro Cys Phe Phe Val Ala Tyr
1               5                   10                  15

Gln Met Gln Val Ala Leu Ile Ile Leu Leu Pro Gly Leu Arg Asn Ser
            20                  25                  30

Lys Thr Val Thr Met Pro Leu Ser Pro Ala Leu Leu Pro Thr Leu Leu
        35                  40                  45

Phe Phe Pro Ser Pro Thr Pro Phe Phe His Pro Phe Leu Ser Val Leu
    50                  55                  60

Cys Cys Phe Lys Tyr Xaa

65                  70

<210> 109
<211> 49
<212> PRT
<213> Homo sapiens

<220>
<221> SITE
<222> (43)
<223> Xaa equals any one of the naturally occurring L-amino acids

<220>
<221> SITE
<222> (49)
<223> Xaa equals stop translation

<400> 109
Met His Ala Thr Cys Thr Arg Thr Trp Arg Ala Gln Val Ser Leu His
1               5                   10                  15

Gln Pro Pro Cys Ser Arg Asp Trp Lys Ile Cys His Leu Leu Val Val
            20                  25                  30

Leu Ser Leu Pro Pro Pro Thr Pro Ala Arg Xaa Pro Glu Phe Leu Asn
        35                  40                  45

Xaa

<210> 110
<211> 193
<212> PRT
<213> Homo sapiens

<220>
<221> SITE
<222> (193)
<223> Xaa equals stop translation

<400> 110
Met Ile Arg Asn Asp Gln Asp Ser Leu Met Gln Leu Leu Gln Leu Gly
1               5                   10                  15

Leu Val Val Leu Gly Ser Gln Glu Ser Gln Glu Ser Asp Leu Ser Lys
            20                  25                  30

Gln Leu Ile Ser Val Ile Ile Gly Leu Gly Val Ala Leu Leu Leu Val
        35                  40                  45

Leu Val Ile Met Thr Met Ala Phe Val Cys Val Arg Lys Ser Tyr Asn
    50                  55                  60

Arg Lys Leu Gln Ala Met Lys Ala Ala Lys Glu Ala Arg Lys Thr Ala
65                  70                  75                  80

Ala Gly Val Met Pro Ser Ala Pro Ala Ile Pro Gly Thr Asn Met Tyr
                85                  90                  95

Asn Thr Glu Arg Ala Asn Pro Met Leu Asn Leu Pro Asn Lys Asp Leu
            100                 105                 110

Gly Leu Glu Tyr Leu Ser Pro Ser Asn Asp Leu Asp Ser Val Ser Val
        115                 120                 125

Asn Ser Leu Asp Asp Asn Ser Val Asp Val Asp Lys Asn Ser Gln Glu
    130                 135                 140

Ile Lys Glu His Arg Pro Pro His Thr Pro Pro Glu Pro Asp Pro Glu
145                 150                 155                 160

Pro Leu Ser Val Val Leu Leu Gly Arg Gln Ala Gly Ala Ser Gly Gln
                165                 170                 175

Leu Glu Gly Pro Ser Tyr Thr Asn Ala Gly Leu Asp Thr Thr Asp Leu
            180                 185                 190

Xaa

<210> 111
<211> 71
<212> PRT
<213> Homo sapiens

<220>
<221> SITE
<222> (64)
<223> Xaa equals any one of the naturally occurring L-amino acids

<400> 111
Met Ala His Val Val Val Ala Arg Asn Glu Cys Leu Ile Arg Ala Phe
1               5                   10                  15

Leu Phe Leu Leu His Cys Val Ser Leu Leu Pro Ser Pro Gly Glu Val
            20                  25                  30

Asn Ile Arg His Thr Leu Phe Thr Val Glu Glu Arg Leu Thr Thr Pro
        35                  40                  45

Arg Ala Leu Lys Leu Ser Leu Ser Leu Ile Val Ser Leu His Ala Xaa
    50                  55                  60

Cys Arg Lys Gln Glu Cys Ser
65                  70

<210> 112
<211> 36
<212> PRT
<213> Homo sapiens

<220>
<221> SITE
<222> (36)
<223> Xaa equals stop translation

<400> 112
Met Arg Leu Thr Glu Lys Asp Thr Val Leu Phe Thr Lys Gly Val Leu
1               5                   10                  15

Phe Leu His Leu Phe Ile Asn Ala Leu Phe Trp Tyr Cys Lys Phe Gly
            20                  25                  30

His Asn Phe Xaa
        35

<210> 113
<211> 60
<212> PRT
<213> Homo sapiens

<220>
<221> SITE
<222> (60)
<223> Xaa equals stop translation

<400> 113
Met Thr Ser Val Ser Thr Gln Leu Ser Leu Val Leu Met Ser Leu Leu
1               5                   10                  15

Leu Val Leu Pro Val Val Glu Ala Val Glu Ala Gly Asp Ala Ile Ala
            20                  25                  30

Leu Leu Leu Gly Val Val Leu Ser Ile Thr Gly Ile Cys Ala Cys Leu
        35                  40                  45

Gly Val Tyr Ala Arg Lys Arg Asn Gly Gln Met Xaa
    50                  55                  60

<210> 114
<211> 29
<212> PRT
<213> Homo sapiens

<220>
<221> SITE
<222> (29)
<223> Xaa equals stop translation

<400> 114
Met Asn Ser Phe Trp Ser Lys Leu Leu Val Leu Pro Leu Leu Ala Pro
1               5                   10                  15

Leu Ser Met Ala Arg Ala Ser Ala Cys Gln Arg Trp Xaa
            20                  25

<210> 115
<211> 25
<212> PRT
<213> Homo sapiens

<220>
<221> SITE

<222> (25)
<223> Xaa equals stop translation

<400> 115
Met Met Arg Leu Leu Asp Leu Arg Ile Phe Leu Met Ile His His Lys
1               5                   10                  15

Ala Lys Ser Trp Glu Ser His Thr Xaa
            20                  25

<210> 116
<211> 35
<212> PRT
<213> Homo sapiens

<220>
<221> SITE
<222> (35)
<223> Xaa equals stop translation

<400> 116
Met Pro Leu Ser Leu Leu Leu Ile Val Trp Lys Leu Glu Leu Cys Val
1               5                   10                  15

Gly Ser Ala Leu Val Leu Ile His Thr Gln Arg Arg Tyr Ile Ile Leu
            20                  25                  30

Gln Val Xaa
        35

<210> 117
<211> 78
<212> PRT
<213> Homo sapiens

<220>
<221> SITE
<222> (78)
<223> Xaa equals stop translation

<400> 117
Met Leu Leu Ala Thr Leu Leu Leu Leu Leu Leu Gly Gly Ala Leu Ala
1               5                   10                  15

His Pro Asp Arg Ile Ile Phe Pro Asn His Ala Cys Glu Asp Pro Pro
            20                  25                  30

Ala Val Leu Leu Glu Val Gln Gly Thr Leu Gln Arg Pro Leu Val Arg
        35                  40                  45

Asp Ser Arg Thr Ser Pro Ala Asn Cys Thr Trp Leu Thr Lys Arg Val
    50                  55                  60

Gln Gln Met Leu Leu Phe His Ser Tyr Gly Ile Ala Gln Xaa
65                  70                  75

<210> 118

<211> 44
<212> PRT
<213> Homo sapiens

<220>
<221> SITE
<222> (44)
<223> Xaa equals stop translation

<400> 118
Met Thr Gly Val Phe Lys Leu Pro Leu Leu Phe Trp Val His Glu Ala
1               5                   10                  15

Ser Val Gly Gly Cys Pro Tyr Val Lys Leu Val Glu Phe Glu Glu Met
            20                  25                  30

Leu Thr Leu Tyr Gly Ile Leu Leu Ile Leu Phe Xaa
        35                  40

<210> 119
<211> 46
<212> PRT
<213> Homo sapiens

<220>
<221> SITE
<222> (46)
<223> Xaa equals stop translation

<400> 119
Met Gln Leu Ala Pro Phe Ile Ser Ile Pro Val Leu Ser Gly Thr Thr
1               5                   10                  15

Pro Trp Thr Ala Val Phe Arg Ala Ser Ser Ile Cys Thr Pro Leu Leu
            20                  25                  30

Thr Leu Ser Ala Ala Gly Met Leu Val Glu Ser Ser Leu Xaa
        35                  40                  45

<210> 120
<211> 29
<212> PRT
<213> Homo sapiens

<220>
<221> SITE
<222> (29)
<223> Xaa equals stop translation

<400> 120
Met Pro Pro Leu Ser Asp Ile Leu Leu Thr Val Ala Val Val Ala Phe
1               5                   10                  15

Glu Met Thr Gly His Ile Tyr Ile Trp Pro His Thr Xaa
            20                  25

<210> 121

<211> 62
<212> PRT
<213> Homo sapiens

<400> 121


<210> 122
<211> 87
<212> PRT
<213> Homo sapiens

<220>
<221> SITE
<222> (68)
<223> Xaa equals any one of the naturally occurring L-amino acids

<220>
<221> SITE
<222> (72)
<223> Xaa equals any one of the naturally occurring L-amino acids

<220>
<221> SITE
<222> (87)
<223> Xaa equals stop translation

<400> 122
Met Pro Val Ser Trp Gly Cys Pro Ser Lys Thr Pro Gln Thr Arg Ala
1               5                   10                  15

Tyr Thr Arg Cys Val Tyr Phe Leu Met Val Leu Glu Ala Gly Val Gly
            20                  25                  30

Gly His Ser Val Ser Arg Val Gly Ser Leu Glu Val Pro Pro Trp Leu
        35                  40                  45

Val Ala Ala Asn Asn Phe Pro His Leu Met Trp Ser Ser Phe Cys Val
    50                  55                  60

Gly Pro His Xaa Val Phe Leu Xaa Asp Pro Ser Leu Pro Asp Pro Gly
65                  70                  75                  80

Pro Pro Asn Asn Leu Thr Xaa
                85


<210> 123
<211> 64

<212> PRT
<213> Homo sapiens

<220>
<221> SITE
<222> (64)
<223> Xaa equals stop translation

<400> 123
Met Cys Tyr Phe Leu Glu Ile Ser Leu Leu Met Val Phe Ala Leu Asn
1               5                   10                  15

Ile Lys Ala Ala Tyr Gly Cys Cys Asn Ile Asn Gly Thr Glu Val His
            20                  25                  30

Arg Ala Lys Gly Pro Val Ser Val Pro Phe Pro Leu Ser Arg Pro Leu
        35                  40                  45

Ser Gly Thr Pro Leu Leu Asp Arg Leu Arg Pro Phe Gln Thr Leu Xaa
    50                  55                  60

<210> 124
<211> 36
<212> PRT
<213> Homo sapiens

<220>
<221> SITE
<222> (36)
<223> Xaa equals stop translation

<400> 124
Met Pro Leu Pro Ser Ser Phe Pro Leu Pro Val Phe Leu Ser Ser Cys
1               5                   10                  15

Pro Phe Leu Met Ser Val Ser Ile Gly Phe Leu Ile Leu Val Phe Asn
            20                  25                  30

Val His Pro Xaa
        35

<210> 125
<211> 32
<212> PRT
<213> Homo sapiens

<220>
<221> SITE
<222> (32)
<223> Xaa equals stop translation

<400> 125
Met Phe Ile Phe Cys Val Ser Leu Ala Phe Leu Pro Arg Phe Ile Ser
1               5                   10                  15

Pro Gln Ser Cys Glu Trp Ala Gly Leu Ser Leu Val Trp His His Xaa
            20                  25                  30

<210> 126
<211> 41
<212> PRT
<213> Homo sapiens

<220>
<221> SITE
<222> (41)
<223> Xaa equals stop translation

<400> 126
Met Lys Asn Asn Thr Gln Lys Arg Leu Phe Leu Trp Gly Glu Leu Leu
  1               5                  10                  15

Leu Gln Asp Leu Ala Leu Ile Leu Tyr Leu Ser Ile Phe Leu Lys Ser
            20                  25                  30

Thr Leu Thr Asn Leu Asn Leu Phe Xaa
        35                  40

<210> 127
<211> 28
<212> PRT
<213> Homo sapiens

<220>
<221> SITE
<222> (28)
<223> Xaa equals stop translation

<400> 127
Met Leu Asn Val Phe Phe Ser Leu Ile Leu Phe Phe Ser Pro Asn Arg
  1               5                  10                  15

Ala Leu Pro Ala Ile Ser Ser Cys Ile Thr Phe Xaa
            20                  25

<210> 128
<211> 69
<212> PRT
<213> Homo sapiens

<220>
<221> SITE
<222> (69)
<223> Xaa equals stop translation

<400> 128
Met Arg Ala Val Gly Glu Arg Leu Leu Leu Lys Leu Gln Arg Leu Pro
  1               5                  10                  15

Gln Ala Glu Pro Val Glu Ile Val Ala Phe Ser Val Ile Ile Leu Phe
            20                  25                  30

Thr Ala Thr Val Leu Leu Leu Leu Leu Ile Ala Cys Ser Cys Cys Cys
        35                  40                  45

Thr His Cys Cys Cys Pro Glu Arg Arg Gly Arg Lys Val Gln Val Gln
    50                  55                  60

Pro Thr Pro Pro Xaa
65

<210> 129
<211> 87
<212> PRT
<213> Homo sapiens

<400> 129
Met Asp Pro Arg Arg Val Thr Ala Cys Cys His Val Trp Thr Val Gly
1               5                   10                  15

Leu Phe Cys Ile Trp Ala Val Gly Leu Ser Cys Ser Leu Ser Leu Ser
            20                  25                  30

His Val Ile Val Trp Leu Ser Gly Ala Gly Cys Thr Leu Ile Cys Glu
        35                  40                  45

Asp Asn Pro Phe Leu Leu Leu Phe Ser Gln Tyr Leu Gln Pro His His
    50                  55                  60

Pro Glu Ile Met Lys Pro Phe Ile Leu Gly His Lys Ser Ser Asn Gly
65                  70                  75                  80

Gly Leu Ser Pro Pro Ser Ala
                85

<210> 130
<211> 64
<212> PRT
<213> Homo sapiens

<220>
<221> SITE
<222> (64)
<223> Xaa equals stop translation

<400> 130
Met Phe Tyr Met Val Cys Val Leu Gly Ser Gly Ala Gln Pro Leu Ser
1               5                   10                  15

Glu Leu Ala Tyr Leu Ala Lys Leu Pro Thr Leu Gln Val Gly Lys Tyr
            20                  25                  30

Asn Pro Leu Phe Asn Lys Ala His Pro Leu His Pro Val Leu Thr Thr
        35                  40                  45

Phe Cys Glu Cys Ala Val Ile Phe Ser Cys Ser Ile Ala Arg Trp Xaa
    50                  55                  60

<210> 131
<211> 54
<212> PRT
<213> Homo sapiens

<400> 131
Met Arg Phe Gln Ser Tyr Leu Trp Pro Ser Arg Ile Leu Val Gly Thr
1               5                   10                  15

Tyr Cys Ile Ala Ala Glu Val Leu Phe Pro Ser Ala Leu Ala Ser Cys
            20                  25                  30

Gly Pro Val Trp Gln Gly Gly Ala Pro Thr Lys Ser Trp Gln Pro Gly
        35                  40                  45

Ala Lys Thr Ile Ile Pro
    50


<210> 132
<211> 41
<212> PRT
<213> Homo sapiens

<220>
<221> SITE
<222> (41)
<223> Xaa equals stop translation

<400> 132
Met Arg Arg Trp Ala Gly Phe Gly Lys Ser Pro Gln Phe Trp Trp Thr
1               5                   10                  15

Gly Ile Leu Val Ala Leu Gly Ala Ala Leu Leu Gly Gly Pro Arg Leu
            20                  25                  30

Gly Arg Arg Leu Thr Phe Gly Leu Xaa
        35                  40


<210> 133
<211> 69
<212> PRT
<213> Homo sapiens

<220>
<221> SITE
<222> (69)
<223> Xaa equals stop translation

<400> 133
Met Ala Leu Ala Ile Phe Ile Pro Val Leu Ile Ile Ser Leu Leu Leu
1               5                   10                  15

Gly Gly Ala Tyr Ile Tyr Ile Thr Arg Cys Arg Tyr Tyr Ser Asn Leu

            20                  25                  30

Arg Leu Pro Leu Met Tyr Ser His Pro Tyr Ser Gln Ile Thr Val Glu
        35                  40                  45

Thr Glu Phe Asp Asn Pro Ile Tyr Glu Thr Gly Glu Thr Arg Glu Tyr
    50                  55                  60

Glu Val Ser Ile Xaa
65

<210> 134
<211> 48
<212> PRT
<213> Homo sapiens

<220>
<221> SITE
<222> (48)
<223> Xaa equals stop translation

<400> 134
Met Gly Phe Leu Phe Leu His Ile Leu Pro Ser Ile Ile Asn Thr Arg
1               5                   10                  15

Ser Ala Pro Gln Pro Thr Ser Cys Arg Met Gln Pro Glu Gln Gln Pro
            20                  25                  30

His Ser Thr Leu Lys Pro Val Ile Leu Gly Met Met Ile Ile Ser Xaa
        35                  40                  45

<210> 135
<211> 76
<212> PRT
<213> Homo sapiens

<400> 135
Met Ser Gly Leu Val Gly Gly Gly Ser Arg Cys Ser Lys Val Arg Phe
1               5                   10                  15

Arg Cys Phe Asn Gly Asp Ser Leu Leu Val Leu Val Leu Gln His His
            20                  25                  30

Phe Arg Leu Cys Ser Trp Cys Leu Ala Pro Ser Leu Phe Leu Leu Leu
        35                  40                  45

Ser Cys Gln Val Val Ser Thr Met Met Glu Gln Asp Pro Val Ile Tyr
    50                  55                  60

Asp Asp Asp Asp Asp Leu Pro Asn Tyr Phe Ser Val
65                  70                  75

<210> 136
<211> 55

<212> PRT
<213> Homo sapiens

<220>
<221> SITE
<222> (32)
<223> Xaa equals any one of the naturally occurring L-amino acids

<220>
<221> SITE
<222> (39)
<223> Xaa equals any one of the naturally occurring L-amino acids

<220>
<221> SITE
<222> (55)
<223> Xaa equals stop translation

<400> 136
Met Phe Leu Glu Leu Pro Met Gln His Ser Asp Val Leu Leu Phe Leu
1               5                   10                  15

Val Cys Trp Lys Ala Met Gly Ser Lys Lys Ser Pro Ser His Phe Xaa
            20                  25                  30

Pro Glu Val Gly Gly Ile Xaa Pro Ser Phe Gly Met Leu Asn Val Thr
        35                  40                  45

Leu Leu Arg Ser Leu Thr Xaa
    50                  55


<210> 137
<211> 54
<212> PRT
<213> Homo sapiens

<400> 137
Met Leu Val Leu Phe Pro Leu Leu Tyr Arg Gly Trp Ser Pro Val Pro
1               5                   10                  15

Gly Thr Ala Glu Gly Gly Met Cys Cys Cys Cys Leu Cys Ile Ser Arg
            20                  25                  30

Tyr Ser Leu Leu Thr Ser Ser Gln Asp Lys Glu Pro Pro Tyr Glu Met
        35                  40                  45

Ser Ser Ser Glu Leu Ser
    50


<210> 138
<211> 36
<212> PRT
<213> Homo sapiens

<220>
<221> SITE
<222> (33)
<223> Xaa equals any one of the naturally occurring L-amino acids

<220>
<221> SITE
<222> (36)
<223> Xaa equals stop translation

<400> 138
Met Thr Cys Tyr Glu Val Ile Leu Phe Phe Ile Lys Leu Phe Ser Asp
1               5                   10                  15

Met Gly Lys Tyr Lys Glu Cys Lys Glu Phe Lys Lys Gln Arg Thr Lys
            20                  25                  30

Xaa Tyr Met Xaa
        35

<210> 139
<211> 80
<212> PRT
<213> Homo sapiens

<400> 139

<210> 140
<211> 67
<212> PRT
<213> Homo sapiens

<400> 140

<210> 141
<211> 51
<212> PRT
<213> Homo sapiens

<220>
<221> SITE
<222> (51)
<223> Xaa equals stop translation

<400> 141
Met Gly Trp Leu Cys Cys Glu Pro Ser Gly Leu Tyr Asn Leu Glu Lys
1               5                   10                  15

Gln Tyr Phe Phe Phe Ser Ser Leu Gln Ala Gly Leu Pro Val Ile Val
            20                  25                  30

Ser Ser Gly Cys Thr Lys Ile Ala Tyr Gly Phe Ala Val Tyr Ser Pro
        35                  40                  45

Ser Ser Xaa
    50

<210> 142
<211> 54
<212> PRT
<213> Homo sapiens

<400> 142
Met Arg Arg Cys Val Arg His Val Leu Gly Ile Gly Leu Ile Val Leu
1               5                   10                  15

Lys Asn Leu Tyr Phe His Lys Asn Ser Met Tyr Pro Ser Pro Lys Leu
            20                  25                  30

Ser Ser Phe Gln Glu Ala Phe Leu Phe Phe Phe Leu Ile Leu Lys Asn
        35                  40                  45

Pro Leu Thr Leu Cys Ser
    50

<210> 143
<211> 50
<212> PRT
<213> Homo sapiens

<220>
<221> SITE
<222> (50)
<223> Xaa equals stop translation

<400> 143
Ile His Pro Ser Arg Ser Thr Leu Ser Ser Gln Leu Val Thr Leu Pro
1               5                   10                  15

Leu Phe Glu Leu Val Phe Pro Ile Pro Ser Ser Gln Ser Pro Phe Ser
            20                  25                  30

Leu Asn Tyr Leu Ser Glu Phe Pro Leu Pro Glu His Glu Pro Cys Leu
        35                  40                  45

Glu Xaa
    50

<210> 144
<211> 87
<212> PRT
<213> Homo sapiens

<220>
<221> SITE
<222> (84)
<223> Xaa equals any one of the naturally occurring L-amino acids

<220>
<221> SITE
<222> (87)
<223> Xaa equals stop translation

<400> 144
Met Thr Cys Cys Cys Leu Leu Cys Lys Leu Gln Gly Ile Phe Phe Phe
1               5                   10                  15

Ser Phe Asn Ser Ser Val Leu Lys Ser Ile Leu Gly Thr Thr Arg Thr
            20                  25                  30

Leu Ser Ala Pro Trp Ile Gly Val Ser Val Lys Gly Thr Gln Trp Ala
        35                  40                  45

Leu Gly Ser Ala Arg Pro Gly Cys Gly Ser Gln Leu Thr Ser Ser Leu
    50                  55                  60

Gly Gly Leu Arg Gln Val Ile Cys Gln Pro His Leu Gln Lys His Asp
65                  70                  75                  80

Ala Lys Leu Xaa Ser Val Xaa
                85

<210> 145
<211> 57
<212> PRT
<213> Homo sapiens

<400> 145
Met His Lys Cys Asn Thr Val Thr Arg Glu Leu Leu Gln Leu Ser Leu
1               5                   10                  15

Leu Ile Leu Pro Ser Gln Cys Gly Asn Cys Ala Thr Ser Thr Lys Arg
            20                  25                  30

Gly Pro Arg Leu Leu Lys Tyr Phe Arg Thr Ser Pro Gln Glu Gln Thr
        35                  40                  45

Pro Leu His Leu Asp Ser Asp Cys Ser
    50                  55

<210> 146
<211> 87
<212> PRT
<213> Homo sapiens

<400> 146

<210> 147
<211> 230
<212> PRT
<213> Homo sapiens

<220>
<221> SITE
<222> (216)
<223> Xaa equals any one of the naturally occurring L-amino acids

<400> 147
Met Gly Leu Ala Leu Tyr Val Leu Pro Val Leu Gly Gln His Val Ala
1               5                   10                  15

Thr Gln His Phe Pro Val Ala Glu Ala Glu Ala Val Val Leu Thr Leu
            20                  25                  30

Leu Ala Ile Tyr Ala Ala Gly Leu Ala Leu Pro His Asn Thr His Arg
        35                  40                  45

Val Val Ser Thr Gln Ala Pro Asp Arg Gly Trp Met Ala Leu Lys Leu
    50                  55                  60

Val Ala Leu Ile Tyr Leu Ala Leu Gln Leu Gly Cys Ile Ala Leu Thr
65                  70                  75                  80

Asn Phe Ser Leu Gly Phe Leu Leu Ala Thr Thr Met Val Pro Thr Ala
                85                  90                  95

Ala Leu Ala Lys Pro His Gly Pro Arg Thr Leu Tyr Ala Ala Leu Leu

            100                 105                 110

Val Leu Thr Ser Pro Ala Ala Thr Leu Leu Gly Ser Leu Phe Leu Trp
        115                 120                 125

Arg Glu Leu Gln Glu Ala Pro Leu Ser Leu Ala Glu Gly Trp Gln Leu
    130                 135                 140

Phe Leu Ala Ala Leu Ala Gln Gly Val Leu Glu His His Thr Thr Ala
145                 150                 155                 160

Pro Cys Ser Ser His Cys Cys Pro Trp Ala Ser Thr Pro Ala Gly Cys
                165                 170                 175

Phe Ser Gly Met Cys Ser Ser Gly Ser Glu Ile Cys Leu Ser Gly Leu
            180                 185                 190

Gly Gln Arg Leu Pro Lys Asp Pro Ile Leu Pro Pro Ser Gly Glu Ile
        195                 200                 205

Asn Glu Cys Leu Phe Gln Gln Xaa Lys Lys Lys Lys Lys Lys Lys Lys
    210                 215                 220

Lys Lys Lys Lys Gly Gly
225                 230

<210> 148
<211> 63
<212> PRT
<213> Homo sapiens

<220>
<221> SITE
<222> (63)
<223> Xaa equals stop translation

<400> 148
Gln Pro Ala Leu Leu Tyr Leu Val Pro Ala Cys Ile Gly Phe Pro Val
1               5                   10                  15

Leu Val Ala Leu Ala Lys Gly Glu Val Thr Glu Met Phe Ser Tyr Glu
            20                  25                  30

Glu Ser Asn Pro Lys Asp Pro Ala Ala Val Thr Glu Ser Lys Glu Gly
        35                  40                  45

Thr Glu Ala Ser Ala Ser Lys Gly Leu Glu Lys Lys Glu Lys Xaa
    50                  55                  60

<210> 149
<211> 18
<212> PRT
<213> Homo sapiens

<220>
<221> SITE
<222> (18)
<223> Xaa equals stop translation

<400> 149
Gln Leu Ile Leu Ser Leu Leu Arg Gly Phe Cys Lys Thr Glu Arg Val
1               5                   10                  15

Gly Xaa

<210> 150
<211> 16
<212> PRT
<213> Homo sapiens

<220>
<221> SITE
<222> (16)
<223> Xaa equals stop translation

<400> 150
Met Ala Leu Gly Ala Arg Glu Leu Pro Gly Ser Leu Ser Arg Trp Xaa
1               5                   10                  15

<210> 151
<211> 22
<212> PRT
<213> Homo sapiens

<220>
<221> SITE
<222> (22)
<223> Xaa equals stop translation

<400> 151
Met Tyr Ser Phe Ser Val Leu Glu Ile Thr Cys Phe Ile Leu Phe Leu
1               5                   10                  15

Trp Pro Ser Trp Val Xaa
            20

<210> 152
<211> 25
<212> PRT
<213> Homo sapiens

<220>
<221> SITE
<222> (25)
<223> Xaa equals stop translation

<400> 152
Met Lys Ile Lys Gln Arg Phe Ser Leu Leu Leu Phe His Cys Pro Phe
1               5                   10                  15

Pro Pro Cys Cys Leu Ser Leu Gly Xaa

            20                  25

<210> 153
<211> 40
<212> PRT
<213> Homo sapiens

<400> 153
Met Asn Gly Leu Phe Gln Leu Glu Ile Ser His Lys Leu Trp Thr Lys
1               5                   10                  15

Ser Lys Thr Ser Leu Met Thr Leu Leu Ser Val Met Ala Leu Leu Trp
            20                  25                  30

Lys Ile Leu Trp Ser Arg Ala Ile
        35                  40

<210> 154
<211> 25
<212> PRT
<213> Homo sapiens

<220>
<221> SITE
<222> (25)
<223> Xaa equals stop translation

<400> 154
Met Thr Pro Gly Leu Phe Leu Tyr Phe Val Cys Val Cys Val Ser His
1               5                   10                  15

Cys Ala Gly Leu Gly Gln Leu Ser Xaa
            20                  25

<210> 155
<211> 103
<212> PRT
<213> Homo sapiens

<400> 155

<210> 156
<211> 46
<212> PRT
<213> Homo sapiens

<400> 156
Phe Leu Ala Ile His Phe Pro Thr Asp Phe Pro Leu Lys Pro Pro Lys
1               5                   10                  15

Val Ala Phe Thr Arg Met Tyr Phe Pro Asn Ser Asn Ser Asn Gly Ser
            20                  25                  30

Thr Cys Leu Asp Ile Leu Trp Ser Gln Trp Ser Pro Ala Leu
        35                  40                  45

<210> 157
<211> 101
<212> PRT
<213> Homo sapiens

<400> 157
Met Leu Leu Thr Pro His Phe Asn Val Ala Asn Pro Gln Asn Leu Leu
1               5                   10                  15

Ala Gly Leu Trp Leu Glu Asn Glu His Ser Phe Thr Leu Met Ala Pro
            20                  25                  30

Glu Arg Ala Arg Thr His His Cys Gln Pro Glu Glu Arg Lys Val Leu
        35                  40                  45

Phe Cys Leu Phe Pro Ile Val Pro Asn Ser Gln Ala Gln Val Gln Pro
    50                  55                  60

Pro Gln Met Pro Pro Phe Cys Cys Ala Ala Ala Lys Glu Lys Thr Gln
65                  70                  75                  80

Glu Glu Gln Leu Gln Glu Pro Leu Gly Ser Gln Cys Pro Asp Thr Cys
                85                  90                  95

Pro Asn Ser Leu Cys
            100

<210> 158
<211> 211
<212> PRT
<213> Homo sapiens

<400> 158
Met Arg Leu Phe Leu Trp Asn Ala Val Leu Thr Leu Phe Val Thr Ser
1               5                   10                  15

Leu Ile Gly Ala Leu Ile Pro Glu Pro Glu Val Lys Ile Glu Val Leu
            20                  25                  30

Gln Lys Pro Phe Ile Cys His Arg Lys Thr Lys Gly Gly Asp Leu Met
        35                  40                  45

Leu Val His Tyr Glu Gly Tyr Leu Glu Lys Asp Gly Ser Leu Phe His
    50                  55                  60

Ser Thr His Lys His Asn Asn Gly Gln Pro Ile Trp Phe Thr Leu Gly
65                  70                  75                  80

Ile Leu Glu Ala Leu Lys Gly Trp Asp Gln Gly Leu Lys Gly Met Cys
                85                  90                  95

Val Gly Glu Lys Arg Lys Leu Ile Ile Pro Pro Ala Leu Gly Tyr Gly
            100                 105                 110

Lys Glu Gly Lys Gly Lys Ile Pro Pro Glu Ser Thr Leu Ile Phe Asn
        115                 120                 125

Ile Asp Leu Leu Glu Ile Arg Asn Gly Pro Arg Ser His Glu Ser Phe
    130                 135                 140

Gln Glu Met Asp Leu Asn Asp Asp Trp Lys Leu Ser Lys Asp Glu Val
145                 150                 155                 160

Lys Ala Tyr Leu Lys Lys Glu Phe Glu Lys His Gly Ala Val Val Asn
                165                 170                 175

Glu Ser His His Asp Ala Leu Val Glu Asp Ile Phe Asp Lys Glu Asp
            180                 185                 190

Glu Asp Lys Asp Gly Phe Ile Ser Ala Arg Glu Phe Thr Tyr Lys His
        195                 200                 205

Asp Glu Leu
    210

<210> 159
<211> 186
<212> PRT
<213> Homo sapiens

<400> 159
Glu Val Lys Ile Glu Val Leu Gln Lys Pro Phe Ile Cys His Arg Lys
1               5                   10                  15

Thr Lys Gly Gly Asp Leu Met Leu Val His Tyr Glu Gly Tyr Leu Glu
            20                  25                  30

Lys Asp Gly Ser Leu Phe His Ser Thr His Lys His Asn Asn Gly Gln
        35                  40                  45

Pro Ile Trp Phe Thr Leu Gly Ile Leu Glu Ala Leu Lys Gly Trp Asp
    50                  55                  60

Gln Gly Leu Lys Gly Met Cys Val Gly Glu Lys Arg Lys Leu Ile Ile
65                  70                  75                  80

Pro Pro Ala Leu Gly Tyr Gly Lys Glu Gly Lys Gly Lys Ile Pro Pro

85                    90                    95

Glu Ser Thr Leu Ile Phe Asn Ile Asp Leu Leu Glu Ile Arg Asn Gly
            100                 105                 110

Pro Arg Ser His Glu Ser Phe Gln Glu Met Asp Leu Asn Asp Asp Trp
        115                 120                 125

Lys Leu Ser Lys Asp Glu Val Lys Ala Tyr Leu Lys Lys Glu Phe Glu
    130                 135                 140

Lys His Gly Ala Val Val Asn Glu Ser His His Asp Ala Leu Val Glu
145                 150                 155                 160

Asp Ile Phe Asp Lys Glu Asp Glu Asp Lys Asp Gly Phe Ile Ser Ala
                165                 170                 175

Arg Glu Phe Thr Tyr Lys His Asp Glu Leu
            180                 185

<210> 160
<211> 633
<212> DNA
<213> Homo sapiens

<400> 160
ATGAGGCTTT TCTTGTGGAA CGCGGTCTTG ACTCTGTTCG TCACTTCTTT GATTGGGGCT     60

TTGATCCCTG AACCAGAAGT GAAAATTGAA GTTCTCCAGA AGCCATTCAT CTGCCATCGC    120

AAGACCAAAG GAGGGGATTT GATGTTGGTC CACTATGAAG GCTACTTAGA AAAGGACGGC    180

TCCTTATTTC ACTCCACTCA CAAACATAAC AATGGTCAGC CCATTTGGTT TACCCTGGGC    240

ATCCTGGAGG CTCTCAAAGG TTGGGACCAG GGCTTGAAAG GAATGTGTGT AGGAGAGAAG    300

AGAAAGCTCA TCATTCCTCC TGCTCTGGGC TATGGAAAAG AAGGAAAAGG TAAAATTCCC    360

CCAGAAAGTA CACTGATATT TAATATTGAT CTCCTGGAGA TTCGAAATGG ACCAAGATCC    420

CATGAATCAT TCCAAGAAAT GGATCTTAAT GATGACTGGA AACTCTCTAA AGATGAGGTT    480

AAAGCATATT TAAAGAAGGA GTTTGAAAAA CATGGTGCGG TGGTGAATGA AAGTCATCAT    540

GATGCTTTGG TGGAGGATAT TTTTGATAAA GAAGATGAAG ACAAAGATGG GTTTATATCT    600

GCCAGAGAAT TTACATATAA ACACGATGAG TTA                                 633

<210> 161
<211> 22
<212> PRT
<213> Homo sapiens

<400> 161
Leu Arg Ser Val Val Gln Asp His Pro Gly Gln His Gly Glu Thr Pro
1               5                   10                  15

Ser Leu Leu Lys Ile Gln
            20

<210> 162
<211> 57
<212> PRT
<213> Homo sapiens

<220>
<221> SITE
<222> (34)
<223> Xaa equals any one of the naturally occurring L-amino acids

<400> 162
Met Phe Tyr Asn Phe Val Arg Gln Leu Asp Thr Val Ser Ile Glu His
 1               5                  10                  15

Ala Gly Lys Ser Lys Leu Lys Met Thr Val Gly Thr Lys Leu Thr Ser
            20                  25                  30

Gly Xaa Gly Pro Arg Lys Ser Ser Gln Ser Gly Arg Ile Ala Ala Ser
        35                  40                  45

Ile Thr Asp Cys Gln Gln Cys Lys Ala
    50                  55

<210> 163
<211> 46
<212> PRT
<213> Homo sapiens

<220>
<221> SITE
<222> (16)
<223> Xaa equals any one of the naturally occurring L-amino acids

<400> 163
Met Glu Ala Ala Ile Leu Pro Leu Trp Leu Leu Phe Leu Gly Pro Xaa
 1               5                  10                  15

Pro Glu Val Ser Phe Val Pro Thr Val Ile Phe Asn Leu Asp Phe Pro
            20                  25                  30

Ala Cys Ser Ile Leu Thr Val Ser Ser Cys Leu Thr Lys Leu
        35                  40                  45

<210> 164
<211> 25
<212> PRT
<213> Homo sapiens

<400> 164
Asn His Gly His Ser Cys Phe Leu Cys Glu Ile Val Ile Arg Ser Gln
 1               5                  10                  15

Phe His Thr Thr Tyr Glu Pro Glu Ala
            20                  25

<210> 165
<211> 48
<212> PRT
<213> Homo sapiens

<400> 165
Ser Gly Arg His Arg Val Glu Leu Gln Leu Leu Phe Pro Leu Val Arg
1               5                   10                  15

Val Asn Phe Glu Leu Gly Val Asn His Gly His Ser Cys Phe Leu Cys
            20                  25                  30

Glu Ile Val Ile Arg Ser Gln Phe His Thr Thr Tyr Glu Pro Glu Ala
        35                  40                  45

<210> 166
<211> 141
<212> PRT
<213> Homo sapiens

<400> 166
Met Asn Ala Arg Gly Leu Gly Ser Glu Leu Lys Asp Ser Ile Pro Val
1               5                   10                  15

Thr Glu Leu Ser Ala Ser Gly Pro Phe Glu Ser His Asp Leu Leu Arg
            20                  25                  30

Lys Gly Phe Ser Cys Val Lys Asn Glu Leu Leu Pro Ser His Pro Leu
        35                  40                  45

Glu Leu Ser Glu Lys Asn Phe Gln Leu Asn Gln Asp Lys Met Asn Phe
    50                  55                  60

Ser Thr Leu Arg Asn Ile Gln Gly Leu Phe Ala Pro Leu Lys Leu Gln
65                  70                  75                  80

Met Glu Phe Lys Ala Val Gln Gln Val Gln Arg Leu Pro Phe Leu Ser
                85                  90                  95

Ser Ser Asn Leu Ser Leu Asp Val Leu Arg Gly Asn Asp Glu Thr Ile
            100                 105                 110

Gly Phe Glu Asp Ile Leu Asn Asp Pro Ser Gln Ser Glu Val Met Gly
        115                 120                 125

Glu Pro His Leu Met Val Glu Tyr Lys Leu Gly Leu Leu
    130                 135                 140

<210> 167
<211> 15
<212> PRT
<213> Homo sapiens

<400> 167
Gly Gly Asn Lys Tyr Gln Thr Ile Asp Asn Tyr Gln Pro Tyr Pro
1               5                   10                  15

<210> 168
<211> 20
<212> PRT
<213> Homo sapiens

<400> 168
Pro Leu Leu Gly Val Ser Ala Thr Leu Asn Ser Val Leu Asn Ser Asn
1               5                   10                  15

Ala Ile Lys Asn
            20

<210> 169
<211> 14
<212> PRT
<213> Homo sapiens

<400> 169
Gly Ser Ala Val Ser Ala Ala Pro Gly Ile Leu Tyr Pro Gly
1               5                   10

<210> 170
<211> 81
<212> PRT
<213> Homo sapiens

<400> 170
Ala Gly Ile Gln His Glu Leu Ala Cys Asp Asn Pro Gly Leu Pro Glu
1               5                   10                  15

Asn Gly Tyr Gln Ile Leu Tyr Lys Arg Leu Tyr Leu Pro Gly Glu Ser
            20                  25                  30

Leu Thr Phe Met Cys Tyr Glu Gly Phe Glu Leu Met Gly Glu Val Thr
        35                  40                  45

Ile Arg Cys Ile Leu Gly Gln Pro Ser His Trp Asn Gly Pro Leu Pro
    50                  55                  60

Val Cys Lys Val Ala Glu Ala Ala Ala Glu Thr Ser Leu Glu Gly Gly
65                  70                  75                  80

Asn

<210> 171
<211> 27
<212> PRT
<213> Homo sapiens

<400> 171
Gln Pro Ser His Trp Asn Gly Pro Leu Pro Val Cys Lys Val Ala Glu

1                   5                   10                  15

Ala Ala Ala Glu Thr Ser Leu Glu Gly Gly Asn
            20                  25

<210> 172
<211> 13
<212> PRT
<213> Homo sapiens

<400> 172
Tyr Glu Thr Gly Glu Thr Arg Glu Tyr Glu Val Ser Ile
1               5                   10

<210> 173
<211> 195
<212> PRT
<213> Homo sapiens

<220>
<221> SITE
<222> (40)
<223> Xaa equals any one of the naturally occurring L-amino acids

<220>
<221> SITE
<222> (161)
<223> Xaa equals any one of the naturally occurring L-amino acids

<400> 173
Asp Asp Asp Gly Leu Pro Phe Pro Thr Asp Val Ile Gln His Arg Leu
1               5                   10                  15

Arg Gln Ile Glu Ala Gly Tyr Lys Gln Glu Val Glu Gln Leu Arg Arg
            20                  25                  30

Gln Val Arg Asp Ser Asp Glu Xaa Gly His Pro Ser Leu Leu Cys Pro
        35                  40                  45

Ser Ser Arg Ala Pro Met Asp Tyr Glu Asp Asp Phe Thr Cys Leu Lys
    50                  55                  60

Glu Ser Asp Gly Ser Asp Thr Glu Asp Phe Gly Ser Asp His Ser Glu
65                  70                  75                  80

Asp Cys Leu Ser Glu Ala Ser Trp Glu Pro Val Asp Lys Lys Glu Thr
                85                  90                  95

Glu Val Thr Arg Trp Val Pro Asp His Met Ala Ser His Cys Tyr Asn
            100                 105                 110

Cys Asp Cys Glu Phe Trp Leu Ala Lys Arg Arg His His Cys Arg Asn
        115                 120                 125

Cys Gly Asn Val Phe Cys Ala Gly Cys Cys His Leu Lys Leu Pro Ile
    130                 135                 140

Pro Asp Gln Gln Leu Tyr Asp Pro Val Leu Val Cys Asn Ser Cys Tyr

145                 150                 155                 160

Xaa Thr His Ser Ser Leu Ser Cys Gln Gly Thr His Glu Pro Thr Ala
                165                 170                 175

Glu Glu Thr His Cys Tyr Ser Phe Gln Leu Asn Ala Gly Glu Lys Pro
            180                 185                 190

Val Gln Phe
        195


<210> 174
<211> 28
<212> PRT
<213> Homo sapiens

<400> 174
Ser Glu Ala Ser Trp Glu Pro Val Asp Lys Lys Glu Thr Glu Val Thr
1               5                   10                  15

Arg Trp Val Pro Asp His Met Ala Ser His Cys Tyr
            20                  25


<210> 175
<211> 10
<212> PRT
<213> Homo sapiens

<400> 175
His His Cys Arg Asn Cys Gly Asn Val Phe
1               5                   10


<210> 176
<211> 14
<212> PRT
<213> Homo sapiens

<400> 176
Arg Leu Arg Gln Ile Glu Ala Gly Tyr Lys Gln Glu Val Glu
1               5                   10


<210> 177
<211> 87
<212> PRT
<213> Homo sapiens

<400> 177

<210> 178
<211> 30
<212> PRT
<213> Homo sapiens

<400> 178
Val Pro Gln Val Gly Gly Leu Thr Asn Ala Gln His Asp Thr Trp Leu
1               5                   10                  15

Ile Phe Val Phe Cys Val Cys Val Cys Glu Pro Leu Arg Arg
            20                  25                  30

## Description

### Field of the Invention

This invention relates to newly identified polynucleotides and the polypeptides encoded by these polynucleotides, uses of such polynucleotides and polypeptides, and their production.

### Background of the Invention

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### Summary of the Invention

The present invention relates to novel polynucleotides and the encoded polypeptides. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of the polypeptides.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

As used herein , a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCI; 0.2M NaH₂PO₄; 0.02M EDTA, pH 7.4), 0.5% SDS, 30% formamide, 100 ug/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone).

The polynucleotide of the present invention can be composed of any polyribonucleotide or polydeoxribonucleotide, which may be unmodified RNA or DNA or modified RNA or DNA. For example, polynucleotides can be composed of single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

### Polynucleotides and Polypeptides of the Invention

### FEATURES OF PROTEIN ENCODED BY GENE NO: 1

Preferred polypeptides encoded by this gene comprise the following amino acid sequence:
IRHELGCSWRFRAVKAASAQGLFLSAPGPAARRCHGVVRCFSTCRALTA RCTGRVPWEACLYSSEPPLTETVARSVSWTCELALTCYAPRALSGAPVLCRHD V (SEQ ID NO:155). Also provided are polynucleotides encoding such polypeptides.

This gene is expressed in human substantia nigra tissue.

Therefore, polynucleotides and polypeptides of the invention are useful as reagents for differential identification of the tissue(s) or cell type(s) present in a biological sample and for diagnosis of diseases and conditions which include, but are not limited to, neurological disorders or abnormalities. Similarly, polypeptides and antibodies directed to these polypeptides are useful in providing immunological probes for differential identification of the tissue(s) or cell type(s). For a number of disorders of the above tissues or cells, particularly of the neurological systems, expression of this gene at significantly higher or lower levels may be routinely detected in certain tissues or cell types (e.g., brain and of the tissue of the nervous system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:83 as residues: Pro-30 to Leu-35.

The tissue distribution indicates that the protein products of this gene are useful for diagnosis and treatment of neurological disorders and abnormalities. Many polynucleotide sequences, such as EST sequences, are publicly available and accessible through sequence databases. Some of these sequences are related to SEQ ID NO:11 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 544 of SEQ ID NO:11, b is an integer of 15 to 558, where both a and b correspond to the positions of nucleotide residues shown in SEQ ID NO: 11, and where the b is greater than or equal to a + 14.

### FEATURES OF PROTEIN ENCODED BY GENE NO: 2

This gene is expressed primarily in breast and testes.

Therefore, polynucleotides and polypeptides of the invention are useful as reagents for differential identification of the tissue(s) or cell type(s) present in a biological sample and for diagnosis of diseases and conditions which include, but are not limited to, tumors, particularly those of the breast or testes. Similarly, polypeptides and antibodies directed to these polypeptides are useful in providing immunological probes for differential identification of the tissue(s) or cell type(s). For a number of disorders of the above tissues or cells, particularly of the breast and testes, expression of this gene at significantly higher or lower levels may be routinely detected in certain tissues or cell types (e.g., reproductive tissue, and cancerous and wounded tissues) or bodily fluids (e.g., breast milk, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:84 as residues: Ser-32 to His-37.

The tissue distribution indicates that the protein products of this gene are useful for diagnosis and treatment of disorders or abnormalities of breast and testes such as tumors of those tissues. Many polynucleotide sequences, such as EST sequences, are publicly available and accessible through sequence databases. Some of these sequences are related to SEQ ID NO: 12 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 701 of SEQ ID NO: 12, b is an integer of 15 to 715, where both a and b correspond to the positions of nucleotide residues shown in SEQ ID NO:12, and where the b is greater than or equal to a + 14.

### FEATURES OF PROTEIN ENCODED BY GENE NO: 3

This gene is expressed in apoptotic T cells.

Therefore, polynucleotides and polypeptides of the invention are useful as reagents for differential identification of the tissue(s) or cell type(s) present in a biological sample and for diagnosis of diseases and conditions which include, but are not limited to, disorder or abnormalities of T cells. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissues of the immune system, developmental tissue, and cancerous and wounded tissues) or bodily fluids (e.g., lymph seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:85 as residues: Met-1 to Glu-6, Leu-39 to Lys-46.

The tissue distribution indicates that polynucleotides and polypeptides corresponding to this gene are useful for diagnosis and treatment of disorders in T cells and other immune system disorders such as inflammation. Many polynucleotide sequences, such as EST sequences, are publicly available and accessible through sequence databases. Some of these sequences are related to SEQ ID NO: 13 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 824 of SEQ ID NO:13, b is an integer of 15 to 838, where both a and b correspond to the positions of nucleotide residues shown in SEQ ID NO:13, and where the b is greater than or equal to a + 14.

### FEATURES OF PROTEIN ENCODED BY GENE NO: 4

The translation product of this gene shares sequence homology with ubiquitin-conjugating enzyme (UCE) involved in selective protein degradation. Based on the sequence similarity, the translation product of this gene is expected to share biological activities with UCE proteins. Such activities are known in the art and described elsewhere herein. Preferred polypeptides encoded by this gene comprise the following amino acid sequence:
FLAIHFPTDFPLKPPKVAFTRMYFPNSNSNGSTCLDILWSQWSPAL (SEQ ID NO: 156). Also provided are polynucleotides encoding such polypeptides.

This gene is expressed primarily in testes.

Therefore, polynucleotides and polypeptides of the invention are useful as reagents for differential identification of the tissue(s) or cell type(s) present in a biological sample and for diagnosis of diseases and conditions which include, but are not limited to, disorders in testes, particularly cell cycle disorders, (e.g. testes tumor). Similarly, polypeptides and antibodies directed to these polypeptides are useful in providing immunological probes for differential identification of the tissue(s) or cell type(s). For a number of disorders of the above tissues or cells, particularly of the testes and reproductive systems, expression of this gene at significantly higher or lower levels may be routinely detected in certain tissues or cell types (e.g., reproductive tissues, and cancerous and wounded tissues) or bodily fluids (e.g., seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to ubiquitin-conjugating enzyme indicates that the protein product of this gene is useful for diagnosis and treatment of disorders in testes and reproductive system such as tumors, as well as the treatment of tumors of other origins. Many polynucleotide sequences, such as EST sequences, are publicly available and accessible through sequence databases. Some of these sequences are related to SEQ ID NO:14 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 499 of SEQ ID NO: 14, b is an integer of 15 to 513, where both a and b correspond to the positions of nucleotide residues shown in SEQ ID NO: 14, and where the b is greater than or equal to a + 14.

### FEATURES OF PROTEIN ENCODED BY GENE NO: 5

This gene is expressed primarily in testes.

Therefore, polynucleotides and polypeptides of the invention are useful as reagents for differential identification of the tissue(s) or cell type(s) present in a biological sample and for diagnosis of diseases and conditions which include, but are not limited to, disorders and abnormalities in the testes. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., reproductive tissue, and cancerous and wounded tissues) or bodily fluids (e.g., seminal fluid, serum, plasma, urine, synovial fluid and spinal fluid) or another tissue or cell sample taken from an individual having such a disorder, relative to the standard gene expression level, (i.e., the expression level in healthy tissue or bodily fluid from an individual not having the disorder). Preferred epitopes include those comprising a sequence shown in SEQ ID NO:87 as residues: Ser-22 to Thr-32, Pro-37 to Ser-42.

The tissue distribution indicates that polynucleotides and polypeptides corresponding to this gene are useful for diagnosis and treatment of disorders in testes and the reproductive system. Many polynucleotide sequences, such as EST sequences, are publicly available and accessible through sequence databases. Some of these sequences are related to SEQ ID NO:15 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 698 of SEQ ID NO: 15, b is an integer of 15 to 712, where both a and b correspond to the positions of nucleotide residues shown in SEQ ID NO:15, and where the b is greater than or equal to a + 14.

### FEATURES OF PROTEIN ENCODED BY GENE NO: 6

This gene is expressed primarily in thymus, activated monocytes and spleen.

Therefore, polynucleotides and polypeptides of the invention are useful as reagents for differential identification of the tissue(s) or cell type(s) present in a biological sample and for diagnosis of diseases and conditions which include, but are not limited to, disorders and abnormalities of immune function and hematopoiesis, e.g. leukemia. Similarly, polypeptides and antibodies directed to these polypeptides are useful in providing immunological probes for differential identification of the tissue(s) or cell type(s). For a number of disorders of the above tissues or cells, particularly of the immune and hematopoietic systems, expression of this gene at significantly higher or lower levels may be routinely detected in certain tissues or cell types (e.g., endocrine tissue cell and tissue of the immune system, and haematopoietic tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that the protein products of this gene are useful for diagnosis and treatment of immunological and hematopoietic disorders such as leukemia. Many polynucleotide sequences, such as EST sequences, are publicly available and accessible through sequence databases. Some of these sequences are related to SEQ ID NO: 16 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 638 of SEQ ID NO: 16, b is an integer of 15 to 652, where both a and b correspond to the positions of nucleotide residues shown in SEQ ID NO: 16, and where the b is greater than or equal to a + 14.

### FEATURES OF PROTEIN ENCODED BY GENE NO: 7

This gene is expressed in T cells.

Therefore, polynucleotides and polypeptides of the invention are useful as reagents for differential identification of the tissue(s) or cell type(s) present in a biological sample and for diagnosis of diseases and conditions which include, but are not limited to, T cell dysfunction. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of disorders in T cells and immune systems. Many polynucleotide sequences, such as EST sequences, are publicly available and accessible through sequence databases. Some of these sequences are related to SEQ ID NO:17 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 728 of SEQ ID NO: 17, b is an integer of 15 to 742, where both a and b correspond to the positions of nucleotide residues shown in SEQ ID NO: 17, and where the b is greater than or equal to a + 14.

### FEATURES OF PROTEIN ENCODED BY GENE NO: 8

This gene is expressed primarily in pleural cancer and to a less extent in T cells.

Therefore, polynucleotides and polypeptides of the invention are useful as reagents for differential identification of the tissue(s) or cell type(s) present in a biological sample and for diagnosis of diseases and conditions which include, but are not limited to, pleural cancer. Similarly, polypeptides and antibodies directed to these polypeptides are useful in providing immunological probes for differential identification of the tissue(s) or cell type(s). For a number of disorders of the above tissues or cells, particularly of the pleural system, expression of this gene at significantly higher or lower levels may be routinely detected in certain tissues or cell types (e.g., cells and tissue of the immune system, pleural tissue and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:90 as residues: Ser-30 to Tyr-37.

The tissue distribution indicates that polynucleotides and polypeptides corresponding to this gene are useful for diagnosis and treatment of pleural cancer. Many polynucleotide sequences, such as EST sequences, are publicly available and accessible through sequence databases. Some of these sequences are related to SEQ ID NO: 18 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1205 of SEQ ID NO: 18, b is an integer of 15 to 1219, where both a and b correspond to the positions of nucleotide residues shown in SEQ ID NO:18, and where the b is greater than or equal to a + 14.

### FEATURES OF PROTEIN ENCODED BY GENE NO: 9

This gene is expressed in endothelial cells that shares the same origin as hematopoietic cells and in spleen and liver which are hematopoietic tissues.

Therefore, polynucleotides and polypeptides of the invention are useful as reagents for differential identification of the tissue(s) or cell type(s) present in a biological sample and for diagnosis of diseases and conditions which include, but are not limited to, disorders of endothelial cells or hematopoiesis. Similarly, polypeptides and antibodies directed to these polypeptides are useful in providing immunological probes for differential identification of the tissue(s) or cell type(s). For a number of disorders of the above tissues or cells, particularly of the endothelial and hematopoietic systems, expression of this gene at significantly higher or lower levels may be routinely detected in certain tissues or cell types (e.g., endothelial tissue, haematopoietic cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of disorders in endothelial or hematopoietic systems. Many polynucleotide sequences, such as EST sequences, are publicly available and accessible through sequence databases. Some of these sequences are related to SEQ ID NO: 19 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 860 of SEQ ID NO: 19, b is an integer of 15 to 874, where both a and b correspond to the positions of nucleotide residues shown in SEQ ID NO: 19, and where the b is greater than or equal to a + 14.

### FEATURES OF PROTEIN ENCODED BY GENE NO: 10

This gene is expressed primarily in breast lymph node and to a lesser extent in melanocytes.

Therefore, polynucleotides and polypeptides of the invention are useful as reagents for differential identification of the tissue(s) or cell type(s) present in a biological sample and for diagnosis of diseases and conditions which include, but are not limited to, metastatic melanoma. Similarly, polypeptides and antibodies directed to these polypeptides are useful in providing immunological probes for differential identification of the tissue(s) or cell type(s). For a number of disorders of the above tissues or cells, particularly of the lymphatic system, expression of this gene at significantly higher or lower levels may be routinely detected in certain tissues or cell types (e.g., reproductive tissue, cells and tissue of the immune system, cancerous and wounded tissues) or bodily fluids (e.g., lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an . individual not having the disorder.

The tissue distribution of this gene in melanocytes and lymph node indicates that the protein product of this gene is useful for the diagnosis and treatment of metastatic melanoma involving lymphatic tissues. Many polynucleotide sequences, such as EST sequences, are publicly available and accessible through sequence databases. Some of these sequences are related to SEQ ID NO:20 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 450 of SEQ ID NO:20, b is an integer of 15 to 464, where both a and b correspond to the positions of nucleotide residues shown in SEQ ID NO:20, and where the b is greater than or equal to a + 14.

### FEATURES OF PROTEIN ENCODED BY GENE NO: 11

This gene is believed to reside on chromosome 2. Therefore, this gene is useful in linkage analysis as a marker for chromosome 2.

This gene is expressed primarily in infant brain and to a lesser extent in fetal liver/spleen.

Therefore, polynucleotides and polypeptides of the invention are useful as reagents for differential identification of the tissue(s) or cell type(s) present in a biological sample and for diagnosis of diseases and conditions which include, but are not limited to, neurological disorders. Similarly, polypeptides and antibodies directed to these polypeptides are useful in providing immunological probes for differential identification of the tissue(s) or cell type(s). For a number of disorders of the above tissues or cells, particularly of the central nervous system, expression of this gene at significantly higher or lower levels may be routinely detected in certain tissues or cell types (e.g., neural tissue, cells and tissue of the immune system, developing tissue, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, (i.e., the expression level in healthy tissue or bodily fluid from an individual not having the disorder). Preferred epitopes include those comprising a sequence shown in SEQ ID NO:93 as residues: Tyr-59 to Gln-68, His-84 to Leu-90, Ser-105 to Asn-110, Leu-112 to Pro-118.

The tissue distribution indicates that polynucleotides and polypeptides corresponding to this gene are useful for diagnosis and treatment of neurological disorders. Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 623 of SEQ ID NO:21, b is an integer of 15 to 637, where both a and b correspond to the positions of nucleotide residues shown in SEQ ID NO:21, and where the b is greater than or equal to a + 14.

### FEATURES OF PROTEIN ENCODED BY GENE NO: 12

This gene is expressed primarily in adipose tissue.

Therefore, polynucleotides and polypeptides of the invention are useful as reagents for differential identification of the tissue(s) or cell type(s) present in a biological sample and for diagnosis of diseases and conditions which include, but are not limited to, metabolic disorders. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g., adipose tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for regulation of fat metabolism and treatment of obesity. Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 738 of SEQ ID NO:22, b is an integer of 15 to 752, where both a and b correspond to the positions of nucleotide residues shown in SEQ ID NO:22, and where the b is greater than or equal to a + 14.

### FEATURES OF PROTEIN ENCODED BY GENE NO: 13

The translation product of this gene shares sequence homology with NADH:ubiquinone oxidoreductase, the first enzyme in the respiratory electron transport chain of mitochondria.

This gene is expressed primarily in HSC172 cells and to a lesser extent in pineal gland.

Therefore, polynucleotides and polypeptides of the invention are useful as reagents for differential identification of the tissue(s) or cell type(s) present in a biological sample and for diagnosis of diseases and conditions which include, but are not limited to, jet lag. Similarly, polypeptides and antibodies directed to these polypeptides are useful in providing immunological probes for differential identification of the tissue(s) or cell type(s). For a number of disorders of the above tissues or cells, particularly of the endocrine system, expression of this gene at significantly higher or lower levels may be routinely detected in certain tissues or cell types (e.g., endocrine tissue, metabolic tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:95 as residues: Thr-30 to Val-38, Glu-49 to Ile-54.

The tissue distribution and homology to NADH:ubiquinone oxidoreductase indicates that polynucleotides and polypeptides corresponding to this gene are useful for minimizing the negative effects of travel across time zones by altering the body's circadean clock. Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 478 of SEQ ID NO:23, b is an integer of 15 to 492, where both a and b correspond to the positions of nucleotide residues shown in SEQ ID NO:23, and where the b is greater than or equal to a + 14.

### FEATURES OF PROTEIN ENCODED BY GENE NO: 14

This gene is expressed primarily in synovial IL-1/TNF stimulated cells.

Therefore, polynucleotides and polypeptides of the invention are useful as reagents for differential identification of the tissue(s) or cell type(s) present in a biological sample and for diagnosis of diseases and conditions which include, but are not limited to, arthritis. Similarly, polypeptides and antibodies directed to these polypeptides are useful in providing immunological probes for differential identification of the tissue(s) or cell type(s). For a number of disorders of the above tissues or cells, particularly of the skeletal system, expression of this gene at significantly higher or lower levels may be routinely detected in certain tissues or cell types (e.g., skeletal tissue and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an . individual not having the disorder.

The tissue distribution indicates that polynucleotides and polypeptides corresponding to this gene are useful for treatment of degenerative conditions of joints, including arthritis. Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 518 of SEQ ID NO:24, b is an integer of 15 to 532, where both a and b correspond to the positions of nucleotide residues shown in SEQ ID NO:24, and where the b is greater than or equal to a + 14.

### FEATURES OF PROTEIN ENCODED BY GENE NO: 15

This gene is expressed primarily in 12 Week Old Early Stage Human.

Therefore, polynucleotides and polypeptides of the invention are useful as reagents for differential identification of the tissue(s) or cell type(s) present in a biological sample and for diagnosis of diseases and conditions which include, but are not limited to, developmental disorders. Similarly, polypeptides and antibodies directed to these polypeptides are useful in providing immunological probes for differential identification of the tissue(s) or cell type(s). For a number of disorders of the above tissues or cells, particularly of the fetal systems, expression of this gene at significantly higher or lower levels may be routinely detected in certain tissues or cell types (e.g., developing tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of some developmental disorders. Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 906 of SEQ ID NO:25, b is an integer of 15 to 920, where both a and b correspond to the positions of nucleotide residues shown in SEQ ID NO:25, and where the b is greater than or equal to a + 14.

### FEATURES OF PROTEIN ENCODED BY GENE NO: 16

This gene is expressed primarily in thymus and to a lesser extent in neutrophils.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an. individual not having the disorder.

The tissue distribution indicates that polynucleotides and polypeptides corresponding to this gene are useful for diagnosis and treatment of some immune disorders including lupus and other disorders involving thymic dysfunction. Many polynucleotide sequences, such as EST sequences, are publicly available and accessible through sequence databases. Some of these sequences are related to SEQ ID NO:26 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 903 of SEQ ID NO:26, b is an integer of 15 to 917, where both a and b correspond to the positions of nucleotide residues shown in SEQ ID NO:26, and where the b is greater than or equal to a + 14.

### FEATURES OF PROTEIN ENCODED BY GENE NO: 17

This gene is expressed primarily in fibrosarcoma and to a lesser extent in IL1 and IPS induced neutrophils.

Therefore, polynucleotides and polypeptides of the invention are useful as reagents for differential identification of the tissue(s) or cell type(s) present in a biological sample and for diagnosis of diseases and conditions which include, but are not limited to, fibrosarcoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., connective tissue, and cells and tissue of the immune system, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:99 as residues: Gly-6 to Pro-11.

The tissue distribution indicates that polynucleotides and polypeptides corresponding to this gene are useful for diagnosis and treatment of fibrosarcoma or other immune disorders. Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 648 of SEQ ID NO:27, b is an integer of 15 to 662, where both a and b correspond to the positions of nucleotide residues shown in SEQ ID NO:27, and where the b is greater than or equal to a + 14.

### FEATURES OF PROTEIN ENCODED BY GENE NO: 18

Preferred polypeptides comprise the following amino acid sequence: MLLTPHFNVANPQNLLAGLWLENEHSFTLMAPERARTHHCQPEERKVLFCLFP IVPNSQAQVQPPQMPPFCCAAAKEKTQEEQLQEPLGSQCPDTCPNSLC (SEQ ID NO: 157). Polynucleotides encoding such polypeptides are also provided.

This gene is expressed primarily in jurkat T-Cells in S phase, and to a lesser extent in IL-1 and LPS induced neutrophils.

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., ells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:100 as residues: Lys-97 to Gln-106, Gln-112 to Pro-118, Pro-123 to Lys-130, Arg-153 to Gly-158.

The tissue distribution indicates that polynucleotides and polypeptides corresponding to this gene are useful for diagnosis and treatment of immune disorders related to jurkat T-cells and induced neutrophils. Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 685 of SEQ ID NO:28, b is an integer of 15 to 699, where both a and b correspond to the positions of nucleotide residues shown in SEQ ID NO:28, and where the b is greater than or equal to a + 14.

### FEATURES OF PROTEIN ENCODED BY GENE NO: 19

The translation product of this gene shares sequence homology with FK506-binding protein FKBP-12 and FKBP13, members of a family of proteins which bind the immunosuppressant drugs FK506 and rapamycin. The homology to FK506 binding proteins indicates that the protein encoded by this gene has similar activity to the known FK506 BP family members. Such activity may be assayed according to methods known in the art and described elsewhere herein. Preferred polypeptides encoded by this gene comprise the following amino acid sequence:
MRLFLWNAVLTLFVTSLIGALIPEPEVK IEVLQKPFICHRKTKGGDLMLVHYEGYLEKDGSLFHSTHKHNNGQPIWFTLGI LEALKGWDQGLKGMCVGEKRKLIIPPALGYGKEGKGKIPPESTLIFNIDLLEIR NGPRSHESFQEMDLNDDWKLSKDEVKAYLKKEFEKHGAVVNESHHDALVEDI FDKEDEDKDGFISAREFTYKHDEL (SEQ ID NO: 158) or comprise a mature form of the foregoing polypeptide having the following amino acid sequence:
EVKIEVLQKPFICHRKTKGGDLMLVHYEGYLEKDGSLFHSTHKHNNGQPIWF TLGILEALKGWDQGLKGMCVGEKRKLIIPPALGYGKEGKGKIPPESTLIFNIDL LEIRNGPRSHESFQEMDLNDDWKLSKDEVKAYLKKEFEKHGAVVNESHHDAL VEDIFDKEDEDKDGFISAREFTYKHDEL (SEQ ID NO: 159). Polynucleotides encoding these polypeptides are also provided. Particularly preferred is the following polynucleotide sequence:
ATGAGGCTTTTCTTGTGGAACGCGGTCTTGACTCTGTTCGTCACTTCT TTGATTGGGGCTTTGATCCCTGAACCAGAAGTGAAAATTGAAGTTCTCCAGA AGCCATTCATCTGCCATCGCAAGACCAAAGGAGGGGATTTGATGTTGGTCC ACTATGAAGGCTACTTAGAAAAGGACGGCTCCTTATTTCACTCCACTCACAA ACATAACAATGGTCAGCCCATTTGGTTTACCCTGGGCATCCTGGAGGCTCTC AAAGGTTGGGACCAGGGCTTGAAAGGAATGTGTGTAGGAGAGAAGAGAAA GCTCATCATTCCTCCTGCTCTGGGCTATGGAAAAGAAGGAAAAGGTAAAATT CCCCCAGAAAGTACACTGATATTTAATATTGATCTCCTGGAGATTCGAAATG GACCAAGATCCCATGAATCATTCCAAGAAATGGATCTTAATGATGACTGGAA ACTCTCTAAAGATGAGGTTAAAGCATATTTAAAGAAGGAGTTTGAAAAACAT GGTGCGGTGGTGAATGAAAGTCATCATGATGCTTTGGTGGAGGATATTTTT GATAAAGAAGATGAAGACAAAGATGGGTTTATATCTGCCAGAGAATTTACAT ATAAACACGATGAG TTA (SEQ ID NO:160), and the portion of it, nucleotide residues 76 to the 3' end, which encode the mature form shown above.

This gene is expressed primarily in fetal heart, ovary, and thymus.

Therefore, polynucleotides and polypeptides of the invention are useful as reagents for differential identification of the tissue(s) or cell type(s) present in a biological sample and for diagnosis of diseases and conditions which include, but are not limited to, immune dysfunction such as autoimmune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to FK506-binding protein FKBP-12 and FKBP13 indicates that polynucleotides and polypeptides corresponding to this gene are useful for identifying immunosuppressant drugs, and may be used in combination with immunosuppressant drugs for therapeutic purposes in the treatment of autoimmune diseases and organ/tissue transplant rejection. Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1623 of SEQ ID NO:29, b is an integer of 15 to 1637, where both a and b correspond to the positions of nucleotide residues shown in SEQ ID NO:29, and where the b is greater than or equal to a + 14.

### FEATURES OF PROTEIN ENCODED BY GENE NO: 20

The protein product of this gene is believed to be the human homologue of yeast GAA1P which anchors proteins to cell surface membranes by glycosylphosphatidylinositols (GPIs). See, for example, Yeast Gaalp is required for attachment of a completed GPI anchor onto proteins (J Cell Biol. 1995 May; 129(3): 629-639. PMID: 7730400; UI: 95247814).

This gene is expressed primarily in primary breast cancer and to a lesser extent in amniotic cells.

Therefore, polynucleotides and polypeptides of the invention are useful as reagents for differential identification of the tissue(s) or cell type(s) present in a biological sample and for diagnosis of diseases and conditions which include, but are not limited to, breast cancer. Similarly, polypeptides and antibodies directed to these polypeptides are useful in providing immunological probes for differential identification of the tissue(s) or cell type(s). For a number of disorders of the above tissues or cells, particularly of the metabolic system, expression of this gene at significantly higher or lower levels may be routinely detected in certain tissues or cell types (e.g., reproductive tissue, amniotic cells and tissue and cancerous and wounded tissues) or bodily fluids (e.g., breast milk, lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:102 as residues: Pro-113 to Met-123.

The tissue distribution and similarity to GAA1P indicates that polynucleotides and polypeptides corresponding to this gene are useful for diagnosis and treatment of primary breast cancers. Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2128 of SEQ ID NO:30, b is an integer of 15 to 2142, where both a and b correspond to the positions of nucleotide residues shown in SEQ ID NO:30, and where the b is greater than or equal to a + 14.

### FEATURES OF PROTEIN ENCODED BY GENE NO: 21

This gene is expressed primarily in activated T-cells and to a lesser extent in prostate cancer and HSC172 cells.

Therefore, polynucleotides and polypeptides of the invention are useful as reagents for differential identification of the tissue(s) or cell type(s) present in a biological sample and for diagnosis of diseases and conditions which include, but are not limited to, immune dysfunction and/or prostate cancer. Similarly, polypeptides and antibodies directed to these polypeptides are useful in providing immunological probes for differential identification of the tissue(s) or cell type(s). For a number of disorders of the above tissues or cells, particularly of the immune and metabolic systems, expression of this gene at significantly higher or lower levels may be routinely detected in certain tissues or cell types (e.g., cells and tissue of the immune system, reproductive tissue, and cancerous and wounded tissues) or bodily fluids (e.g., lymph, seminal fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 103 as residues: Ala-17 to Pro-26, Phe-92 to Tyr-97, Gly-104 to Glu-111.

The tissue distribution indicates that polynucleotides and polypeptides corresponding to this gene are useful for diagnosis and treatment of immune disorders and/or prostate cancer. Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1550 of SEQ ID NO:31, b is an integer of 15 to 1564, where both a and b correspond to the positions of nucleotide residues shown in SEQ ID NO:31, and where the b is greater than or equal to a + 14.

### FEATURES OF PROTEIN ENCODED BY GENE NO: 22

This gene maps to chromosome 12, and therefore, may be used as a marker in linkage analysis for chromosome 12.

This gene is expressed primarily in breast tissues and to a lesser extent in fetal spleen.

Therefore, polynucleotides and polypeptides of the invention are useful as reagents for differential identification of the tissue(s) or cell type(s) present in a biological sample and for diagnosis of diseases and conditions which include, but are not limited to, breast cancer, lactation deficiencies, and other breast related diseases, including breast cancer. Similarly, polypeptides and antibodies directed to these polypeptides are useful in providing immunological probes for differential identification of the tissue(s) or cell type(s). For a number of disorders of the above tissues or cells, particularly of the haemolymphoid and reproductive systems, expression of this gene at significantly higher or lower levels may be routinely detected in certain tissues or cell types (e.g., breast tissue, cells and tissue of the immune system, and haemolymphoid tissue, cancerous and wounded tissues) or bodily fluids (e.g., lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in tumors of breast origins indicates that polynucleotides and polypeptides corresponding to this gene are useful for diagnosis and intervention of these tumors, in addition to other tumors where expression has been indicated. Protein, as well as, antibodies directed against the protein may show utility as a tissue-specific marker and/or immunotherapy target for the above listed tissues. Alternatively, The tissue distribution indicates that polynucleotides and polypeptides corresponding to this gene are . useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1617 of SEQ ID NO:32, b is an integer of 15 to 1631, where both a and b correspond to the positions of nucleotide residues shown in SEQ ID NO:32, and where the b is greater than or equal to a + 14.

### FEATURES OF PROTEIN ENCODED BY GENE NO: 23

The translation product of this gene shares sequence homology with mutant or polymorphic BRCA1 gene which is thought to be important in the diagnosis and therapy of human breast and ovarian cancer as a predisposing gene (See Genebank Accession No T17455).This gene maps to chromosome 18, and therefore, may be used as a marker in linkage analysis for chromosome 18.

This gene is expressed primarily in primary dendritic cells.

Therefore, polynucleotides and polypeptides of the invention are useful as reagents for differential identification of the tissue(s) or cell type(s) present in a biological sample and for diagnosis of diseases and conditions which include, but are not limited to, breast, ovarian, and other reproductive related disorders and cancers. Similarly, polypeptides and antibodies directed to these polypeptides are useful in providing immunological probes for differential identification of the tissue(s) or cell type(s). For a number of disorders of the above tissues or cells, particularly of the haemolymphoid and reproductive systems, expression of this gene at significantly higher or lower levels may be routinely detected in certain tissues or cell types (e.g., reproductive tissue, pleural tissue, breast tissue, and cancerous and wounded tissues) or bodily fluids (e.g., breast milk, lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:105 as residues: Pro-13 to Lys-18, Ala-50 to Leu-58.

The homology to the mutant or polymorphic BRCA1 gene indicates that polynucleotides and polypeptides corresponding to this gene are useful for diagnosis and intervention of breast and/or ovarian tumors, in addition to other tumors where expression has been indicated. Protein, as well as, antibodies directed against the protein may show utility as a tissue-specific marker and/or immunotherapy target for the above listed tissues. Alternatively, the tissue distribution within dendritic cells indicates that the protein product of this gene is useful for the detection/treatment of neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 964 of SEQ ID NO:33, b is an integer of 15 to 978, where both a and b correspond to the positions of nucleotide residues shown in SEQ ID NO:33, and where the b is greater than or equal to a + 14.

### FEATURES OF PROTEIN ENCODED BY GENE NO: 24

The translation product of this gene shares sequence homology with smaller hepatocellular oncoprotein which is thought to be important in protein synthesis (See Genebank Accession No. R07057).One embodiment of this gene comprises polypeptides of the following amino acid sequence: LRSVVQDHPGQHGETPSLLKIQ @ (SEQ ID NO: 161). An additional embodiment is the polynucleotides encoding these polypeptides.

This gene is expressed primarily in embryonic tissues and to a lesser extent in uterine cancer.

Therefore, polynucleotides and polypeptides of the invention are useful as reagents for differential identification of the tissue(s) or cell type(s) present in a biological sample and for diagnosis of diseases and conditions which include, but are not limited to, heptocellular tumors, and other disorders characterized by proliferating and/or developing tissues. Similarly, polypeptides and antibodies directed to these polypeptides are useful in providing immunological probes for differential identification of the tissue(s) or cell type(s). For a number of disorders of the above tissues or cells, particularly of the haemolymphoid system, expression of this gene at significantly higher or lower levels may be routinely detected in certain tissues or cell types (e.g., reproductive tissue, cells and tissue of the immune system, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:106 as residues: Asn-15 to Ser-20, Ile-32 to Asn-37.

The tissue distribution combine with the homology to a conserved human hepatocellular oncoprotein indicates that polynucleotides and polypeptides corresponding to this gene are useful for the diagnosis and treatment of cancer and other proliferative disorders. Expression within embryonic tissue and other cellular sources marked by proliferating cells indicates that this protein may play a role in the regulation of cellular division. Additionally, the expression also indicates that this protein may play a role in the proliferation, differentiation, and/or survival of hematopoietic cell lineages. In such an event, this gene may be useful in the treatment of lymphoproliferative disorders, and in the maintenance and differentiation of various hematopoietic lineages from early hematopoietic stem and committed progenitor cells. Similarly, embryonic development also involves decisions involving cell differentiation and/or apoptosis in pattern formation. Thus this protein may also be involved in apoptosis or tissue differentiation and could again be useful in cancer therapy. Alternatively, the homology to a hepatocellular protein indicates that the protein product of this gene is useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma. Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 884 of SEQ ID NO:34, b is an integer of 15 to 898, where both a and b correspond to the positions of nucleotide residues shown in SEQ ID NO:34, and where the b is greater than or equal to a + 14.

### FEATURES OF PROTEIN ENCODED BY GENE NO: 25

The translation product of this gene shares sequence homology with a gene encoding for anti-heparanase activity which is thought to be important in inhibition of heparin or heparan sulphate degradation. Moreover, this gene was shown to have homology to the human 3-oxo-5-beta-steroid 4-dehydrogenase, which is known to be important in metabolism since it catalyzes the reduction of delta(4) double bonds of bile acid intermediates and steroid hormones carrying the delta(4)-3-one structure in the A/B Cis configuration (See Genebank Accession No.Z28339) One embodiment of this gene comprises polypeptides of the following amino acid sequence:
MFYNFVRQLDTVSIEHAGKSKLKMTVGTKLTSGXGPRKSSQSGRIAASITDCQ QCKA @ (SEQ ID NO:162), and/or
MEAAILPLWLLFLGPXPEVSFVPTVIFNLDFPACSILTVSSCLTKL @ (SEQ ID NO:163). An additional embodiment is the polynucleotides encoding these polypeptides.

This gene is expressed primarily in fetal tissues.

Therefore, polynucleotides and polypeptides of the invention are useful as reagents for differential identification of the tissue(s) or cell type(s) present in a biological sample and for diagnosis of diseases and conditions which include, but are not limited to; arthritis, metabolic disorders, as well as reproductive disorders. Similarly, polypeptides and antibodies directed to these polypeptides are useful in providing immunological probes for differential identification of the tissue(s) or cell type(s). For a number of disorders of the above tissues or cells, particularly of the haemolymphoid system, expression of this gene at significantly higher or lower levels may be routinely detected in certain tissues or cell types (e.g. reproductive, metabolic, cancerous and wounded tissues) or bodily fluids (e.g., bile acid, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of cancer and other proliferative disorders. Expression within embryonic tissue and other cellular sources marked by proliferating cells indicates that this protein may play a role in the regulation of cellular division. Additionally, the expression indicates that this protein may play a role in the proliferation, differentiation, and/or survival of hematopoietic cell lineages. In such an event, this gene may be useful in the treatment of lymphoproliferative disorders, and in the maintenance and differentiation of various hematopoietic lineages from early hematopoietic stem and committed progenitor cells. Similarly, embryonic development also involves decisions involving cell differentiation and/or apoptosis in pattern formation. Thus this protein may also be involved in apoptosis or tissue differentiation and could again be useful in cancer therapy. Alternatively, based upon its homology to a conserved human anti-heparanase gene, mutations of which are known to be important in the predisposition of arthritis, may suggest that this protein may also be important in the diagnosis or treatment of various autoimmune disorders such as rheumatoid arthritis, lupus, scleroderma, and dermatomyositis as well as dwarfism, spinal deformation, and specific joint abnormalities as well as chondrodysplasias i.e. spondyloepiphyseal dysplasia congenita, familial osteoarthritis, Atelosteogenesis type II, metaphyseal chondrodysplasia type Schmid. Moreover, the homology to a conserved human metabolic gene may suggest that the protein product of this gene is useful for the diagnosis, prevention, and/or treatment of various metabolic disorders such as Tay-Sachs disease, phenylkenonuria, galactosemia, porphyrias, and Hurler's syndrome. Many polynucleotide sequences, such as EST sequences, are publicly available and accessible through sequence databases. Some of these sequences are related to SEQ ID NO:35 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 740 of SEQ ID NO:35, b is an integer of 15 to 754, where both a and b correspond to the positions of nucleotide residues shown in SEQ ID NO:35, and where the b is greater than or equal to a + 14.

### FEATURES OF PROTEIN ENCODED BY GENE NO: 26

The translation product of this gene shares sequence homology with pulmonary hydrophobic surfactant-associated proteins which is thought to be important in useful for normalising pulmonary surface tension (See Genebank Accession No. N80643 ).

This gene is expressed primarily in embryonic tissues.

Therefore, polynucleotides and polypeptides of the invention are useful as reagents for differential identification of the tissue(s) or cell type(s) present in a biological sample and for diagnosis of diseases and conditions which include, but are not limited to, hyaline membrane disease, developmental and pulmonary disorders. Similarly, polypeptides and antibodies directed to these polypeptides are useful in providing immunological probes for differential identification of the tissue(s) or cell type(s). For a number of disorders of the above tissues or cells, particularly of the respiratory system, expression of this gene at significantly higher or lower levels may be routinely detected in certain tissues or cell types (e.g., pulmonary tissue, developing tissue and cancerous and wounded tissues) or bodily fluids (e.g., surfactant, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution and homology to pulmonary hydrophobic surfactant-associated proteins indicates that polynucleotides and polypeptides corresponding to this gene are useful for the treatment and diagnosis of such lung related diseases such as hyaline membrane disease which is often characteristic of premature infants - leading to significant pulmonary disorders throughout childhood. Alternatively, The tissue distribution indicates that polynucleotides and polypeptides corresponding to this gene are . useful for the diagnosis and treatment of cancer and other proliferative disorders. Expression within embryonic tissue and other cellular sources marked by proliferating cells indicates that this protein may play a role in the regulation of cellular division. Additionally, the expression indicates that this protein may play a role in the proliferation, differentiation, and/or survival of hematopoietic cell lineages. In such an event, this gene may be useful in the treatment of lymphoproliferative disorders, and in the maintenance and differentiation of various hematopoietic lineages from early hematopoietic stem and committed progenitor cells. Similarly, embryonic development also involves decisions involving cell differentiation and/or apoptosis in pattern formation. Thus this protein may also be involved in apoptosis or tissue differentiation and could again be useful in cancer therapy. Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 685 of SEQ ID NO:36, b is an integer of 15 to 699, where both a and b correspond to the positions of nucleotide residues shown in SEQ ID NO:36, and where the b is greater than or equal to a + 14.

### FEATURES OF PROTEIN ENCODED BY GENE NO: 27

This gene is expressed primarily in fetal tissue and to a lesser extent in thymus.

Therefore, polynucleotides and polypeptides of the invention are useful as reagents for differential identification of the tissue(s) or cell type(s) present in a biological sample and for diagnosis of diseases and conditions which include, but are not limited to, immunological disorders, particularly immunodeficiency. Similarly, polypeptides and antibodies directed to these polypeptides are useful in providing immunological probes for differential identification of the tissue(s) or cell type(s). For a number of disorders of the above tissues or cells, particularly of the haemolymphoid system, expression of this gene at significantly higher or lower levels may be routinely detected in certain tissues or cell types (e.g., cells and tissue of the immune system, developing tissue, and cancerous and wounded tissues) or bodily fluids (e.g., amniotic fluid, lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:109 as residues: Pro-18 to Trp-24.

The tissue distribution indicates that polynucleotides and polypeptides corresponding to this gene are useful for the diagnosis and treatment of a variety of immune system disorders. Expression of this gene product in thymus indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:37 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 957 of SEQ ID NO:37, b is an integer of 15 to 971, where both a and b correspond to the positions of nucleotide residues shown in SEQ ID NO:37, and where the b is greater than or equal to a + 14.

### FEATURES OF PROTEIN ENCODED BY GENE NO: 28

This gene is expressed primarily in small intestine and to a lesser extent in ulcerative colitis.

Therefore, polynucleotides and polypeptides of the invention are useful as reagents for differential identification of the tissue(s) or cell type(s) present in a biological sample and for diagnosis of diseases and conditions which include, but are not limited to, ulcers, acid reflux and other gastrointestinal disorders. Similarly, polypeptides and antibodies directed to these polypeptides are useful in providing immunological probes for differential identification of the tissue(s) or cell type(s). For a number of disorders of the above tissues or cells, particularly of the digestive system, expression of this gene at significantly higher or lower levels may be routinely detected in certain tissues or cell types (e.g., gastrointestinal tissue, and cancerous and wounded tissues) or bodily fluids (e.g., bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:110 as residues: Ile-2 to Ser-8, Gln-23 to Ser-31, Lys-61 to Lys-66, Lys-74 to Thr-79, Val-138 to Glu-160, Glu-178 to Thr-183.

The tissue distribution indicates that polynucleotides and polypeptides corresponding to this gene are useful for the treatment and diagnosis of digestive and gastrointestinal problems such as acid reflux or ulcers. Alternatively, the tissue distribution may suggest that the protein product of this gene is useful for the diagnosis, prevention, and/or treatment of various metabolic disorders such as Tay-Sachs disease, phenylkenonuria, galactosemia, porphyrias, and Hurler's syndrome. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 858 of SEQ ID NO:38, b is an integer of 15 to 872, where both a and b correspond to the positions of nucleotide residues shown in SEQ ID NO:38, and where the b is greater than or equal to a + 14.

### FEATURES OF PROTEIN ENCODED BY GENE NO: 29

The translation product of this gene was shown to have homology to catalase (See Genebank Accession No. pirlA550921A55092) which is known to play an integral role in the oxidative prophylaxis in eukaryotic and mammalian cells. One embodiment of this gene comprises polypeptides of the following amino acid sequence:
NHGHSCFLCEIVIRSQFHTTYEPEA @ (SEQ ID NO:164), and/or
SGRHRVELQLLFPLVRVNFELGVNHGHSCFLCEIVIRSQFHTTYEPEA @ (SEQ ID NO:165). An additional embodiment is the polynucleotides encoding these polypeptides. This gene maps to chromosome 3, and therefore, may be used as a marker in linkage analysis for chromosome 3.

This gene is expressed primarily in skin and to a lesser extent in human uterine cancer.

Therefore, polynucleotides and polypeptides of the invention are useful as reagents for differential identification of the tissue(s) or cell type(s) present in a biological sample and for diagnosis of diseases and conditions which include, but are not limited to, uterine cancer; fibrosis; melanoma. Similarly, polypeptides and antibodies directed to these polypeptides are useful in providing immunological probes for differential identification of the tissue(s) or cell type(s). For a number of disorders of the above tissues or cells, particularly of the epidermis and/or reproductive system, expression of this gene at significantly higher or lower levels may be routinely detected in certain tissues or cell types (e.g., integumentary tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution combined with the homology to catalase indicates that polynucleotides and polypeptides corresponding to this gene are useful for the treatment and/or diagnosis of human uterine cancer and potentially, all cancers in general (particularly of the epidermis) due to the fact that diminished catalase activity has been shown to lead to significant cellular damage which could predispose cancer. Likewise, this gene and/or its protein product may be useful in the treatment and/or diagnosis of other disorders of the skin, such as fibrosis or in wound healing. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:39 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 594 of SEQ ID NO:39, b is an integer of 15 to 608, where both a and b correspond to the positions of nucleotide residues shown in SEQ ID NO:39, and where the b is greater than or equal to a + 14.

### FEATURES OF PROTEIN ENCODED BY GENE NO: 30

This gene is expressed primarily in fetal liver/spleen and bone marrow stromal cells, and to a lesser extent in neural tissues (brain; spinal cord) and cancers (glioblastoma; chondrosarcoma).

Therefore, polynucleotides and polypeptides of the invention are useful as reagents for differential identification of the tissue(s) or cell type(s) present in a biological sample and for diagnosis of diseases and conditions which include, but are not limited to, cancers, particularly of glial cells and cartilage; hematopoietic and other immune disorders. Similarly, polypeptides and antibodies directed to these polypeptides are useful in providing immunological probes for differential identification of the tissue(s) or cell type(s). For a number of disorders of the above tissues or cells, particularly of the hematopoietic/immune system and CNS, expression of this gene at significantly higher or lower levels may be routinely detected in certain tissues or cell types (e.g., hematopoietic cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 112 as residues: Arg-2 to Asp-7.

The tissue distribution indicates that polynucleotides and polypeptides corresponding to this gene are useful for the expansion, proliferation, and/or differentiation of hematopoietic cells. This gene is expressed predominantly at sites of hematopoiesis, such as fetal liver and bone marrow, and thus may control the proliferation and/or differentiation of hematopoietic stem and progenitor cells. Thus it could be used for chemoprotection, or for the production of specific blood cell lineages, as well as the amplification of stem cells. Additionally, its expression in neural cells of the brain and spinal cord suggest that it may also play a role in the maintenance and differentiation of neuronal stem cells, or in the treatment of neurological disorders, such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Finally, its observed expression in various cancers suggest that it may play a role in the control of cell proliferation. Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 841 of SEQ ID NO:40, b is an integer of 15 to 855, where both a and b correspond to the positions of nucleotide residues shown in SEQ ID NO:40, and where the b is greater than or equal to a + 14.

### FEATURES OF PROTEIN ENCODED BY GENE NO: 31

This gene is expressed primarily in placenta, bone marrow, and fetal liver/spleen and to a lesser extent in brain and CNS.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; placental insufficiency; neurological disorders. Similarly, polypeptides and antibodies directed to these polypeptides are useful in providing immunological probes for differential identification of the tissue(s) or cell type(s). For a number of disorders of the above tissues or cells, particularly of the immune system and/or CNS, expression of this gene at significantly higher or lower levels may be routinely detected in certain tissues or cell types (e.g., hematopoietic cells and tissue, cells and tissue of the immune system, and neurological tissue, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 113 as residues: Arg-53 to Gln-58.

The tissue distribution indicates that polynucleotides and polypeptides corresponding to this gene are useful for the proliferation; maintenance, and/or differentiation of hematopoietic cells. In addition, it may be involved in the maintenance and establishment of the vasculature, and may play a role in the regulation of angiogenesis. Thus, it may play a role in the establishment and/or maintenance of tumors. Expression in the CNS indicates that this gene product may also be useful in the treatment of neurodegenerative disorders, such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1028 of SEQ ID NO:41, b is an integer of 15 to 1042, where both a and b correspond to the positions of nucleotide residues shown in SEQ ID NO:41, and where the b is greater than or equal to a + 14.

### FEATURES OF PROTEIN ENCODED BY GENE NO: 32

The translation product of this gene was shown to have homology to the ras-related protein RABB from Dictyostelium discoideum (See Genebank Accession No.P34142). Members of the Ras family of proteins are known to be essential to normal cell cycle control and mutations of which have been shown to lead to the predisposition of cancer.

This gene is expressed primarily in Jurkat T cells.

Therefore, polynucleotides and polypeptides of the invention are useful as reagents for differential identification of the tissue(s) or cell type(s) present in a biological sample and for diagnosis of diseases and conditions which include, but are not limited to, T cell lymphomas; defects in immune surveillance and/or T cell activation. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution combined with its homology to a Ras-related protein indicates that polynucleotides and polypeptides corresponding to this gene are useful for the treatment of defects in immune surveillance and cancer. Expression of this gene product in T cells indicates that it represents a secreted protein that may regulate T cells in an autocrine fashion, thereby impacting on their ability to recognize antigen and become activated, or may be involved in immune modulation. Likewise, this may represent a secreted protein made by T cells that affects other hematopoietic cells and regulates their proliferation and/or differentiation.Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:42 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 688 of SEQ ID NO:42, b is an integer of 15 to 702, where both a and b correspond to the positions of nucleotide residues shown in SEQ ID NO:42, and where the b is greater than or equal to a + 14.

### FEATURES OF PROTEIN ENCODED BY GENE NO: 33

The translation product of this gene shares sequence homology with a known human spliceosome associated protein (See Genebank Accession No. AA523942) as well as a yeast protein (ORF YBR173c [Saccharomyces cerevisiae]).Preferred polypeptides comprise the following amino acid sequence:
MNARGLGSELKDSIPVTELSASGPFES HDLLRKGFSCVKNELLPSHPLELSEKNFQLNQDKMNFSTLRNIQGLFAPLKLQ MEFKAVQQVQRLPFLSSSNLSLDVLRGNDETIGFEDILNDPSQSEVMGEPHLMV EYKLGLL (SEQ ID NO:166). Also preferred are the polynucleotides encoding these polypeptides.

This gene is expressed primarily in bone marrow and stromal cells, and to a lesser extent in T cells and peripheral blood cells.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; leukemias; defects in immune surveillance; defects in T cell activation; inflammation; bacterial infections, and other disorders characterized by immunodeficiency. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:115 as residues: Lys-16 to Thr-24.

The tissue distribution indicates that polynucleotides and polypeptides corresponding to this gene are useful for the proliferation, differentiation, and/or activation of hematopoietic cell lineages. Such treatments could involve chemoprotection or expansion of either progenitor cells or specific mature blood lineages. Likewise, this gene product could be involved in immune modulation, or in affecting T cell activation and antigen recognition. Alternatively, the protein product of this gene is useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above tissues. Many polynucleotide sequences, such as EST sequences, are publicly available and accessible through sequence databases. Some of these sequences are related to SEQ ID NO:43 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 628 of SEQ ID NO:43, b is an integer of 15 to 642, where both a and b correspond to the positions of nucleotide residues shown in SEQ ID NO:43, and where the b is greater than or equal to a + 14.

### FEATURES OF PROTEIN ENCODED BY GENE NO: 34

This gene maps to chromosome 4, and therefore, may be used as a marker in linkage analysis for chromosome 4.

This gene is expressed primarily in placenta and brain, and to a lesser extent in specific tumors and cancers (kidney, colorectal, colon, osteoclastoma).

Therefore, polynucleotides and polypeptides of the invention are useful as reagents for differential identification of the tissue(s) or cell type(s) present in a biological sample and for diagnosis of diseases and conditions which include, but are not limited to, tumors of the kidney, colon, and bone; neurological disorders; defects of the vasculature. Similarly, polypeptides and antibodies directed to these polypeptides are useful in providing immunological probes for differential identification of the tissue(s) or cell type(s). For a number of disorders of the above tissues or cells, particularly of the vasculature and CNS, expression of this gene at significantly higher or lower levels may be routinely detected in certain tissues or cell types (e.g., reproductive tissue, cells and tissue of the immune system, neural tissue, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the treatment of neurological disorders, such as Alzheimers or schizophrenia. Expression of this gene in the placenta may indicate a role in fetal development, or may simply be a hallmark of expression in the vasculature. Expression of this gene product in endothelial cells may indicate secretion of the protein product into the circulation, where it may have effects on circulating blood cells, or on tissues at distant locations. At such sites, it may control cellular proliferation and/or differentiation. In addition, expression in a variety of tumors indicates that this gene product may play a role in cellular proliferation. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:44 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1205 of SEQ ID NO:44, b is an integer of 15 to 1219, where both a and b correspond to the positions of nucleotide residues shown in SEQ ID NO:44, and where the b is greater than or equal to a + 14.

### FEATURES OF PROTEIN ENCODED BY GENE NO: 35

This gene is expressed primarily in macrophages, treated with GM-CSF.

Therefore, polynucleotides and polypeptides of the invention are useful as reagents for differential identification of the tissue(s) or cell type(s) present in a biological sample and for diagnosis of diseases and conditions which include, but are not limited to, immune deficiencies; susceptibility to bacterial infections; improper stimulation of lymphocyte pools. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:117 as residues: Arg-48 to Asn-56.

The tissue distribution indicates that polynucleotides and polypeptides corresponding to this gene are useful for the regulation and/or stimulation of hematopoietic cells, particularly the circulating lymphocytes. Macrophages are one of the front lines of immune defense, particularly against bacterial pathogens, and are able to secrete cytokines and proteins that affect other blood cells. Thus, this gene product may be important in regulation of the immune system, in activation of hematopoietic cells, such as T cells; and may play a role in antigen recognition. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:45 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 423 of SEQ ID NO:45, b is an integer of 15 to 437, where both a and b correspond to the positions of nucleotide residues shown in SEQ ID NO:45, and where the b is greater than or equal to a + 14.

### FEATURES OF PROTEIN ENCODED BY GENE NO: 36

This gene is expressed primarily in endothelial cells.

Therefore, polynucleotides and polypeptides of the invention are useful as reagents for differential identification of the tissue(s) or cell type(s) present in a biological sample and for diagnosis of diseases and conditions which include, but are not limited to, inflammation and circulatory system disorders. Similarly, polypeptides and antibodies directed to these polypeptides are useful in providing immunological probes for differential identification of the tissue(s) or cell type(s). For a number of disorders of the above tissues or cells, particularly of the circulatory system, expression of this gene at significantly higher or lower levels may be routinely detected in certain tissues or cell types (e.g., endothelial cells, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of inflammatory disorders involving endothelial cells, such as sepsis, inflammatory bowel diseases, psoriasis, and rheumatoid arthritis as well as atherosclerosis, which can lead to strokes and heart attacks. Protein, as well as, antibodies directed against the protein may show utility as a tumor marker and/or immunotherapy targets for the above listed tissues Many polynucleotide sequences, such as EST sequences, are publicly available and accessible through sequence databases. Some of these sequences are related to SEQ ID NO:46 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 519 of SEQ ID NO:46, b is an integer of 15 to 533, where both a and b correspond to the positions of nucleotide residues shown in SEQ ID NO:46, and where the b is greater than or equal to a + 14.

### FEATURES OF PROTEIN ENCODED BY GENE NO: 37

The translation product of this gene was shown to have homology to the human transducin(beta)-like 1 protein (See Genebank Accession No P38262).

This gene is expressed primarily in fetal lung and to a lesser extent in adult lung and breast.

Therefore, polynucleotides and polypeptides of the invention are useful as reagents for differential identification of the tissue(s) or cell type(s) present in a biological sample and for diagnosis of diseases and conditions which include, but are not limited to, respiratory system diseases, and/or disorders. Similarly, polypeptides and antibodies directed to these polypeptides are useful in providing immunological probes for differential identification of the tissue(s) or cell type(s). For a number of disorders of the above tissues or cells, particularly of the respiratory system, expression of this gene at significantly higher or lower levels may be routinely detected in certain tissues or cell types (e.g., pulmonary tissue, and cancerous and wounded tissues) or bodily fluids (e.g., pulmonary surfactant, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and disease affecting the lung such as lung cancer, emphysema, pulmonary edema, asthma, cystic fibrosis, and pulmonary emboli. Alternatively, the protein product of this gene may be useful in the detection, treatment, and/or prevention of various inflammatory conditions afflicting endothelial tissue such as the vasculature and cardiovascular systems. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:47 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1835 of SEQ ID NO:47, b is an integer of 15 to 1849, where both a and b correspond to the positions of nucleotide residues shown in SEQ ID NO:47, and where the b is greater than or equal to a + 14.

### FEATURES OF PROTEIN ENCODED BY GENE NO: 38

This gene is expressed primarily in prostate cancer and to a lesser extent in osteoblasts.

Therefore, polynucleotides and polypeptides of the invention are useful as reagents for differential identification of the tissue(s) or cell type(s) present in a biological sample and for diagnosis of diseases and conditions which include, but are not limited to, prostate cancer, osteoporosis. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., skeletal tissue, reproductive tissue, and cancerous and wounded tissues) or bodily fluids (e.g., seminal fluid, lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in tumors of prostate origins indicates that polynucleotides and polypeptides corresponding to this gene are useful for diagnosis and intervention of these tumors, in addition to other tissues where expression has been indicated. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:48 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 912 of SEQ ID NO:48, b is an integer of 15 to 926, where both a and b correspond to the positions of nucleotide residues shown in SEQ ID NO:48, and where the b is greater than or equal to a + 14.

### FEATURES OF PROTEIN ENCODED BY GENE NO: 39

The translation product of this gene was shown to have homology to the mdkk-1 protein from Mus musculus (See Genebank Accession No. gil2736292 (AF030433)) which has been shown to be important as a possible homeobox gene inducer specific to head development. One embodiment of this gene comprises polypeptides of the following amino acid sequence: GGNKYQTIDNYQPYP @ (SEQ ID NO:167), PLLGVSATLNSVLNSNAIKN @ (SEQ ID NO:168), and/or GSAVSAAPGILYPG. An additional embodiment is the polynucleotides encoding these polypeptides (SEQ ID NO:169).

This gene is expressed primarily in placenta and to a lesser extent in smooth muscle.

Therefore, polynucleotides and polypeptides of the invention are useful as reagents for differential identification of the tissue(s) or cell type(s) present in a biological sample and for diagnosis of diseases and conditions which include, but are not limited to, developmental disorders of the fetus, and/or reproductive disorders, particularly of the female. Similarly, polypeptides and antibodies directed to these polypeptides are useful in providing immunological probes for differential identification of the tissue(s) or cell type(s). For a number of disorders of the above tissues or cells, particularly of developing tissues in the fetus, expression of this gene at significantly higher or lower levels may be routinely detected in certain tissues or cell types (e.g., reproductive tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution combined with the homology to a suspected homeobox domain inducer indicates that polynucleotides and polypeptides corresponding to this gene are useful for diagnosis and treatment of developmental disorders, particularly those involving hematopoesis and pattern formation in the embryo. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:49 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1579 of SEQ ID NO:49, b is an integer of 15 to 1593, where both a and b correspond to the positions of nucleotide residues shown in SEQ ID NO:49, and where the b is greater than or equal to a + 14.

### FEATURES OF PROTEIN ENCODED BY GENE NO: 40

When tested against sensory neuron cell lines (PC12), supernatants removed from cells containing this gene activated the early growth response gene 1 pathway (EGR1). Thus, it is likely that this gene activates neuronal cells through the Jaks-STAT signal transduction pathway. EGR1 (early growth response gene 1) is a separate signal transduction pathway from Jaks-STAT, genes containing the EGR1 promoter are induced in various tissues and cell types upon activation, leading the cells to undergo differentiation and proliferation.

This gene is expressed primarily in neutrophils and to a lesser extent in pancreatic carcinoma.

Therefore, polynucleotides and polypeptides of the invention are useful as reagents for differential identification of the tissue(s) or cell type(s) present in a biological sample and for diagnosis of diseases and conditions which include, but are not limited to, inflammation, gastrointestinal, and immune disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:122 as residues: Pro-8 to Ala-16.

The tissue distribution indicates that polynucleotides and polypeptides corresponding to this gene are useful for the diagnosis and treatment of a variety of immune system disorders. Expression of this gene product in tonsils indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, and leukemia. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:50 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 964 of SEQ ID NO:50, b is an integer of 15 to 978, where both a and b correspond to the positions of nucleotide residues shown in SEQ ID NO:50, and where the b is greater than or equal to a + 14.

### FEATURES OF PROTEIN ENCODED BY GENE NO: 41

This gene is expressed primarily in breast lymph node and to a lesser extent in anergic T-cell.

Therefore, polynucleotides and polypeptides of the invention are useful as reagents for differential identification of the tissue(s) or cell type(s) present in a biological sample and for diagnosis of diseases and conditions which include, but are not limited to, inflammation, and immunodeficiency disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:51 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 419 of SEQ ID NO:51, b is an integer of 15 to 433, where both a and b correspond to the positions of nucleotide residues shown in SEQ ID NO:51, and where the b is greater than or equal to a + 14.

### FEATURES OF PROTEIN ENCODED BY GENE NO: 42

This gene is expressed primarily in synovium.

Therefore, polynucleotides and polypeptides of the invention are useful as reagents for differential identification of the tissue(s) or cell type(s) present in a biological sample and for diagnosis of diseases and conditions which include, but are not limited to, inflammation and diseases of the joints. Similarly, polypeptides and antibodies directed to these polypeptides are useful in providing immunological probes for differential identification of the tissue(s) or cell type(s). For a number of disorders of the above tissues or cells, particularly of the immune system and connective tissue, expression of this gene at significantly higher or lower levels may be routinely detected in certain tissues or cell types (e.g., skeletal tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of inflammatory disorders, particularly those involving the joints and skeletal system, such as rheumatoid arthritis and in particular the connective tissues (e.g. trauma, tendonitis, chrondomalacia). Protein, as well as, antibodies directed against the protein may show utility as a tumor marker and/or immunotherapy targets for the above listed tissues.. Many polynucleotide sequences, such as EST sequences, are publicly available and accessible through sequence databases. Some of these sequences are related to SEQ ID NO:52 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 847 of SEQ ID NO:52, b is an integer of 15 to 861, where both a and b correspond to the positions of nucleotide residues shown in SEQ ID NO:52, and where the b is greater than or equal to a + 14.

### FEATURES OF PROTEIN ENCODED BY GENE NO: 43

This gene maps to chromosome 5, and therefore, may be used as a marker in linkage analysis for chromosome 5.

This gene is expressed primarily in synovium.

Therefore, polynucleotides and polypeptides of the invention are useful as reagents for differential identification of the tissue(s) or cell type(s) present in a biological sample and for diagnosis of diseases and conditions which include, but are not limited to, diseases of the joints and connective tissues. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., skeletal tissue, and cancerous and wounded tissues) or bodily fluids (e.g. serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for diagnosis and treatment of inflammatory joint disorders as well as suggest a role in the detection and treatment of disorders and conditions affecting the skeletal system, in particular the connective tissues (e.g. arthritis, trauma, tendonitis, chrondomalacia and inflammation). Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:53 and may have been publicly available prior to conception of the present invention. Preferably, such related polynucleotides are specifically excluded from the scope of the present invention. To list every related sequence is cumbersome. Accordingly, preferably excluded from the present invention are one of more polynucleotides comprising a nucleotide sequence described by the general formula of a-b, where a is any integer between 1 to 496 of SEQ ID NO:53, b is an integer of 15 to 510, where both a and b correspond to the positions of nucleotide residues shown in SEQ ID NO:53, and where the b is greater than or equal to a + 14.

### FEATURES OF PROTEIN ENCODED BY GENE NO: 44

This gene is expressed primarily in activated T cells.

Therefore, polynucleotides and polypeptides of the invention are useful as reagents for differential identification of the tissue(s) or cell type(s) present in a biological sample and for diagnosis of diseases and conditions which include, but are not limited to, inflammation and other immune system disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 126 as residues: Met-1 to Lys-7.

The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:54 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 295 of SEQ ID NO:54, b is an integer of 15 to 309, where both a and b correspond to the positions of nucleotide residues shown in SEQ ID NO:54, and where the b is greater than or equal to a + 14.

### FEATURES OF PROTEIN ENCODED BY GENE NO: 45

This gene is expressed primarily in placenta, liver, lung, endometrial stromal cell and embryo.

Therefore, polynucleotides and polypeptides of the invention are useful as reagents for differential identification of the tissue(s) or cell type(s) present in a biological sample and for diagnosis of diseases and conditions such as cancers, immunodeficiency and autoimmune diseases, as well as reproductive and hepatic disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., reproductive tissue, and cancerous and wounded tissues) or bodily fluids (e.g., amniotic fluid, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:55 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1571 of SEQ ID NO:55, b is an integer of 15 to 1585, where both a and b correspond to the positions of nucleotide residues shown in SEQ ID NO:55, and where the b is greater than or equal to a + 14.

### FEATURES OF PROTEIN ENCODED BY GENE NO: 46

This gene is expressed primarily in placenta, amniotic cells and adrenal gland tumor.

Therefore, polynucleotides and polypeptides of the invention are useful as reagents for differential identification of the tissue(s) or cell type(s) present in a biological sample and for diagnosis of diseases and conditions such as cancers, as well as various reproductive and endocrine disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., endocrine tissue, reproductive tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:128 as residues: Cys-52 to Val-61.

The tissue distribution within placenta and amniotic tissues indicates that polynucleotides and polypeptides corresponding to this gene useful for the treatment and diagnosis of reproductive disorders, particularly those of the female reproductive system, including cancer and tissues characteristic of the developing embryo. In addition, the tissue distribution within the adrenal gland indicates that the protein product of this gene is useful for the detection, treatment, and/or prevention of various endocrine disorders and cancers, particularly Addisonís disease, Cushingís Syndrome, and disorders and/or cancers of the pancrease (e.g. diabetes mellitus), adrenal cortex, ovaries, pituitary (e.g., hyper-, hypopituitarism), thyroid (e.g. hyper-, hypothyroidism), parathyroid (e.g. hyper-,hypoparathyroidism) , hypothallamus, and testes. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:56 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 860 of SEQ ID NO:56, b is an integer of 15 to 874, where both a and b correspond to the positions of nucleotide residues shown in SEQ ID NO:56, and where the b is greater than or equal to a + 14.

### FEATURES OF PROTEIN ENCODED BY GENE NO: 47

This gene is expressed primarily in melanocyte, melanoma, dendritic cells and fetal brain.

Therefore, polynucleotides and polypeptides of the invention are useful as reagents for differential identification of the tissue(s) or cell type(s) present in a biological sample and for diagnosis of diseases and conditions which include, but are not limited to, skin disorders, particularly melanoma, as well as neurodegenerative disorders and cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., neural tissue, integumentary tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:129 as residues: Lys-76 to Gly-81.

The tissue distribution indicates that polynucleotides and polypeptides corresponding to this gene are useful for the treatment, diagnosis, and/or prevention of various skin disorders including congenital disorders (i.e. nevi, moles, freckles, Mongolian spots, hemangiomas, port-wine syndrome), integumentary tumors (i.e. keratoses, Bowenís disease, basal cell carcinoma, squamous cell carcinoma, malignant melanoma, Pagetís disease, mycosis fungoides, and Kaposiís sarcoma), injuries and inflammation of the skin (i.e., wounds, rashes, prickly heat disorder, psoriasis, dermatitis), atherosclerosis, uticaria, eczema, photosensitivity, autoimmune disorders (i.e. lupus erythematosus, vitiligo, dermatomyositis, morphea, scleroderma, pemphigoid, and pemphigus), keloids, striae, erythema, petechiae, purpura, and xanthelasma. Moreover, such disorders may predispose increased susceptibility to viral and bacterial infections of the skin (i.e. cold sores, warts, chickenpox, molluscum contagiosum, herpes zoster, boils, cellulitis, erysipelas, impetigo, tinea, althletes foot, and ringworm). Alternatively, the tissue distribution within dendritic cells and fetal brain indicates that the protein product of this gene is useful for the detection/treatment of neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:57 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1155 of SEQ ID NO:57, b is an integer of 15 to 1169, where both a and b correspond to the positions of nucleotide residues shown in SEQ ID NO:57, and where the b is greater than or equal to a + 14.

### FEATURES OF PROTEIN ENCODED BY GENE NO: 48

This gene maps to chromosome 1, and therefore, may be used as a marker in linkage analysis for chromosome 1.

This gene is expressed primarily in ovary tumors and to a lesser extent in breast, placenta, pineal gland, infant brain, T cell and B cell lymphoma.

Therefore, polynucleotides and polypeptides of the invention are useful as reagents for differential identification of the tissue(s) or cell type(s) present in a biological sample and for diagnosis of diseases and conditions which include, but are not limited to, ovary cancer, reproductive disorders, particularly of the female reproductive system, as well as disorders of the immune system, including lymphoma and immunodeficiencies. Similarly, polypeptides and antibodies directed to these polypeptides are useful in providing immunological probes for differential identification of the tissue(s) or cell type(s). For a number of disorders of the above tissues or cells, particularly of the immune system and female reproductive system, expression of this gene at significantly higher or lower levels may be routinely detected in certain tissues or cell types (e.g., reproductive, endocrine tissue, cells and tissue of the immune system, and cancerous and wounded tissues) or bodily fluids (e.g., amniotic fluid, lymph, breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:130 as residues: Asn-33 to Lys-38.

The tissue distribution in tumors of the ovary and lymph nodes, as well as, in breast and placental tissues indicates that polynucleotides and polypeptides corresponding to this gene are useful for diagnosis and intervention of such tumors, in addition to other tumors where expression has been indicated. Protein, as well as, antibodies directed against the protein may show utility as a tissue-specific marker and/or immunotherapy target for the above listed tissues. Alternatively, The tissue distribution indicates that polynucleotides and polypeptides corresponding to this gene are useful for the diagnosis and treatment of a variety of immune system disorders. Expression of this gene product in T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Many polynucleotide sequences, such as EST sequences, are publicly available and accessible through sequence databases. Some of these sequences are related to SEQ ID NO:58 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1052 of SEQ ID NO:58, b is an integer of 15 to 1066, where both a and b correspond to the positions of nucleotide residues shown in SEQ ID NO:58, and where the b is greater than or equal to a + 14.

### FEATURES OF PROTEIN ENCODED BY GENE NO: 49

This gene is expressed primarily in breast tissue.

Therefore, polynucleotides and polypeptides of the invention are useful as reagents for differential identification of the tissue(s) or cell type(s) present in a biological sample and for diagnosis of diseases and conditions which include, but are not limited to, breast cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., breast tissue and cancerous and wounded tissues) or bodily fluids (e.g., breast milk, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 131 as residues: Ala-40 to Trp-45.

The tissue distribution in breast tissue indicates that polynucleotides and polypeptides corresponding to this gene are useful for diagnosis and intervention of tumors within this tissue. Alternatively, the expression in breast may suggest that the protein product of this gene is useful for the diagnosis, treatment, and/or prevention of various reproductive system disorders, particularly of the female reproductive system. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:59 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 758 of SEQ ID NO:59, b is an integer of 15 to 772, where both a and b correspond to the positions of nucleotide residues shown in SEQ ID NO:59, and where the b is greater than or equal to a + 14.

### FEATURES OF PROTEIN ENCODED BY GENE NO: 50

This gene is expressed primarily in fetal liver, retina and to a lesser extent in thyroid and pineal gland.

Therefore, polynucleotides and polypeptides of the invention are useful as reagents for differential identification of the tissue(s) or cell type(s) present in a biological sample and for diagnosis of diseases and conditions which include but are not limited to, hepatic and endocrine disorders. Similarly, polypeptides and antibodies directed to these polypeptides are useful in providing immunological probes for differential identification of the tissue(s) or cell type(s). For a number of disorders of the above tissues or cells, particularly of the hematopoesis and immune system, expression of this gene at significantly higher or lower levels may be routinely detected in certain tissues or cell types (e.g., liver, endocrine tissue, and cancerous and wounded tissues) or bodily fluids (e.g., bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:132 as residues: Lys-9 to Trp-14.

The tissue distribution indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma. Alternatively, expression in pineal and thyroid glands indicates that the protein product of this gene is useful for the detection, treatment, and/or prevention of various endocrine disorders and cancers, particularly Addisonís disease, Cushingís Syndrome, and disorders and/or cancers of the pancrease (e.g. diabetes mellitus), adrenal cortex, ovaries, pituitary (e.g., hyper-, hypopituitarism), thyroid (e.g. hyper-, hypothyroidism), parathyroid (e.g. hyper-, hypoparathyroidism) , hypothallamus, and testes. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:60 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1184 of SEQ ID NO:60, b is an integer of 15 to 1198, where both a and b correspond to the positions of nucleotide residues shown in SEQ ID NO:60, and where the b is greater than or equal to a + 14.

### FEATURES OF PROTEIN ENCODED BY GENE NO: 51

The translation product of this gene shares sequence homology with seizure-related gene product 6 type 2 precursor which is a novel, brain-specific, gene thought to be important in the predisposition of seizures (See Genebank Accession No.gnllPIDld1006729). One embodiment of this gene comprises polypeptides of the following amino acid sequence: AGIQHELACDNPGLPENGYQILYKRLYLPGESLT FMCYEGFELMGEVTIRCILGQPSHWNGPLPVCKVAE AAAETSLEGGN @ (SEQ ID NO:170) QPSHWNGPLPVCKVAEAAAETSLEGGN @ (SEQ ID NO:171), and/or YETGETREYEVSI (SEQ ID NO:172). An additional embodiment is the polynucleotides encoding these polypeptides.

This gene is expressed primarily in brain.

Therefore, polynucleotides and polypeptides of the invention are useful as reagents for differential identification of the tissue(s) or cell type(s) present in a biological sample and for diagnosis of diseases and conditions which include, but are not limited to, seizures and other neural and CNS disorders. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g., neural tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 133 as residues: Cys-26 to Leu-32, Thr-49 to Ile-55, Glu-57 to Glu-63.

The tissue distribution in brain combined with its homology to a putative seizure gene indicates that polynucleotides and polypeptides corresponding to this gene are useful for the detection/treatment of seizures and epilepsy, including neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:61 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 544 of SEQ ID NO:61, b is an integer of 15 to 558, where both a and b correspond to the positions of nucleotide residues shown in SEQ ID NO:61, and where the b is greater than or equal to a + 14.

### FEATURES OF PROTEIN ENCODED BY GENE NO: 52

When tested against PC12 cell lines, supernatants removed from cells containing this gene activated the EGR1 (early growth response 1) pathway. Thus, it is likely that this gene activates sensory neuron cells through the EGR1 signal transduction pathway. EGR1 is a separate signal transduction pathway from Jaks-STAT, genes containing the EGR1 promoter are induced in various tissues and cell types upon activation, leading the cells to undergo differentiation and proliferation.

This gene is expressed primarily in fetal brain.

Therefore, polynucleotides and polypeptides of the invention are useful as reagents for differential identification of the tissue(s) or cell type(s) present in a biological sample and for diagnosis of diseases and conditions which include, but are not limited to, neurological disorders, particularly of the developing embryo. Similarly, polypeptides and antibodies directed to these polypeptides are useful in providing immunological probes for differential identification of the tissue(s) or cell type(s). For a number of disorders of the above tissues or cells, particularly of the fetal brain and CNS, expression of this gene at significantly higher or lower levels may be routinely detected in certain tissues , serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 134 as residues: Arg-16 to Thr-35.

The tissue distribution in fetal brain combined with the detected biological activity within sensory neurons indicates that polynucleotides and polypeptides corresponding to this gene are useful for the detection/treatment of neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:62 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 602 of SEQ ID NO:62, b is an integer of 15 to 616, where both a and b correspond to the positions of nucleotide residues shown in SEQ ID NO:62, and where the b is greater than or equal to a + 14.

### FEATURES OF PROTEIN ENCODED BY GENE NO: 53

This gene is expressed primarily in frontal cortex, and schizophrenoid brain tissue.

Therefore, polynucleotides and polypeptides of the invention are useful as reagents for differential identification of the tissue(s) or cell type(s) present in a biological sample and for diagnosis of diseases and conditions which include, but are not limited to, Schizophrenia, and other neurodegenerative disorders, including cancer. Similarly, polypeptides and antibodies directed to these polypeptides are useful in providing immunological probes for differential identification of the tissue(s) or cell type(s). For a number of disorders of the above tissues or cells, particularly of the brain, expression of this gene at significantly higher or lower levels may be routinely detected in certain tissues or cell types (e.g., neural tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:135 as residues: Asp-65 to Asn-72.

The tissue distribution indicates that polynucleotides and polypeptides corresponding to this gene are useful for the detection/treatment of neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:63 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 797 of SEQ ID NO:63, b is an integer of 15 to 811, where both a and b correspond to the positions of nucleotide residues shown in SEQ ID NO:63, and where the b is greater than or equal to a + 14.

### FEATURES OF PROTEIN ENCODED BY GENE NO: 54

This gene is expressed primarily in osteoclastoma, gall bladder, and infant brain.

Therefore, polynucleotides and polypeptides of the invention are useful as reagents for differential identification of the tissue(s) or cell type(s) present in a biological sample and for diagnosis of diseases and conditions which include, but are not limited to, Osteoclastoma, and gastrointestinal, skeletal, and neural disorders, particularly cancer. Similarly, polypeptides and antibodies directed to these polypeptides are useful in providing immunological probes for differential identification of the tissue(s) or cell type(s). For a number of disorders of the above tissues or cells, particularly of the bone, expression of this gene at significantly higher or lower levels may be routinely detected in certain tissues or cell types (e.g., skeletal tissue, neural tissue, and cancerous and wounded tissues) or bodily fluids (e.g., bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 136 as residues: Gly-23 to His-30.

The tissue distribution in osteoclastoma cells indicates that polynucleotides and polypeptides corresponding to this gene are useful for the detection, treatment, and/or prevention of various skeletal system disorders, particularly bone cancer. Moreover, the tissue distribution within the gall bladder indicates that the protein product of this gene is useful for the detection and treatment of liver disorders and cancers (e.g. hepatoblastoma, jaundice, hepatitis, liver metabolic diseases and conditions that are attributable to the differentiation of hepatocyte progenitor cells). In addition the expression in fetus would suggest a useful role for the protein product in developmental abnormalities, fetal deficiencies, pre-natal disorders and various would-healing models and/or tissue trauma. Osteoclastoma, Gall Bladder tumor. Many polynucleotide sequences, such as EST sequences, are publicly available and accessible through sequence databases. Some of these sequences are related to SEQ ID NO:64 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between I to 979 of SEQ ID NO:64, b is an integer of 15 to 993, where both a and b correspond to the positions of nucleotide residues shown in SEQ ID NO:64, and where the b is greater than or equal to a + 14.

### FEATURES OF PROTEIN ENCODED BY GENE NO: 55

The translation product of this gene shares sequence homology with b cell growth factor which is thought to be important in B cell development.

This gene is expressed primarily in breast lymph node and primary dendritic cells

Therefore, polynucleotides and polypeptides of the invention are useful as reagents for differential identification of the tissue(s) or cell type(s) present in a biological sample and for diagnosis of diseases and conditions which include, but are not limited to, immune disorders. Similarly, polypeptides and antibodies directed to these polypeptides are useful in providing immunological probes for differential identification of the tissue(s) or cell type(s). For a number of disorders of the above tissues or cells, particularly of the immune system expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 137 as residues: Ser-39 to Tyr-46.

The tissue distribution combined with its homology to a B-cell growth factor indicates that polynucleotides and polypeptides corresponding to this gene are useful for the diagnosis and treatment of a variety of immune system disorders. Expression of this gene product in lymph nodes indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:65 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 675 of SEQ ID NO:65, b is an integer of 15 to 689, where both a and b correspond to the positions of nucleotide residues shown in SEQ ID NO:65, and where the b is greater than or equal to a + 14.

### FEATURES OF PROTEIN ENCODED BY GENE NO: 56

The translation product of this gene shares sequence homology with small hepatocellular oncoprotein which is thought to be important in liver development (See Genebank Accession No. R07057).This gene maps to chromosome 20, and therefore, may be used as a marker in linkage analysis for chromosome 20.

This gene is expressed primarily in monocytes.

Therefore, polynucleotides and polypeptides of the invention are useful as reagents for differential identification of the tissue(s) or cell type(s) present in a biological sample and for diagnosis of diseases and conditions which include, but are not limited to, liver disorders, particularly hepatoma. Similarly, polypeptides and antibodies directed to these polypeptides are useful in providing immunological probes for differential identification of the tissue(s) or cell type(s). For a number of disorders of the above tissues or cells, particularly of the immune and digestive systems, expression of this gene at significantly higher or lower levels may be routinely detected in certain tissues or cell types (e.g., haematopoetic cells and tissue, liver, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:138 as residues: Met-17 to Lys-32.

The homology to a hepatocellular oncogene indicates that polynucleotides and polypeptides corresponding to this gene are useful for the detection and treatment of liver disorders and cancers (e.g. hepatoblastoma, jaundice, hepatitis, liver metabolic diseases and conditions that are attributable to the differentiation of hepatocyte progenitor cells). The expression also indicates a useful role for the protein product in developmental abnormalities, fetal deficiencies, pre-natal disorders and various would-healing models and/or tissue trauma. Protein, as well as, antibodies directed against the protein may show utility as a tumor marker and/or immunotherapy targets for the above listed tissues. Alternatively, The tissue distribution indicates that polynucleotides and polypeptides corresponding to this gene are . useful for the treatment and diagnosis of hematopoetic related disorders such as anemia, pancytopenia, leukopenia, thrombocytopenia or leukemia since stromal cells are important in the production of cells of hematopoietic lineages. The uses include bone marrow cell ex vivo culture, bone marrow transplantation, bone marrow reconstitution, radiotherapy or chemotherapy of neoplasia. The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Many polynucleotide sequences, such as EST sequences, are publicly available and accessible through sequence databases. Some of these sequences are related to SEQ ID NO:66 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 928 of SEQ ID NO:66, b is an integer of 15 to 942, where both a and b correspond to the positions of nucleotide residues shown in SEQ ID NO:66, and where the b is greater than or equal to a + 14.

### FEATURES OF PROTEIN ENCODED BY GENE NO: 57

The translation product of this gene was shown to have homology to the human proteins myotubularin related protein 3 and NTII-1 nerve protein (See Genebank Accession Nos. gil1378120 and R99800, respectively) which are both thought to play important roles as growth factors in muscle and nerve tissue, respectively.Preferred polypeptides comprise the following amino acid sequence:
DDDGLPFPTDVIQHRLRQIEAGYKQEVEQLRR QVRDSDEXGHPSLLCPSSRAPMDYEDDFTCLKESDGSDTEDFGSDHSEDCLSEA SWEPVDKKETEVTRWVPDHMASHCYNCDCEFWLAKRRHHCRNCGNVFCAG CCHLKLPIPDQQLYDPVLVCNSCYXTHSSLSCQGTHEPTAEETHCYSFQLNAGE KPVQF (SEQ ID NO:173), SEASWEPVDKKETEVTRWVPDHMASHCY (SEQ ID NO:174), HHCRNCGNVF (SEQ ID NO: 175, and/or RLRQIEAGYKQEVE (SEQ ID NO:176). Also preferred are the polynucleotides encoding these polypeptides.

This gene is expressed primarily in bone, spleen, brain, apoptotic T cells, hypothalmus, and other immune cells.

Therefore, polynucleotides and polypeptides of the invention are useful as reagents for differential identification of the tissue(s) or cell type(s) present in a biological sample and for diagnosis of diseases and conditions which include, but are not limited to, immune system, musculoskeletal, and neural disorders. Similarly, polypeptides and antibodies directed to these polypeptides are useful in providing immunological probes for differential identification of the tissue(s) or cell type(s). For a number of disorders of the above tissues or cells, particularly of the central nervous and immune systems, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:139 as residues: Glu-63 to Asp-68.

The tissue distribution in neural tissue combined with the homology to a nerve growth factor indicates that polynucleotides and polypeptides corresponding to this gene are useful for the detection/treatment of neurodegenerative disease states and behavioral disorders such as Alzheimers Disease, Parkinsons Disease, Huntingtons Disease, Tourette Syndrome, schizophrenia, mania, dementia, paranoia, obsessive compulsive disorder, panic disorder, learning disabilities, ALS, psychoses , autism, and altered behaviors, including disorders in feeding, sleep patterns, balance, and perception, as well as neuromuscular disorders such as MS and muscular dystrophy. In addition, the gene or gene product may also play a role in the treatment and/or detection of developmental disorders associated with the developing embryo, sexually-linked disorders, or disorders of the cardiovascular system. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:67 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2295 of SEQ ID NO:67, b is an integer of 15 to 2309, where both a and b correspond to the positions of nucleotide residues shown in SEQ ID NO:67, and where the b is greater than or equal to a + 14.

### FEATURES OF PROTEIN ENCODED BY GENE NO: 58

This gene is expressed primarily in ovary.

Therefore, polynucleotides and polypeptides of the invention are useful as reagents for differential identification of the tissue(s) or cell type(s) present in a biological sample and for diagnosis of diseases and conditions which include, but are not limited to, ovarian cancer, and other disorders that afflict the female reproductive system. Similarly, polypeptides and antibodies directed to these polypeptides are useful in providing immunological probes for differential identification of the tissue(s) or cell type(s). For a number of disorders of the above tissues or cells, particularly of the female reproductive system, expression of this gene at significantly higher or lower levels may be routinely detected in certain tissues or cell types (e.g., reproductive tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:140 as residues: Pro-23 to Gly-54.

The tissue distribution in tumors the ovary indicates that polynucleotides and polypeptides corresponding to this gene are useful for diagnosis and intervention of these tumors, in addition to other tumors where expression has been indicated. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:68 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 800 of SEQ ID NO:68, b is an integer of 15 to 814, where both a and b correspond to the positions of nucleotide residues shown in SEQ ID NO:68, and where the b is greater than or equal to a + 14.

### FEATURES OF PROTEIN ENCODED BY GENE NO: 59

This gene is expressed primarily in ovary.

Therefore, polynucleotides and polypeptides of the invention are useful as reagents for differential identification of the tissue(s) or cell type(s) present in a biological sample and for diagnosis of diseases and conditions which include, but are not limited to, ovarian cancer, and other disorders afflicting the female reproductive system. Similarly, polypeptides and antibodies directed to these polypeptides are useful in providing immunological probes for differential identification of the tissue(s) or cell type(s). For a number of disorders of the above tissues or cells, particularly of the reproductive system, expression of this gene at significantly higher or lower levels may be routinely detected in certain tissues or cell types (e.g., reproductive tissue, and cancerous and wounded tissues) or bodily fluids (e.g., amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in ovarian tissue indicates that polynucleotides and polypeptides corresponding to this gene are useful for diagnosis and intervention of ovarian tumors, in addition to other tumors of the female reproductive system. Protein, as well as, antibodies directed against the protein may show utility as a tissue-specific marker and/or immunotherapy target for the above listed tissues. Many polynucleotide sequences, such as EST sequences, are publicly available and accessible through sequence databases. Some of these sequences are related to SEQ ID NO:69 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 774 of SEQ ID NO:69, b is an integer of 15 to 788, where both a and b correspond to the positions of nucleotide residues shown in SEQ ID NO:69, and where the b is greater than or equal to a + 14.

### FEATURES OF PROTEIN ENCODED BY GENE NO: 60

When tested against PC12 cell lines, supernatants removed from cells containing this gene activated the EGR1 (early growth response 1) pathway. Thus, it is likely that this gene activates sensory neuronal cells through the EGR1 signal transduction pathway.. EGR1 is a separate signal transduction pathway from Jaks-STAT, genes containing the EGR1 promoter are induced in various tissues and cell types upon activation, leading the cells to undergo differentiation and proliferation.

This gene is expressed primarily in osteoblast.

Therefore, polynucleotides and polypeptides of the invention are useful as reagents for differential identification of the tissue(s) or cell type(s) present in a biological sample and for diagnosis of diseases and conditions which include, but are not limited to, skeletal disorders, particular bone cancer. Similarly, polypeptides and antibodies directed to these polypeptides are useful in providing immunological probes for differential identification of the tissue(s) or cell type(s). For a number of disorders of the above tissues or cells, particularly of the Bone, expression of this gene at significantly higher or lower levels may be routinely detected in certain tissues or cell types (e.g., skeletal tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:142 as residues: Tyr-20 to Lys-31.

In addition the expression of this gene product in synovium would suggest a role in the detection and treatment of disorders and conditions affecting the skeletal system, in particular osteoporosis as well as disorders afflicting connective tissues (e.g. arthritis, trauma, tendonitis, chrondomalacia and inflammation) as well as in the diagnosis or treatment of various autoimmune disorders such as rheumatoid arthritis, lupus, scleroderma, and dermatomyositis as well as dwarfism, spinal deformation, andspecific joint abnormalities as well as chondrodysplasias i.e. spondyloepiphyseal dysplasia congenita, familial osteoarthritis, Atelosteogenesis type II, metaphyseal chondrodysplasia type Schmid. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:70 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 777 of SEQ ID NO:70, b is an integer of 15 to 791, where both a and b correspond to the positions of nucleotide residues shown in SEQ ID NO:70, and where the b is greater than or equal to a + 14.

### FEATURES OF PROTEIN ENCODED BY GENE NO: 61

This gene is expressed primarily in adipocyte.

Therefore, polynucleotides and polypeptides of the invention are useful as reagents for differential identification of the tissue(s) or cell type(s) present in a biological sample and for diagnosis of diseases and conditions which include, but are not limited to, obesity. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., adipose tissue, and cancerous and wounded tissues) or bodily fluids (e.g., serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 143 as residues: His-2 to Leu-8.

The tissue distribution would suggest that polynucleotides and polypeptides corresponding to this gene are useful in the diagnosis, treatment, and/or prevention of obesity and lipid metabolism disorders. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:71 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 790 of SEQ ID NO:71, b is an integer of 15 to 804, where both a and b correspond to the positions of nucleotide residues shown in SEQ ID NO:71, and where the b is greater than or equal to a + 14.

### FEATURES OF PROTEIN ENCODED BY GENE NO: 62

When tested against Jurkat T-cell lines, supernatants removed from cells containing this gene activated the GAS (gamma activation site) pathway. Thus, it is likely that this gene activates T-cells through the Jak-STAT signal transduction pathway. GAS is a promoter element found upstream in many genes which are involved in the Jaks-STAT pathway. The Jaks-STAT pathway is a large, signal transduction pathway involved in the differentiation and proliferation of cells.

This gene is expressed primarily in synovial tissue.

Therefore, polynucleotides and polypeptides of the invention are useful as reagents for differential identification of the tissue(s) or cell type(s) present in a biological sample and for diagnosis of diseases and conditions which include, but are not limited to, chronic synovitis, immune. Similarly, polypeptides and antibodies directed to these polypeptides are useful in providing immunological probes for differential identification of the tissue(s) or cell type(s). For a number of disorders of the above tissues or cells, particularly of the immune system expression of this gene at significantly higher or lower levels may be routinely detected in certain tissues or cell types (e.g., cells and tissue of the immune system, and skeletal tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO: 144 as residues: Pro-74 to Lys-82.

The biological assay results indicating activity in Jurkat T-cells for the protein product of this gene indicates that polynucleotides and polypeptides corresponding to this gene useful for the diagnosis and treatment of a variety of immune system disorders. This gene product may be involved in the regulation of cytokine production, antigen presentation, or other processes that may also suggest a usefulness in the treatment of cancer (e.g. by boosting immune responses). Since the gene has been shown to activate genes in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. In addition the expression of this gene product in synovial tissues indicates a role for this protein in the detection and treatment of disorders and conditions affecting the skeletal system, in particular the connective tissues (e.g. arthritis, trauma, tendonitis, chrondomalacia and inflammation) as well as in the diagnosis or treatment of various autoimmune disorders such as rheumatoid arthritis, lupus, scleroderma, and dermatomyositis as well as dwarfism, spinal deformation, and specific joint abnormalities as well as chondrodysplasias i.e. spondyloepiphyseal dysplasia congenita, familial osteoarthritis, Atelosteogenesis type II, metaphyseal chondrodysplasia type Schmid. Protein, as well as, antibodies directed against the protein may show utility as a tumor marker and/or immunotherapy targets for the above listed tissues.. Many polynucleotide sequences, such as EST sequences, are publicly available and accessible through sequence databases. Some of these sequences are related to SEQ ID NO:72 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 769 of SEQ ID NO:72, b is an integer of 15 to 783, where both a and b correspond to the positions of nucleotide residues shown in SEQ ID NO:72, and where the b is greater than or equal to a + 14.

### FEATURES OF PROTEIN ENCODED BY GENE NO: 63

The translation product of this gene shares sequence homology with fetal troponin which is known to be essential for normal muscular function in all skeletal and cardiovascular muscles.

This gene is expressed primarily in melanocytes, fetal liver, brain,testes, spleen, and placenta.

Therefore, polynucleotides and polypeptides of the invention are useful as reagents for differential identification of the tissue(s) or cell type(s) present in a biological sample and for diagnosis of diseases and conditions which include, but are not limited to, melanoma, neuromuscular disorders, such as multiple sclerosis, and endothelial-related diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels may be routinely detected in certain tissues or cell types (e.g., muscle, endothelial cells and tissue, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:145 as residues: Ala-27 to Leu-36, Phe-40 to Leu-50.

The tissue distribution and homology to troponin indicates that polynucleotides and polypeptides corresponding to this gene are useful for treatment, diagnosis, and/or prevention of various neuromuscular disorders such as MS, muscular dystrophy, cardiomyopathy, myositis, myomas, leiomyomas, rhabdomyosarcomas, and coronary heart disease. Alternatively, the expression in a variety of fetal immune tissues indicates that the protein product of this gene is useful for the diagnosis and treatment of a variety of immune system disorders. Expression of this gene product in spleen and fetal liver indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:73 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1509 of SEQ ID NO:73, b is an integer of 15 to 1523, where both a and b correspond to the positions of nucleotide residues shown in SEQ ID NO:73, and where the b is greater than or equal to a + 14.

### FEATURES OF PROTEIN ENCODED BY GENE NO: 64

The translation product of this gene shares sequence homology with small hepatocellular oncoprotein gene which is thought to be important in liver disorders (See Genebank Accession No. R07057). One embodiment of this gene comprises polypeptides of the following amino acid sequence:
MSHCARPLFFETFFILLSPRLKCSGTNTVHYSLDLLGSSNSASVPQVGGLTNAQ HDTWLIFVFCVCVCEPLRRPWAAFLISVTSSIK (SEQ ID NO:177), and/or VPQVGGLTNAQHDTWLIFVFCVCVCEPLRR (SEQ ID NO: 178). An additional embodiment is the polynucleotides encoding these polypeptides.

This gene is expressed primarily in neutrophils, hemangiopericytoma, activated T cells.

Therefore, polynucleotides and polypeptides of the invention are useful as reagents for differential identification of the tissue(s) or cell type(s) present in a biological sample and for diagnosis of diseases and conditions which include, but are not limited to, immune and hepatic disorders. Similarly, polypeptides and antibodies directed to these polypeptides are useful in providing immunological probes for differential identification of the tissue(s) or cell type(s). For a number of disorders of the above tissues or cells, particularly of the hepatoma and immune system disorder, expression of this gene at significantly higher or lower levels may be routinely detected in certain tissues or cell types (e.g., cells and tissue of the immune system, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. Preferred epitopes include those comprising a sequence shown in SEQ ID NO:146 as residues: Arg-20 to Gly-25.

The tissue distribution indicates that polynucleotides and polypeptides corresponding to this gene are useful for the diagnosis and treatment of a variety of immune system disorders. Expression of this gene product in neutrophils and T-cells indicates a role in the regulation of the proliferation; survival; differentiation; and/or activation of potentially all hematopoietic cell lineages, including blood stem cells. This gene product may be involved in the regulation of cytokine production, antigen presentation, or other processes that may also suggest a usefulness in the treatment of cancer (e.g. by boosting immune responses). Since the gene is expressed in cells of lymphoid origin, the natural gene product may be involved in immune functions. Therefore it may be also used as an agent for immunological disorders including arthritis, asthma, immune deficiency diseases such as AIDS, leukemia, rheumatoid arthritis, inflammatory bowel disease, sepsis, acne, and psoriasis. Protein, as well as, antibodies directed against the protein may show utility as a tumor marker and/or immunotherapy targets for the above listed tumors and tissues. In addition, this gene product may have commercial utility in the expansion of stem cells and committed progenitors of various blood lineages, and in the differentiation and/or proliferation of various cell types. Alternatively, the homology to a hepatic oncogene indicates that the protein product of this gene is useful for the detection and treatment of liver disorders and cancers (e.g. hepatoblastoma, jaundice, hepatitis, liver metabolic diseases and conditions that are attributable to the differentiation of hepatocyte progenitor cells). In addition, the expression would suggest a useful role for the protein product in developmental abnormalities, fetal deficiencies, pre-natal disorders and various would-healing models and/or tissue trauma. Protein, as well as, antibodies directed against the protein may show utility as a tumor marker and/or immunotherapy targets for the above listed tissues. Many polynucleotide sequences, such as EST sequences, are publicly available and accessible through sequence databases. Some of these sequences are related to SEQ ID NO:74 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 744 of SEQ ID NO:74, b is an integer of 15 to 758, where both a and b correspond to the positions of nucleotide residues shown in SEQ ID NO:74, and where the b is greater than or equal to a + 14.

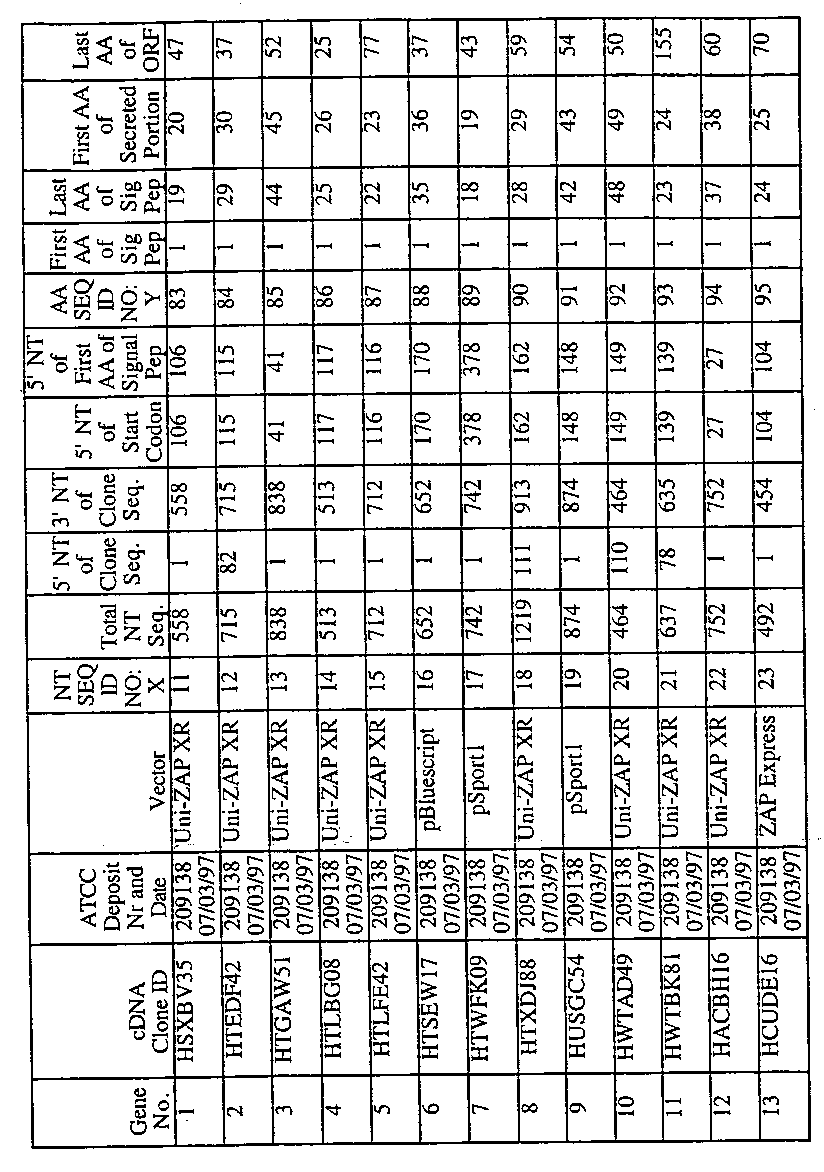

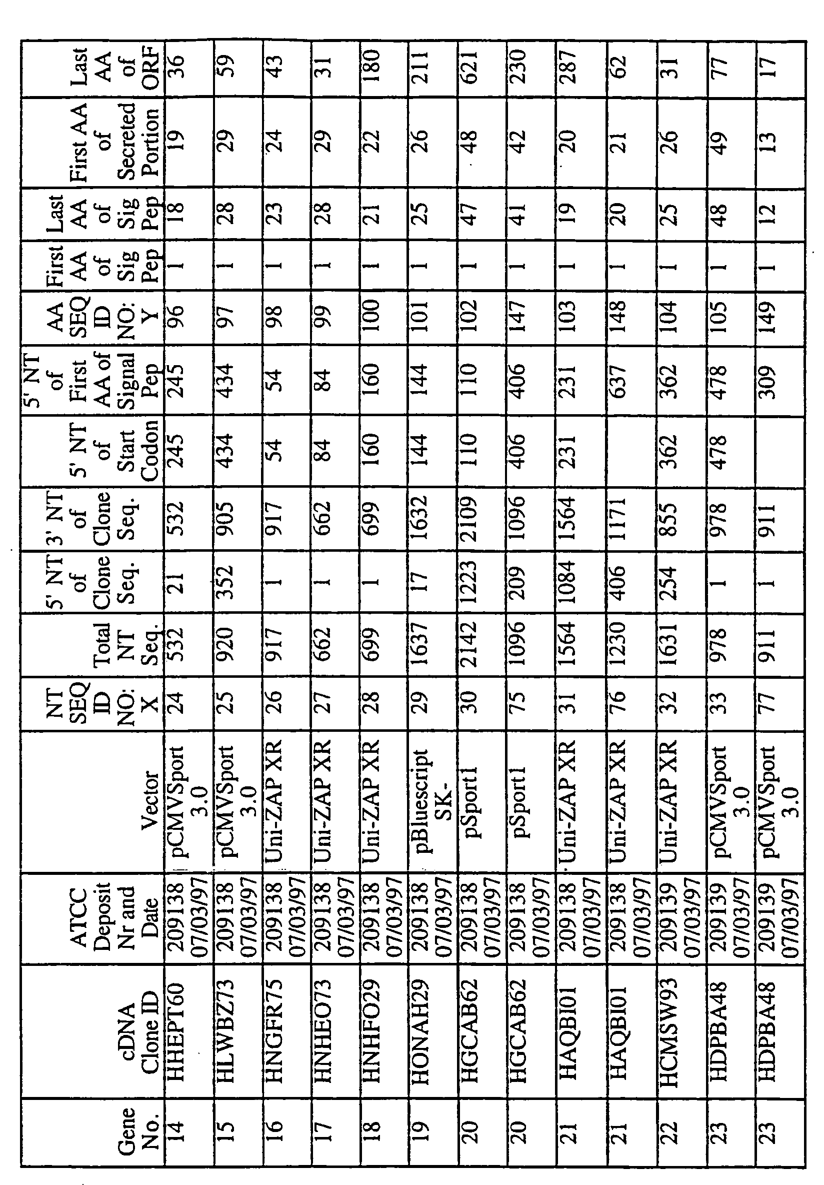

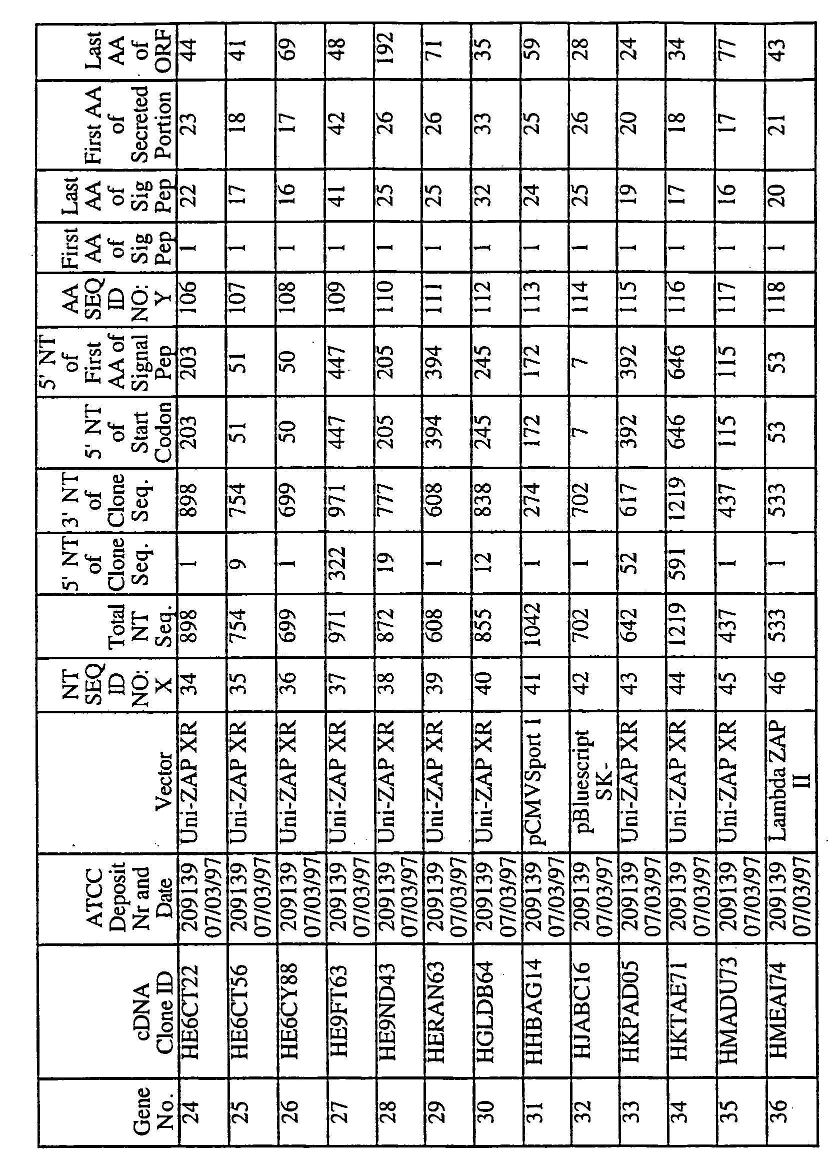

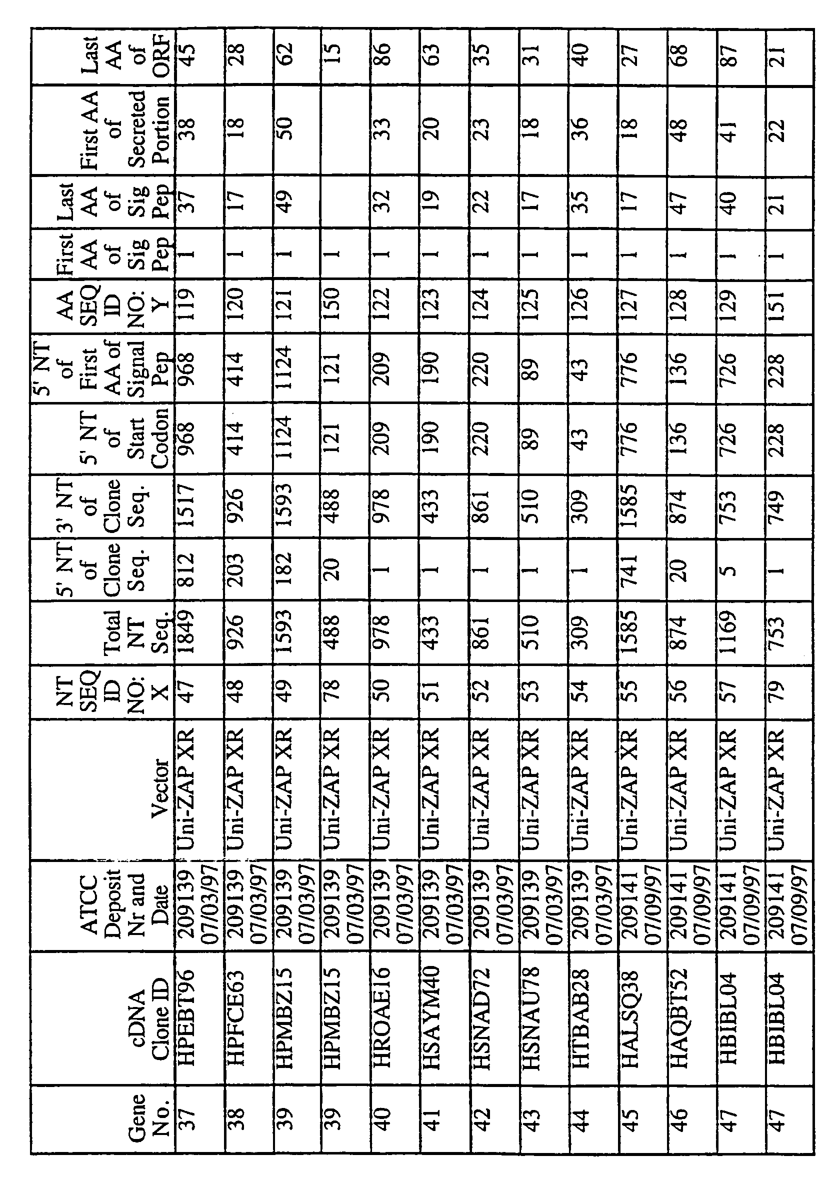

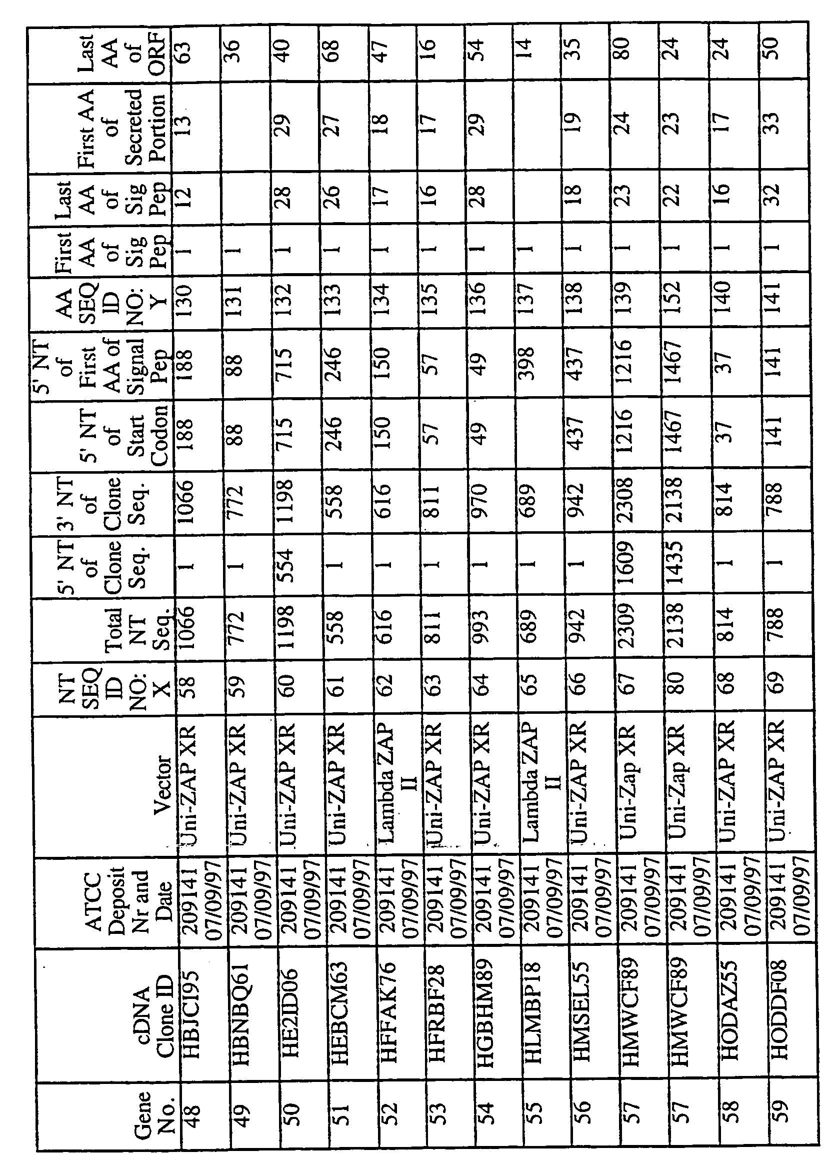

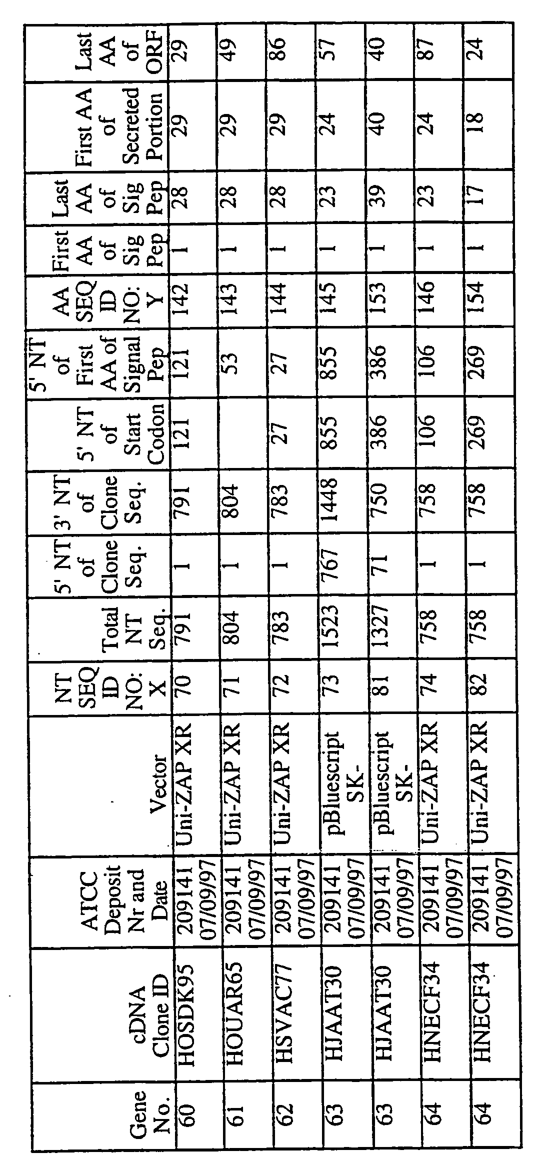

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X and the translated SEQ ID NO:Y are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used to generate antibodies which bind specifically to the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are species homologs. Species homologs may be isolated and identified by making suitable probes or primers from the sequences provided herein and screening a suitable nucleic acid source for the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified by the one-step method described in Smith and Johnson, Gene 67:31-40 (1988).
Polypeptides of the invention also can be purified from natural or recombinant sources using antibodies of the invention raised against the secreted protein in methods which are well known in the art.

### Signal Sequences

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13 to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. These polypeptides, and the polynucleotides encoding such polypeptides, are contemplated by the present invention.

### Polynucleotide and Polypeptide Variants

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the polypeptide. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragement specified as described herein.

As a practical matter, whether any particular nucleic acid molecule or polypeptide is at least 90%, 95%, 96%, 97%, 98% or 99% identical to a nucleotide sequence of the presence invention can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are:
Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is becuase the FASTDB program does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignement of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to, for instance, the amino acid sequences shown in Table 1 or to the amino acid sequence encoded by deposited DNA clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. (1990) 6:237-245). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, J. U. et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1085 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

### Polynucleotide and Polypeptide Fragments

In the present invention, a "polynucleotide fragment" refers to a short polynucleotide having a nucleic acid sequence contained in the deposited clone or shown in SEQ ID NO:X. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clone or the nucleotide sequence shown in SEQ ID NO:X. These nucleotide fragments are useful as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments having a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, or 2001 to the end of SEQ ID NO:X or the cDNA contained in the deposited clone. In this context "about" includes the particularly recited ranges, larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein.

In the present invention, a "polypeptide fragment" refers to a short amino acid sequence contained in SEQ ID NO:Y or encoded by the cDNA contained in the deposited clone. Protein fragments may be "free-standing," or comprised within a larger polypeptide of which the fragment forms a part or region, most preferably as a single continuous region. Representative examples of polypeptide fragments of the invention, include, for example, fragments from about amino acid number 1-20, 21-40, 41-60, 61-80, 81-100, 102-120, 121-140, 141-160, or 161 to the end of the coding region. Moreover, polypeptide fragments can be about 20, 30, 40, 50, 60, 70, 80, 90, 100, 110, 120, 130, 140, or 150 amino acids in length. In this context "about" includes the particularly recited ranges, larger or smaller by several (5, 4, 3, 2, or 1) amino acids, at either extreme or at both extremes.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotide fragments encoding these polypeptide fragments are also preferred.

Particularly, N-terminal deletions of the polypeptide of the present invention can be described by the general formula m-p, where p is the total number of amino acids in the polypeptide and m is an integer from 2 to (p-1), and where both of these integers (m & p) correspond to the position of the amino acid residue identified in SEQ ID NO:Y.

Moreover, C-terminal deletions of the polypeptide of the present invention can also be described by the general formula 1-n, where n is an integer from 2 to (p-1), and again where these integers (n & p) correspond to the position of the amino acid residue identified in SEQ ID NO:Y.

The invention also provides polypeptides having one or more amino acids deleted from both the amino and the carboxyl termini, which may be described generally as having residues m-n of SEQ ID NO:Y, where m and n are integers as described above.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotide fragments encoding these domains are also contemplated.

Other preferred fragments are biologically active fragments. Biologically active fragments are those exhibiting activity similar, but not necessarily identical, to an activity of the polypeptide of the present invention. The biological activity of the fragments may include an improved desired activity, or a decreased undesirable activity.

### Epitopes & Antibodies

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998-4002 (1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope. (See, for instance, Wilson et al., Cell 37:767-778 (1984); Sutcliffe, J. G. et al., Science 219:660-666 (1983).)

Similarly, immunogenic epitopes can be used to induce antibodies according to methods well known in the art. (See, for instance, Sutcliffe et al., supra; Wilson et al., supra; Chow, M. et al., Proc. Natl. Acad. Sci. USA 82:910-914; and Bittle, F. J. et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting.)

As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies.

### Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgG), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331:84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270:9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

### Vectors, Host Cells, and Protein Production

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAF-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X. Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sublocalization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques," Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library) .) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

In addition to the foregoing, a polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee et al., Nucl. Acids Res. 6:3073 (1979); Cooney et al., Science 241:456 (1988); and Dervan et al., Science 251:1360(1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell . Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn are used to measure protein expression from a recombinant cell, as a way of assessing transformation of the host cell. Moreover, the polypeptides of the present invention can be used to test the following biological activities.

### Biological Activities

The polynucleotides and polypeptides of the present invention can be used in assays to test for one or more biological activities. If these polynucleotides and polypeptides do exhibit activity in a particular assay, it is likely that these molecules may be involved in the diseases associated with the biological activity. Thus, the polynucleotides and polypeptides could be used to treat the associated disease.

### Immune Activity

A polypeptide or polynucleotide of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, a polynucleotide or polypeptide of the present invention can be used as a marker or detector of a particular immune system disease or disorder.

A polynucleotide or polypeptide of the present invention may be useful in treating or detecting deficiencies or disorders of hematopoietic cells. A polypeptide or polynucleotide of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polypeptide or polynucleotide of the present invention could also be used to modulate hemostatic (the stopping of bleeding) or thrombolytic activity (clot formation). For example, by increasing hemostatic or thrombolytic activity, a polynucleotide or polypeptide of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotide or polypeptide of the present invention that can decrease hemostatic or thrombolytic activity could be used to inhibit or dissolve clotting. These molecules could be important in the treatment of heart attacks (infarction), strokes, or scarring.

A polynucleotide or polypeptide of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polypeptide or polynucleotide of the present invention that inhibits an immune response, particularly the proliferation, differentiation, or chemotaxis of T-cells, may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polypeptide or polynucleotide of the present invention. Moreover, these molecules can be used to treat anaphylaxis, hypersensitivity to an antigenic molecule, or blood group incompatibility.

A polynucleotide or polypeptide of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polypeptide or polynucleotide of the present invention that inhibits an immune response, particularly the proliferation, differentiation, or chemotaxis of T-cells, may be an effective therapy in preventing organ rejection or GVHD.

Similarly, a polypeptide or polynucleotide of the present invention may also be used to modulate inflammation. For example, the polypeptide or polynucleotide may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

A polypeptide or polynucleotide can be used to treat or detect hyperproliferative disorders, including neoplasms. A polypeptide or polynucleotide of the present invention may inhibit the proliferation of the disorder through direct or indirect interactions. Alternatively, a polypeptide or polynucleotide of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotide or polypeptide of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotide or polypeptide of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

A polypeptide or polynucleotide of the present invention can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, the polypeptide or polynucleotide of the present invention may also directly inhibit the infectious agent, without necessarily eliciting an immune response.

Viruses are one example of an infectious agent that can cause disease or symptoms that can be treated or detected by a polynucleotide or polypeptide of the present invention. Examples of viruses, include, but are not limited to the following DNA and RNA viral families: Arbovirus, Adenoviridae, Arenaviridae, Arterivirus, Bimaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-I, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. A polypeptide or polynucleotide of the present invention can be used to treat or detect any of these symptoms or diseases.

Similarly, bacterial or fungal agents that can cause disease or symptoms and that can be treated or detected by a polynucleotide or polypeptide of the present invention include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. A polypeptide or polynucleotide of the present invention can be used to treat or detect any of these symptoms or diseases.

Moreover, parasitic agents causing disease or symptoms that can be treated or detected by a polynucleotide or polypeptide of the present invention include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. A polypeptide or polynucleotide of the present invention can be used to treat or detect any of these symptoms or diseases.

Preferably, treatment using a polypeptide or polynucleotide of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

### Regeneration

A polynucleotide or polypeptide of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, burns, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide of the present invention may increase regeneration of tissues difficult to heal. For example, increased tendon/ligament regeneration would quicken recovery time after damage. A polynucleotide or polypeptide of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide of the present invention.

### Chemotaxis

A polynucleotide or polypeptide of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide of the present invention may inhibit chemotactic activity. These molecules could also be used to treat disorders. Thus, a polynucleotide or polypeptide of the present invention could be used as an inhibitor of chemotaxis.

### Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptide from suitably manipulated cells or tissues.

Therefore, the invention includes a method of identifying compounds which bind to a polypeptide of the invention comprising the steps of: (a) incubating a candidate binding compound with a polypeptide of the invention; and (b) determining if binding has occurred. Moreover, the invention includes a method of identifying agonists/antagonists comprising the steps of: (a) incubating a candidate compound with a polypeptide of the invention, (b) assaying a biological activity , and (b) determining if a biological activity of the polypeptide has been altered.

### Other Activities

A polypeptide or polynucleotide of the present invention may also increase or decrease the differentiation or proliferation of embryonic stem cells, besides, as discussed above, hematopoietic lineage.

A polypeptide or polynucleotide of the present invention may also be used to modulate mammalian characteristics, such as body height, weight, hair color, eye color, skin, percentage of adipose tissue, pigmentation, size, and shape (e.g., cosmetic surgery). Similarly, a polypeptide or polynucleotide of the present invention may be used to modulate mammalian metabolism affecting catabolism, anabolism, processing, utilization, and storage of energy.

A polypeptide or polynucleotide of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

A polypeptide or polynucleotide of the present invention may also be used as a food additive or preservative, such as to increase or decrease storage capabilities, fat content, lipid, protein, carbohydrate, vitamins, minerals, cofactors or other nutritional components.

### Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

Also preferred is an isolated nucleic acid molecule comprising a nucleotide sequence which is at least 95% identical to a sequence of at least about 150 conuguous nucleotides in the nucleotide sequence of SEQ ID NO:X.

Further preferred is an isolated nucleic acid molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a nucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is a polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide, polynucleotide, or antibody of the claimed invention effective to increase the level of said protein activity in said individual.

Having generally described the invention, the same will be more readily understood by reference to the following examples, which are provided by way of illustration and are not intended as limiting.

### Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL-1 Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance, with ³²P-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 µl of reaction mixture with 0.5 ug of the above cDNA template. A convenient reaction mixture is 1.5-5 mM MgCl₂, 0.01% (w/v) gelatin, 20 µM each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

### Example 2: Isolation of Genomic Clones Corresponding to a Polynucleotide

A human genomic P1 library (Genomic Systems, Inc.) is screened by PCR using primers selected for the cDNA sequence corresponding to SEQ ID NO:X., according to the method described in Example 1. (See also, Sambrook.)

### Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with P³² using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

### Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance (Amp^{r}), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCI by stirring for 3-4 hours at 4°C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4° C or frozen at -80° C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4°C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 µm membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 µg of purified protein is loaded. The purified protein can also be tested for endotoxin/LPS contamination, and typically the LPS content is less than 0.1 ng/ml according to LAL assays.

### Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five µg of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27° C. The transfection solution is then removed from the plate and 1 ml of Grace's insect medium supplemented with 10% fetal calf serum is added. Cultivation is then continued at 27° C for four days.

After four days the supernatant is collected and a plaque assay is performed, as described by Summers and Smith, *supra*. An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4° C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

### Example 8: Expression of a Polypeptide in Mammalian Cells

The polypeptide of the present invention can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100 - 200 µM. Expression of the desired gene product is analyzed, for instance, by SDS-PAGE and Western blot or by reversed phase HPLC analysis.

### Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly, fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)
Human IgG Fc region:

GGGATCCGGAGCCCAAATCTTCTGACAAAACTCACACATGCCCACCGTGCC
CAGCACCTGAATTCGAGGGTGCACCGTCAGTCTTCCTCTTCCCCCCAAAACC
CAAGGACACCCTCATGATCTCCCGGACTCCTGAGGTCACATGCGTGGTGGT
GGACGTAAGCCACGAAGACCCTGAGGTCAAGTTCAACTGGTACGTGGACG
GCGTGGAGGTGCATAATGCCAAGACAAAGCCGCGGGAGGAGCAGTACAAC
AGCACGTACCGTGTGGTCAGCGTCCTCACCGTCCTGCACCAGGACTGGCTG

AATGGCAAGGAGTACAAGTGCAAGGTCTCCAACAAAGCCCTCCCAACCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCCT
GACCTGCCTGGTCAAAGGCTTCTATCCAAGCGACATCGCCGTGGAGTGGGA
GAGCAATGGGCAGCCGGAGAACAACTACAAGACCACGCCTCCCGTGCTGG
ACTCCGACGGCTCCTTCTTCCTCTACAGCAAGCTCACCGTGGACAAGAGCA
GGTGGCAGCAGGGGAACGTCTTCTCATGCTCCGTGATGCATGAGGCTCTGC
ACAACCACTACACGCAGAAGAGCCTCTCCCTGTCTCCGGGTAAATGAGTGC
GACGGCCGCGACTCTAGAGGAT (SEQ ID NO:1)

### Example 10: Production of an Antibody from a Polypeptide

The antibodies of the present invention can be prepared by a variety of methods. (See, Current Protocols, Chapter 2.) For example, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).)

### Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x 10⁵ cells/well in .5ml DMEM(Dulbecco's Modified Eagle Medium)(with 4.5 G/L glucose and L-glutamine (12-604F Biowhittaker))/10% heat inactivated FBS(14-503F Biowhittaker)/lx Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with .5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37°C for 6 hours.

While cells are incubating, prepare appropriate media, either 1%BSA in DMEM with 1x penstrep, or CHO-5 media (116.6 mg/L of CaCl2 (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of Kcl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂0; 71.02 mg/L of Na₂HPO4; .4320 mg/L of ZnSO₄-7H₂O; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-H₂0; 6.65 mg/ml of L-Aspartic Acid; 29.56 mg/ml of L-Cystine-2HCL-H₂0; 31.29 mg/ml of L-Cystine-2HCL; 7.35 mg/ml of L-Glutamic Acid; 365.0 mg/ml of L-Glutamine; 18.75 mg/ml of Glycine; 52.48 mg/ml of L-Histidine-HCL-H₂0; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂0; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; and 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37°C for 45 or 72 hours depending on the media used: 1%BSA for 45 hours or CHO-5 for 72 hours.

On day four, using a 300ul multichannel pipetter, aliquot 600ul in one 1ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

### Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Damell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proxial region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |

| gp130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) ? | | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) - | | + | - | + | 6 | GAS (IRF1 = IFP »Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp140 family | | | | | | |
|---|---|---|---|---|---|---|
| IL-3 (myeloid) | - | - | + | - | 5 | GAS (IRF1>IFP»Ly6) |
| IL-5 (myeloid) | - | - | + | - | 5 | GAS |
| GM-CSF (myeloid)- | | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCG
AAATGATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATG
ATTTCCCCGAAATATCTGCCATCTCAATTAGTCAGCAACCATAGTCCCGCCC
CTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCGGC
CTCTGAGCTATTCCAGAAGTAGTGAGGAGGCTTTTTTGGAGGCCTAGGCTTT
TGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using Sail and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity by identifying factors, such as growth factors and cytokines, that may proliferate or differentiate T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing a polypeptide as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity

The following protocol is used to assess myeloid activity by identifying factors, such as growth factors and cytokines, that may proliferate or differentiate myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest 2x10e⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM Na₂HPO₄.7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 ul cells per well in the 96-well plate (or 1x10⁵ cells/well).

Add 50 ul of the supernatant prepared by the protocol described in Example 11. Incubate at 37°C for 48 to 72 hr. As a positive control, 100 Unit/ml interferon gamma can be used which is known to activate U937 cells. Over 30 fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes XhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 ul of the cell suspension to each well of 96-well plate (equivalent to 1x10⁵ cells/well). Add 50 ul supernatant produced by Example 11, 37°C for 48 to 72 hr. As a positive control, a growth factor known to activate PC12 cells through EGR can be used, such as 50 ng/ul of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity

NF-κB (Nuclear Factor κB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-κB regulates the expression of genes involved in immune cell activation, control of apoptosis (NF-κB appears to shield cells from apoptosis), B and T-cell development, anti-viral and antimicrobial responses, and multiple stress responses.

In non-stimulated conditions, NF-κB is retained in the cytoplasm with I-κB (Inhibitor κB). However, upon stimulation, I- κB is phosphorylated and degraded, causing NF- κB to shuttle to the nucleus, thereby activating transcription of target genes. Target genes activated by NF- κB include IL-2, IL-6, GM-CSF, ICAM-1 and class 1 MHC.

Due to its central role and ability to respond to a range of stimuli, reporter constructs utilizing the NF-κB promoter element are used to screen the supematants produced in Example 11. Activators or inhibitors of NF-kB would be useful in treating diseases. For example, inhibitors of NF-κB could be used to treat those diseases related to the acute or chronic activation of NF-kB, such as rheumatoid arthritis.

To construct a vector containing the NF-κB promoter element, a PCR based strategy is employed. The upstream primer contains four tandem copies of the NF-κB binding site (GGGGACTTTCCC) (SEQ ID NO:8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGAC
TTTCCATCCTGCCATCTCAATTAG:3' (SEQ ID NO:9)

The downstream primer is complementary to the 3' end of the SV40 promoter and is flanked with a Hind III site:

5':GCGGCAAGCTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCC
ATCTGCCATCTCAATTAGTCAGCAACCATAGTCCCGCCCCTAACTCCGCCCA
TCCCGCCCCTAACTCCGCCCAGTTCCGCCCATTCTCCGCCCCATGGCTGACT
AATTTTTTTTATTTATGCAGAGGCCGAGGCCGCCTCGGCCTCTGAGCTATTC
CAGAAGTAGTGAGGAGGCTTTTTTGGAGGCCTAGGCTTTTGCAAAAAGCTT:
3' (SEQ ID NO:10)

Next, replace the SV40 minimal promoter element present in the pSEAP2-promoter plasmid (Clontech) with this NF-κB/SV40 fragment using XhoI and HindIII. However, this vector does not contain a neomycin resistance gene, and therefore, is not preferred for mammalian expression systems.

In order to generate stable mammalian cell lines, the NF-κB/SV40/SEAP cassette is removed from the above NF-κB/SEAP vector using restriction enzymes Sail and NotI, and inserted into a vector containing neomycin resistance. Particularly, the NF-κB/SV40/SEAP cassette was inserted into pGFP-1 (Clontech), replacing the GFP gene, after restricting pGFP-1 with Sail and NotI.

Once NF-κB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H11, with a 5-10 fold activation typically observed.

### Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability

Binding of a ligand to a receptor is known to alter intracellular levels of small molecules, such as calcium, potassium, sodium, and pH, as well as alter membrane potential. These alterations can be measured in an assay to identify supematants which bind to receptors of a particular cell. Although the following protocol describes an assay for calcium, this protocol can easily be modified to detect changes in potassium, sodium; pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000 -20,000 cells/well in a Co-star black 96-well plate with clear bottom. The plate is incubated in a CO₂ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 ul of 12 ug/ml fluo-3 is added to each well. The plate is incubated at 37°C in a CO₂ incubator for 60 min. The plate is washed four times in the Biotek washer with HBSS leaving 100 ul of buffer.

For non-adherent cells, the cells are spun down from culture media. Cells are re-suspended to 2-5x10⁶ cells/ml with HBSS in a 50-ml conical tube. 4 ul of 1 mg/ml fluo-3 solution in 10% pluronic acid DMSO is added to each ml of cell suspension. The tube is then placed in a 37°C water bath for 30-60 min. The cells are washed twice with HBSS, resuspended to 1x10⁶ cells/ml, and dispensed into a microplate, 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular Ca⁺⁺ concentration.

### Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, Ick, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul ATP/Mg₂₊ (5mM ATP/50mM MgCl₂), then 10ul of 5x Assay Buffer (40mM imidazole hydrochloride, pH7.3, 40 mM beta-glycerophosphate, 1mM EGTA, 100mM MgCl₂, 5 mM MnCl_{2,} 0.5 mg/ml BSA), then 5ul of Sodium Vanadate(1mM), and then 5ul of water. Mix the components gently and preincubate the reaction mix at 30°C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37°C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37°C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (1ug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supematants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

### Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95°C for 30 seconds; 60-120 seconds at 52-58°C; and 60-120 seconds at 70°C, using buffer solutions described in Sidransky, D., et al., Science 252:706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is then cloned and sequenced to validate the results of the direct sequencing.

PCR products is cloned into T-tailed vectors as described in Holton, T.A. and Graham, M.W., Nucleic Acids Research, 19:1156 (1991) and sequenced with T7 polymerase (United States Biochemical). Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson, Cg. et al., Methods Cell Biol. 35:73-99 (1991). Hybridization with the labeled probe is carried out using a vast excess of human cot-1 DNA for specific hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson, Cv. et al., Genet. Anal. Tech. Appl., 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

### Example 22: Method of Detecting Abnormal Levels of a Polypeptide in a Biological Sample

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbance of the Y-axis (linear scale). Interpolate the concentration of the polypeptide in the sample using the standard curve.

### Example 23: Formulating a Polypeptide

The secreted polypeptide composition will be formulated and dosed in a fashion consistent with good medical practice, taking into account the clinical condition of the individual patient (especially the side effects of treatment with the secreted polypeptide alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the secreted polypeptide is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrastemal, subcutaneous and intraarticular injection and infusion.

The secreted polypeptide is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al.) or poly-D- (-)-3-hydroxybutyric acid (EP 133,988). Sustained-release compositions also include liposomally entrapped polypeptides. Liposomes containing the secreted polypeptide are prepared by methods known per se: DE 3,218,121; Epstein et al., Proc. Natl. Acad. Sci. USA 82:3688-3692 (1985); Hwang et al., Proc. Natl. Acad. Sci. USA 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, the secreted polypeptide is formulated generaliy by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The secreted polypeptide is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any polypeptide to be used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multi-dose containers, for example, sealed ampoules or vials, as an aqueous solution or as a lyophilized formulation for reconstitution. As an example of a lyophilized formulation, 10-ml vials are tilled with 5 ml of sterile-filtered 1% (w/v) aqueous polypeptide solution, and the resulting mixture is lyophilized. The infusion solution is prepared by reconstituting the lyophilized polypeptide using bacteriostatic Water-for-Injection.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

### Example 24: Method of Treating Decreased Levels of the Polypeptide

It will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a pharmaceutical composition comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

### Example 25: Method of Treating Increased Levels of the Polypeptide

Antisense technology is used to inhibit production of a polypeptide of the present invention. This technology is one example of a method of decreasing levels of a polypeptide, preferably a secreted form, due to a variety of etiologies, such as cancer.

For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention can be amplified using PCR primers which correspond to the 5' and 3' end sequences respectively as set forth in Example 1. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.

The engineered fibroblasts are then transplanted onto the host, either alone or after having been grown to confluence on cytodex 3 microcarrier beads.

### Example 27: Method of Treatment Using Gene Therapy - In Vivo

Another aspect of the present invention is using *in vivo* gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide of the present invention. A polynucleotide of the present invention may be operatively linked to a promoter or any other genetic elements necessary for the expression of the encoded polypeptide by the target tissue. Such gene therapy and delivery techniques and methods are known in the art, see, for example, WO90/11092, WO98/11779; U.S. Patent NO. 5693622, 5705151, 5580859; Tabata H. et al. (1997) Cardiovasc. Res. 35(3):470-479, Chao J et al. (1997) Pharmacol. Res. 35(6):517-522, Wolff J.A. (1997) Neuromuscul. Disord. 7(5):314-318, Schwartz B. et al. (1996) Gene Ther. 3(5):405-411, Tsurumi Y. et al. (1996) Circulation 94(12):3281-3290 (incorporated herein by reference).

The polynucleotide constructs of the present invention may be delivered by any method that delivers injectable materials to the cells of an animal, such as, injection into the interstitial space of tissues (heart, muscle, skin, lung, liver, intestine and the like). These polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides may also be delivered in liposome formulations (such as those taught in Felgner P.L. et al. (1995) Ann. NY Acad. Sci. 772:126-139 and Abdallah B. et al. (1995) Biol. Cell 85(1):1-7) which can be prepared by methods well known to those skilled in the art.

The polynucleotide vector constructs of the present invention used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the present invention can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for the polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA of the present invention.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

Further, the Sequence Listing submitted herewith in paper and computer readable forms are herein incorporated by reference in their entireties.

## Claims

1. A protein selected from the group consisting of:
(a) a protein comprising amino acid residues 22 to 180 of SEQ ID NO:100;
(b) the protein of (a) which comprises amino acid residues 2 to 180 of SEQ ID NO:100;
(c) the protein of (a) which comprises amino acid residues 1 to 180 of SEQ ID NO:100;
(d) a protein comprising the amino acid sequence of the secreted portion of the polypeptide encoded by the HNHFO29 cDNA contained in ATCC Deposit No. 209138;
(e) the protein of (d) which comprises the amino acid sequence of the complete polypeptide encoded by the HNHFO29 cDNA contained in ATCC Deposit No. 209138, excepting the N-terminal methionine;
(f) the protein of (d) which comprises the amino acid sequence of the complete polypeptide encoded by the HNHFO29 cDNA contained in ATCC Deposit No. 209138;
(g) a protein comprising a polypeptide sequence which is at least 90% identical to amino acid residues 22 to 180 of SEQ ID NO:100;
(h) the protein of (g), wherein said polypeptide sequence is at least 90% identical to amino acid residues 1 to 180 of SEQ ID NO:100;
(i) the protein of (g), wherein said polypeptide sequence is at least 95% identical to amino acid residues 22 to 180 of SEQ ID NO:100;
(j) the protein of (g), wherein said polypeptide sequence is at least 95% identical to amino acid residues 1 to 180 of SEQ ID NO:100;
(k) a protein comprising a polypeptide sequence which is at least 90% identical to the secreted portion of the polypeptide encoded by the HNHFO29 cDNA contained in ATCC Deposit No. 209138;
(I) the protein of (k), wherein said polypeptide sequence is at least 90% identical to the complete polypeptide encoded by the HNHFO29 cDNA contained in ATCC Deposit No. 209138;
(m) the protein of (k), wherein said polypeptide sequence is at least 95% identical to the secreted portion of the polypeptide encoded by the HNHFO29 cDNA contained in ATCC Deposit No. 209138;
(n) the protein of (k), wherein said polypeptide sequence is at least 95% identical to the complete polypeptide encoded by the HNHFO29 cDNA contained in ATCC Deposit No. 209138;
(o) a protein comprising at least 30 contiguous amino acid residues of amino acid residues 22 to 180 of SEQ ID NO:100;
(p) the protein of (o) which comprises at least 50 contiguous amino acid residues of amino acid residues 22 to 180 of SEQ ID NO:100;
(q) a protein comprising at least 30 contiguous amino acid residues of the secreted portion of the polypeptide encoded by the HNHFO29 cDNA contained in ATCC Deposit No. 209138;
(r) the protein of (q) which comprises at least 50 contiguous amino acid residues of the secreted portion of the polypeptide encoded by the HNHFO29 cDNA contained in ATCC Deposit No. 209138;
(s) a protein comprising at least 30 contiguous amino acid residues of amino acid residues 1 to 180 of SEQ ID NO:100;
(t) the protein of (s) which comprises at least 50 contiguous amino acid residues of amino acid residues 1 to 180 of SEQ ID NO:100;
(u) a protein comprising at least 30 contiguous amino acid residues of the complete polypeptide encoded by the HNHFO29 cDNA contained in ATCC Deposit No. 209138;
(v) the protein of (u) which comprises at least 50 contiguous amino acid residues of the complete polypeptide encoded by the HNHFO29 cDNA contained in ATCC Deposit No. 209138.

2. The protein of claim 1 which comprises a heterologous polypeptide sequence.

3. A composition comprising the protein of claim 1 and a pharmaceutically acceptable carrier.

4. A protein produced by the method comprising:
(a) expressing the protein of claim 1 by a cell; and
(b) recovering said protein.
